(19) **Europäisches Patentamt / European Patent Office / Office européen des brevets**

(11) **EP 1 506 021 B1**

(12) **EUROPÄISCHE PATENTSCHRIFT**

(45) Veröffentlichungstag und Bekanntmachung des Hinweises auf die Patenterteilung:
**01.05.2019 Patentblatt 2019/18**

(51) Int Cl.:
*A61K 48/00* (2006.01)     *C12N 15/86* (2006.01)
*A61P 35/00* (2006.01)     *A61K 35/761* (2015.01)
*C07K 14/005* (2006.01)     *A61K 35/763* (2015.01)
*C12N 7/00* (2006.01)

(21) Anmeldenummer: **03755145.4**

(22) Anmeldetag: **27.05.2003**

(86) Internationale Anmeldenummer:
**PCT/EP2003/005583**

(87) Internationale Veröffentlichungsnummer:
**WO 2003/099859 (04.12.2003 Gazette 2003/49)**

(54) **VERWENDUNG VON ADENOVIREN UND DAFUR KODIERENDEN NUKLEINSÄUREN**

USE OF ADENOVIRUSES AND NUCLEIC ACIDS CODING THEREFOR

UTILISATION D'ADENOVIRUS ET ACIDES NUCLEIQUES CODANT POUR CES DERNIERS

(84) Benannte Vertragsstaaten:
**AT BE BG CH CY CZ DE DK EE ES FI FR GB GR HU IE IT LI LU MC NL PT RO SE SI SK TR**

(30) Priorität: 27.05.2002  DE 10223534
07.06.2002  DE 10225400
15.10.2002  DE 10248039
19.05.2003  DE 10322530

(43) Veröffentlichungstag der Anmeldung:
**16.02.2005 Patentblatt 2005/07**

(73) Patentinhaber: **Holm, Per Sonne**
**82256 Fürstenfeldbruck (DE)**

(72) Erfinder: **Holm, Per Sonne**
**82256 Fürstenfeldbruck (DE)**

(74) Vertreter: **Bohmann, Armin K.**
**Bohmann**
**Anwaltssozietät**
**Nymphenburger Straße 1**
**80335 München (DE)**

(56) Entgegenhaltungen:
**EP-A- 0 931 830**

• **GANLY I ET AL: "Replication and cytolysis of an E1B-attenuated adenovirus in drug-resistant ovarian tumour cells is associated with reduced apoptosis." GENE THERAPY. ENGLAND MAR 2001, Bd. 8, Nr. 5, März 2001 (2001-03), Seiten 369-375, XP002260275 ISSN: 0969-7128**

• **HOLM P S ET AL: "YB-1 relocates to the nucleus in adenovirus infected cells and facilitates viral replication E2 gene expression through the E2--- late promoter" JOURNAL OF BIOLOGICAL CHEMISTRY, AMERICAN SOCIETY OF BIOLOGICAL CHEMISTS, BALTIMORE, MD, US, Bd. 277, Nr. 12, 22. März 2002 (2002-03-22), Seiten 10427-10434, XP002237181 ISSN: 0021-9258 in der Anmeldung erwähnt**

• **HOWE J A ET AL: "Evaluation of E1-mutant adenoviruses as conditionally replicating agents for cancer therapy." MOLECULAR THERAPY: THE JOURNAL OF THE AMERICAN SOCIETY OF GENE THERAPY. UNITED STATES NOV 2000, Bd. 2, Nr. 5, November 2000 (2000-11), Seiten 485-495, XP002260276 ISSN: 1525-0016**

• **HEISE C ET AL: "An adenovirus E1A mutant that demonstrates potent and selective systemic anti-tumoral efficacy." NATURE MEDICINE. UNITED STATES OCT 2000, Bd. 6, Nr. 10, Oktober 2000 (2000-10), Seiten 1134-1139, XP002260277 ISSN: 1078-8956**

• **WONG H K ET AL: "Complementary functions of E1a conserved region 1 cooperate with conserved region 3 to activate adenovirus serotype 5 early promoters." JOURNAL OF VIROLOGY. UNITED STATES AUG 1994, Bd. 68, Nr. 8, August 1994 (1994-08), Seiten 4910-4920, XP002260278 ISSN: 0022-538X**

**(Forts. nächste Seite)**

- BARGOU R C ET AL: "NUCLEAR LOCALIZATION AND INCREASED LEVELS OF TRANSCRIPTION FACTOR YB-1 IN PRIMARY HUMAN BREAST CANCERS ARE ASSOCIATED WITH INTRINSIC MDR1 GENE EXPRESSION" NATURE MEDICINE, NATURE PUBLISHING, CO, US, Bd. 3, Nr. 4, April 1997 (1997-04), Seiten 447-450, XP001148855 ISSN: 1078-8956 in der Anmeldung erwähnt

- STEIN ULRIKE ET AL: "Hyperthermia-induced nuclear translocation of transcription factor YB-1 leads to enhanced expression of multidrug resistance-related ABC transporters" JOURNAL OF BIOLOGICAL CHEMISTRY, Bd. 276, Nr. 30, 27. Juli 2001 (2001-07-27), Seiten 28562-28569, XP002260279 ISSN: 0021-9258 in der Anmeldung erwähnt

**Beschreibung**

Die Erfindung betrifft die Verwendung eines Adenovirus zur Herstellung eines Medikamentes, einen Adenovirus zur Verwendung als Medikament, die Verwendung eines Adenovirus zur Replikation in Zellen, die Verwendung eines adenoviralen Replikationssystems umfassend eine für den Virus codierende Nukleinsäure umfasst, die Verwendung einer für den Adenovirus codierenden Nukleinsäure zur Herstellung eines Medikamentes, die Verwendung einer für den Adenovirus codierenden Nukleinsäure zur Replikation in Zellen, die Verwendung eines die Nukleinsäure umfassenden Vektors zur Herstellung eines Medikamentes, und die Verwendung eines mit YB-1 wechselwirkenden Mittels.

Bei der Behandlung von Tumoren werden derzeit eine Vielzahl von Therapiekonzepten verfolgt. Neben der Verwendung chirurgischer Techniken stehen dabei die Chemotherapie und die Strahlentherapie im Vordergrund. All diese Techniken sind jedoch für den Patienten mit nicht unerheblichen Nebenwirkungen verbunden. Mit der Verwendung von replikationsselektiven onkolytischen Viren wurde eine neue Plattform für die Behandlung von Tumoren geschaffen. Dabei wird eine selektive intratumorale Replikation eines viralen Agens herbeigeführt, die in der Folge zur Virusreplikation, Lyse der infizierten Tumorzelle und Verbreitung des Virus auf benachbarte Tumorzellen führt. Infolge der Beschränkung der Replikationsfähigkeit des Virus auf Tumorzellen bleibt normales Gewebe von der Replikation und damit der Lyse durch das Virus verschont.

Derzeit finden verschiedene virale Systeme in klinischen Studien mit dem Ziel der Tumorlyse Anwendung. Ein Beispiel für einen derartigen Adenovirus ist dl1520 (Onyx-015), wie er als solches auch in Ganly I et al. (Ganly I et al., Gene Therapy (2001) 8, S. 369-375) beschrieben ist und der bereits erfolgreich in den klinischen Phasen I und II eingesetzt wurde (Khuri, F. et al. Nature Medicine 6, 879-885, 2000). Onyx-015 ist ein Adenovirus, bei dem das E1B-55kDA-Gen vollständig deletiert ist. Die vollständige Deletion des E1B55kDa-Proteins des Adenovirus beruht dabei auf der Beobachtung, dass im adenoviralen Vektor die Replikation und somit die Lyse von Zellen mit defektem p53 möglich ist (Kirn, D. et al., Proc. Am. Soc. Clin. Oncol. 17, 391a, 1998), wobei normale Zellen nicht geschädigt werden. Genauer ist das E1B-55kDa-Genprodukt beteiligt an der Inhibierung von p53, dem Transport viraler mRNA und dem Abschalten der Proteinsynthese der Wirtszelle. Die Inhibierung von p53 erfolgt dabei durch Ausbilden eines Komplexes aus p53 und dem adenoviral codierten E1B-55kDa Protein und/oder Komplex bestehend aus E1B-55kDA und E4orf6. Von p53, codiert von TP53, geht ein komplexer regulatorischer Mechanismus aus (Zambetti, G.P. et al., FASEB J. 7, 855-865, 1993), der u.a. auch dazu führt, dass eine effiziente Replikation von Viren wie Adenoviren in der Zelle unterdrückt wird. Das Gen TP 53 ist in etwa 50 % aller menschlichen Tumoren deletiert oder mutiert mit der Folge, dass es zu keiner - erwünschten - Apoptose infolge einer Chemotherapie oder einer Bestrahlungstherapie kommt und damit der Erfolg dieser Tumorbehandlungen im Normalfall unterbleibt.

Ein weiteres Konzept tumorlytischer Viren beruht auf der Beobachtung, dass wenn das E1A-Protein in einer bestimmten Weise deletiert vorliegt bzw. eine oder mehrere Mutationen aufweist, welche die Bindung von Rb/E2F und/oder p107/E2F und/oder p130/E2F nicht beeinflussen, solche Adenoviren den Eintritt der infizierten Zellen in die S-Phase nicht induzieren und zur Replikation in Tumorzellen befähigt sind, die kein funktionelles Rb-Protein aufweisen. Zudem kann das E1A-Protein am N-Terminus deletiert vorliegen bzw. eine oder mehrere Mutationen im Bereich der Aminosäurepositionen 1 bis 76 des E1A-Proteins umfassen, um die Bindung von E1A an p300 zu unterbinden, um somit eine selektivere Replikation in Tumorzellen zu bewerkstelligen. Diese Vorgehensweisen sind beispielhaft beschrieben in dem europäischen Patent EP 0 931 830. Beispiele derartiger Adenoviren sind AdΔ24, dl922 - 947, E1Ad/01/07 und CB016 (Howe, J. A. et al., Molecular Therapy 2, 485-495, 2000; Fueyo, J. et al., Oncogene 19, 2-12, 2000; Heise, C. et al., Nature Medicine 6, 11341139, 2001; Balague, C. et al., J. Virol. 75, 7602-7611, 2001). Diese im Stand der Technik bekannten adenoviralen Systeme zur Onkolyse weisen somit bestimmte Deletionen im E1A Protein auf, wobei diese Deletion vorgenommen worden war unter der Annahme, dass intakte Rb-Proteine bzw. Komplex bestehend aus intaktem Rb-Protein und E2F einer effiziente Replikation in vivo entgegenwirken würden und um eine adenovirale Replikation in vivo nur in Rb-negativen/mutierten Zellen sicher zu stellen. Diese adenoviralen Systeme nach dem Stand der Technik gehen auf E1A zurück, um mittels des frühen E2-Promotors (engl. E2 early Promotor) und freiem E2F die in vivo Replikation zu steuern (Dyson, N. Genes & Development, 12, 2245-2262, 1998).

Eine weitere Form von tumorlytischen adenoviralen Systemen beruht auf dem Einsatz selektiver Promotoren, um das virale Onkogen E1A spezifisch zu exprimieren, was eine selektive Replikation in Tumorzellen erlaubt (Rodriguez, R. et al., Cancer Res. 57, 2559-2563, 1997).

Wie vorstehend beschrieben kommt es bei den verschiedenen Konzepten adenoviraler tumorlytischer Viren auf die Auswahl eines für das dem jeweiligen Konzept zugrundeliegenden Wirkmechanismus geeigneten zellulären Hintergrundes an. Mit anderen Worten, die verschiedenen derzeit bekannten adenoviralen Systeme zur Tumorlyse können nur bei Vorliegen bestimmter molekularbiologischer Voraussetzungen angewandt werden. Dies beschränkt die Anwendung derartiger Systeme auf bestimmte Patientenkollektive.

Ein besonderes Problem bei der Behandlung von Tumorerkrankungen stellt sich dann ein, wenn die Patienten eine sogenannte klassische Vielfachresistenz (engl. multidrug resistence (MDR)) entwickeln, die eine besonders gut untersuchte Resistenzform von Tumoren gegenüber Zytostatika darstellt (Gottesman und Pastan, Annu. Rev. Biochem. 62, 385-427, 1993). Sie beruht auf der Überexpression des membranständigen Transportproteins P-Glykoprotein, das zu den sogenannten ABC-Transportern gehört (Stein, U. et al., JBC 276, 28562-69, 2001, J. Wijnholds, Novartis Found Symp., 243, 69-79, 2002). Bargou, R. C. et al. und Oda, Y. et al (Bargou, R. C. et al., Nature Medicine 3, 447-450, 1997; Clin. Cancer Res. 4, 2273-2277, 1998) konnten zeigen, dass die Kernlokalisation des humanen Transkriptionsfaktors YB-1 unmittelbar an der Aktivierung der Expression des P-Glykoproteins beteiligt ist. Weitere Untersuchungen belegen, dass YB-1 durch verschiedene Streßbedingungen in den Zellkern gelangt, wie z.B. UV-Bestrahlung, Zytostatika-Applikation (Koike, K. et al., FEBS Lett 17, 390-394, 1997) und Hyperthermie (Stein, U. et al., JBC 276, 28562-69, 2001). Weitere Untersuchungen belegen, dass die nukläre Lokalisation von YB-1 einen Einfluss auf einen weiteren ABC-Transporter ausübt. Dieser ABC-Transporter wird als MRP (engl. multidrug resistance-related protein) bezeichnet und ist mit der Ausbildung des sogenannten atypischen, nicht P-Glykoprotein-abhängigen Vielfachresistenz beteiligt (Stein, U. et al., JBC 276, 28562-69, 2001).

Holm P S et al (Holm P S et al. JBC 277, S. 10427-10434, 2002) offenbaren, dass YB-1 in mit Adenoviren infizierten Zellen in den Zellkern transloziert wird und die virale Replikation durch Induktion der E2-Gen-Expression vermittels des E2 late-Promotors erleichtert wird.

Howe J A et al (Howe J A et al., Molecular Therapy Bd. 2, Nr. 5, November 2000, S. 485-494) offenbaren verschiedene Formen von E1A-Protein.

Wong H K et al (Wong H K et al, Journal of Virology, Aug 1994, Bd. 68, Nr. 8, S. 4910-4920) beschreiben den Aufbau von E1A und insbesondere die Bedeutung der CR3-Region für die beobachtete transaktivierende Funktion von E1A im Rahmen der adenoviralen Replikation.

Der vorliegenden Erfindung liegt die Aufgabe zugrunde, eine technische Lehre und insbesondere ein Mittel bereitzustellen, welches erlaubt, einen Organismus, speziell einen menschlichen Organismus bzw. ein Patientenkollektiv spezifisch mit tumorlytisch wirkenden Agenzien zu behandeln. Weiterhin ist es eine der vorliegenden Erfindung zugrundeliegenden Aufgabe, ein Mittel bereitzustellen, welches geeignet ist, bei Patienten mit Tumorerkrankungen eine Tumorlyse herbeizuführen, die gegenüber Zytostatika resistent sind, insbesondere solche, die eine Vielfachresistenz zeigen.

Erfindungsgemäß wird die Aufgabe gelöst durch den Gegenstand der beigefügten unabhängigen Ansprüche. Besonders bevorzugte Ausführungsformen ergeben sich aus den Unteransprüchen.

Die Aufgabe wird erfindungsgemäß in einem ersten Aspekt auch gelöst durch die Verwendung eines Adenovirus zur Herstellung eines Medikamentes für die Behandlung von Tumoren, dadurch gekennzeichnet, dass der Virus in Zellen repliziert, die YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, wobei das Onkogenprotein E1A ist und die den Tumor oder einen Teil davon ausbildenden Zellen YB-1 im Kern aufweisen.

Die Aufgabe wird erfindungsgemäß in einem zweiten Aspekt auch gelöst durch die *in vitro* Verwendung eines Adenovirus zur Replikation in Zellen, dadurch gekennzeichnet, dass die Zellen YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, und das Onkogenprotein E1A ist.

Die Aufgabe wird erfindungsgemäß in einem dritten Aspekt auch gelöst durch einen Adenovirus zur Verwendung als Medikament, dadurch gekennzeichnet, dass der Virus in Zellen repliziert, die YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, wobei das Onkogenprotein E1A ist.

In einer Ausführungsform des dritten Aspektes ist vorgesehen, dass die Verwendung die Verwendung bei der Behandlung von Tumoren, wobei die den Tumor oder einen Teil davon ausbildenden Zellen YB-1 im Kern aufweisen.

In einer Ausführungsform des ersten, zweiten und dritten Aspektes ist vorgesehen, dass das virale Onkogenprotein E1A nicht die nukläre Lokalisation von YB-1 induziert.

In einer Ausführungsform des ersten, zweiten und dritten Aspektes ist vorgesehen, dass die Zellen Rb-negativ und im Zellkern YB-1 positiv, insbesondere unabhängig vom Zellzyklus im Zellkern YB-1 positiv sind.

In einer Ausführungsform des ersten und dritten Aspektes ist vorgesehen, dass die den Tumor oder Teile davon ausbildenden Zellen eine Resistenz gegen pharmakologische Wirkstoffe aufweisen.

In einer Ausführungsform des ersten und dritten Aspektes ist vorgesehen, dass die Zellen eine Überexpression des membranständigen Transportproteins P-Glykoprotein zeigen.

In einer Ausführungsform des ersten, zweiten und dritten Aspektes ist vorgesehen, dass das virale Onkogenprotein unter der Kontrolle eines Gewebes-und/oder Tumor-spezifischen Promotors steht.

In einer Ausführungsform des ersten, zweiten und dritten Aspektes ist vorgesehen, dass der Virus für YB-1 codiert.

In einer Ausführungsform des ersten, zweiten und dritten Aspektes ist vorgesehen, dass YB-1 unter der Kontrolle eines Gewebe- und/oder Tumor-spezifischen Promotors steht.

In einer Ausführungsform des ersten, zweiten und dritten Aspektes ist vorgesehen, dass der Virus für mindestens ein Protein codiert, das ausgewählt ist aus der Gruppe, die E4orf6, E4orf3, E1B55k und adenovirales E3ADP-Protein umfasst.

In einer Ausführungsform des ersten, zweiten und dritten Aspektes ist vorgesehen, dass die den Tumor oder einen Teil davon ausbildenden Zellen YB-1 im Kern aufweisen.

In einer Ausführungsform des ersten, zweiten und dritten Aspektes ist vorgesehen, dass der Tumor YB-1 im Kern nach Induktion des Transports von YB-1 in den Kern enthält.

In einer Ausführungsform des ersten, zweiten und dritten Aspektes ist vorgesehen, dass der Virus ausgewählt ist aus der Gruppe, die AdΔ24, dl922-947, E1Ad/01/07, dl1119/1131, CB 016, dl520, und Viren, denen ein exprimiertes virales Onkogen fehlt, das zur Bindung eines funktionellen Rb-Tumorsuppressor-Genprodukts fähig ist, umfasst.

In einer Ausführungsform des ersten, zweiten und dritten Aspektes ist vorgesehen, dass der Virus E1B 19 kDa-defizient ist.

Die Aufgabe wird erfindungsgemäß in einem vierten Aspekt auch gelöst durch die Verwendung eines adenoviralen Replikationssystems umfassend eine Nukleinsäure, die für einen Adenovirus, wie im Zusammenhang mit dem ersten, zweiten und dritten Aspekt, einschließlich einer jeden Ausführungsform davon, definiert, codiert, und umfassend eine Nukleinsäure eines Helfervirus, wobei die Nukleinsäure des Helfervirus eine Nukleinsäuresequenz umfasst, die für YB-1 codiert, zur Herstellung eines Medikamentes für die Behandlung von Tumoren, wobei die den Tumor oder einen Teil davon ausbildenden Zellen YB-1 im Kern aufweisen, oder zur Replikation in Zellen, die YB-1 aufweisen.

Die Aufgabe wird erfindungsgemäß in einem fünften Aspekt auch gelöst durch die Verwendung einer Nukleinsäure codierend für einen Adenovirus, wie im Zusammenhang mit dem ersten, zweiten und dritten Aspekt, einschließlich einer jeden Ausführungsform davon, definiert, zur Herstellung eines Medikamentes für die Behandlung von Tumoren, wobei die den Tumor oder einen Teil davon ausbildenden Zellen YB-1 im Kern aufweisen.

In einer Ausführungsform des fünften Aspektes ist vorgesehen, dass die den Tumor oder Teile davon ausbildenden Zellen eine Resistenz gegen pharmakologische Wirkstoffe aufweisen.

In einer Ausführungsform des fünften Aspektes ist vorgesehen, dass die Resistenz eine Mehrfachresistenz ist.

In einer Ausführungsform des fünften Aspektes ist vorgesehen, dass der pharmakologische Wirkstoff ein Antitumormittel ist.

In einer Ausführungsform des fünften Aspektes ist vorgesehen, dass das Antitumormittel ein Zytostatikum ist.

Die Aufgabe wird erfindungsgemäß in einem sechsten Aspekt auch gelöst durch die *in* vitro Verwendung einer Nukleinsäure, die für einen Adenovirus, wie im Zusammenhang mit dem ersten, zweiten und dritten Aspekt, einschließlich einer jeden Ausführungsform davon, definiert, codiert, zur Replikation in Zellen, dadurch gekennzeichnet, dass die Zellen YB-1 im Kern aufweisen, der Virus replikationsdefizient ist in Zellen, die YB-1 nicht im Kern aufweisen, und der Virus für ein Onkogen oder Onkogenprodukt codiert, das zumindest ein virales Gen transaktiviert, wobei das Gen ausgewählt ist aus der Gruppe, die E1B55kDa, E4orf6, E4orf3 und E3ADP umfasst.

Die Aufgabe wird erfindungsgemäß in einem siebten Aspekt auch gelöst durch die Verwendung eines Vektors umfassend eine Nukleinsäure, wie im Zusammenhang mit dem fünften Aspekt, einschließlich einer jeden Ausführungsform davon, definiert zur Herstellung eines Medikamentes zur Behandlung von Tumoren, wobei die den Tumor oder einen Teil davon ausbildenden Zellen YB-1 im Kern aufweisen, oder zur Replikation in Zellen, die YB-1 im Kern aufweisen.

Die Aufgabe wird erfindungsgemäß in einem achten Aspekt auch gelöst durch die in vitro Verwendung eines mit YB-1 wechselwirkenden Mittels zur Charakterisierung von Zellen, Zellen eines Tumorgewebes oder Patienten, um zu bestimmen, ob diese(r) mit einem Virus wie im Zusammenhang mit dem ersten, zweiten und dritten Aspekt, einschließlich einer jeden Ausführungsform davon, definiert kontaktiert und/oder behandelt werden sollen, wobei das Mittel ausgewählt ist aus der Gruppe, die Antikörper, Antikaline, Aptamere, Aptazyme und Spiegelmere umfasst.

In einer Ausführungsform des ersten, zweiten und dritten Aspektes ist vorgesehen, dass der Virus eine für ein Transgen codierende Nukleinsäure umfasst.

In einer Ausführungsform des ersten, zweiten und dritten Aspektes ist vorgesehen, dass der Virus das Translations- und/oder das Transkriptionsprodukt eines Transgens umfasst.

In einer Ausführungsform des vierten Aspektes ist vorgesehen, dass die Nukleinsäure des adenoviralen Replikationssystems und/oder die Nukleinsäure des Helfervirus ein Transgen oder eine für ein Transgen codierende Nukleinsäure umfasst.

In einer Ausführungsform des fünften Aspektes ist vorgesehen, dass die Nukleinsäure ein Transgen oder eine für ein Transgen codierende Nukleinsäure umfasst.

In einer Ausführungsform des ersten, zweiten, dritten, vierten und fünften Aspektes ist vorgesehen, dass das Transgen ausgewählt ist aus der Gruppe, die Prodruggene, Zytokine, Apoptose-induzierende Gene, Tumorsuppressorgene, Gene für Metalloproteinasen-Inhibitoren und Gene für Angiogene-Inhibitoren umfasst.

In einer Ausführungsform des ersten, zweiten, dritten, vierten und fünften Aspektes ist vorgesehen, dass das Transgen ausgewählt ist aus der Gruppe, die Nukleinsäuren für siRNA, für Aptamere, für Antisense-Moleküle und für Ribozyme umfasst, wobei die siRNA, die Aptamere, die Antisense-Moleküle und/oder die Ribozyme gegen ein Zielmolekül gerichtet ist.

In einer Ausführungsform des ersten, zweiten, dritten, vierten und fünften Aspektes ist vorgesehen, dass das Zielmolekül ausgewählt ist aus der Gruppe, die Resistenz-relevante Faktoren, Anti-Apoptose-Faktoren, Onkogene, Angiogenese-Faktoren, DNA-Synthese-Enzyme, DNA-Reparaturenzyme, Wachstumsfaktoren, Rezeptoren für Wachstumsfaktoren, Transkriptionsfaktoren, Metalloproteinasen, insbesondere Matrix-Metalloproteinkinasen, und Plasminogenaktivator vom Urokinase-Typ umfasst.

In einer Ausführungsform des ersten, dritten, vierten und fünften Aspektes ist vorgesehen, dass das Medikament weiterhin mindestens eine pharmazeutisch wirksame Verbindung enthält.

In einer Ausführungsform des ersten, dritten und fünften Aspektes ist vorgesehen, dass die Behandlung weiterhin die Verabreichung mindestens einer pharmazeutisch wirksame Verbindung umfasst.

In einer Ausführungsform des ersten, dritten und fünften Aspektes ist vorgesehen, dass die pharmazeutisch wirksame Verbindung ausgewählt ist aus der Gruppe, die Zytokine, Metalloproteinasen-Inhibitoren, Angiogenese-Inhibitoren, Cytostatika und Zellzyklus-Inhibitoren umfasst.

In einer bevorzugten Ausführungsform des ersten, zweiten und dritten Aspekts ist vorgesehen, dass das virale Onkogenprotein E1A zur Bindung eines funktionellen Rb-Tumorsuppressor-Genprodukts fähig ist.

In einer alternativen Ausführungsform des ersten, zweiten und dritten Aspekts ist vorgesehen, dass das virale Onkogenprotein E1A zur Bindung eines funktionellen Rb-Tumorsuppressor-Genprodukts nicht fähig ist.

In einer noch weiteren Ausführungsform des ersten und dritten Aspektes ist vorgesehen, dass das Medikament für Patienten bestimmt ist, deren Zellen Rb-positiv oder Rb-negativ sind, bzw. die zu behandelnden Patienten solche sind, deren Zellen Rb-positiv oder Rb-negativ sind.

In einer bevorzugten Ausführungsform ist dabei vorgesehen, dass die Zellen diejenigen Zellen sind, die an der Ausbildung des Zustandes beteiligt sind, der mit dem Medikament bzw. der Behandlung beeinflusst werden soll.

In einer noch weiteren Ausführungsform des ersten, zweiten und dritten Aspektes ist vorgesehen, dass die Zellen, insbesondere die den Tumor oder Teile davon ausbildenden Zellen eine Resistenz, insbesondere Mehrfachresistenz gegen pharmakologische Wirkstoffe, bevorzugter Weise Antitumormittel und bevorzugtererweise Zytostatika, aufweisen.

In einer bevorzugten Ausführungsform des ersten, zweiten und dritten Aspektes ist vorgesehen, dass die Zellen eine Expression, bevorzugterweise eine Überexpression des membranständigen Transportproteins P-Glykoprotein und/oder von MRP zeigen.

In einer weiteren Ausführungsform des ersten, zweiten und dritten Aspektes ist vorgesehen, dass die Zellen p53-positiv oder p53-negativ sind.

In einer Ausführungsform des ersten, zweiten und dritten Aspektes ist vorgesehen, dass das Onkogenprotein gegenüber dem Wildtyp-Onkogenprotein E1A eine oder mehrere Mutationen oder Deletionen aufweist, wobei die Deletion bevorzugt eine solche ist, die ausgewählt ist aus der Gruppe, die Deletionen des Bereichs CR3 und Deletionen des N-Terminus und Deletionen des C-Terminus umfasst. Dabei ist vorgesehen, dass das E1A-Onkogenprotein an Rb binden kann.

In einer weiteren Ausführungsform des ersten, zweiten und dritten Aspektes ist vorgesehen, dass das Onkogenprotein gegenüber dem Wildtyp-Onkogenprotein eine oder mehrere Mutationen oder Deletionen aufweist, wobei die Deletion bevorzugt eine solche in der CR1-Region und/oder der CR2-Region ist. Dabei ist vorgesehen, dass das Onkogenprotein E1A nicht an Rb zu binden in der Lage ist.

In einer alternativen Ausführungsform des ersten, zweiten und dritten Aspektes ist vorgesehen, dass die Zellen YB-1 im Kern aufweisen, insbesondere die den Tumor oder einen Teil davon ausbildenden Zellen YB-1 im Kern aufweisen.

In einer weiteren Ausführungsform des ersten, zweiten und dritten Aspektes ist vorgesehen, dass der Tumor YB-1 im Kern nach Induktion des Transports von YB-1 in den Kern enthält.

In einer bevorzugten Ausführungsform des ersten, zweiten und dritten Aspektes ist vorgesehen, dass der Transport von YB-1 in den Kern ausgelöst wird durch zumindest eine Maßnahme, die ausgewählt ist aus der Gruppe, die Bestrahlung, Gabe von Zytostatika und Hyperthermie umfasst.

In einer besonders bevorzugten Ausführungsform des ersten, zweiten und dritten Aspektes ist vorgesehen, dass die Maßnahme an einer Zelle, einem Organ oder einem Organismus angewandt wird.

Des Weiteren offenbart ist durch die Verwendung eines Virus, insbesondere des Adenovirus, zur Herstellung eines Medikaments, wobei der Virus, insbesondere der Adenovirus, so ausgebildet ist, dass die Replikation durch oder vermittels YB-1 über die Aktivierung des E2-Late-Promotors gesteuert wird, bevorzugterweise überwiegend über die Aktivierung des E2-Late-Promotors gesteuert wird. In einer Ausführungsform ist dabei vorgesehen, dass das YB-1 entweder ein transgenes YB-1 ist oder ein zelluläres, insbesondere zelluläres dereguliertes YB-1 ist. Unter einem transgenen YB-1 wird dabei bevorzugterweise ein solches verstanden, das durch einen Vektor, insbesondere einen oder den Adenovirus in einer Zelle zur Expression gebracht wird. Bei dem E2-Late-Promotor handelt es sich bevorzugterweise um den adenoviralen E2-Late-Promotors, wie er im Wildtyp-Adenovirus vorhanden ist, oder um einen E2-Late-Promotor, wie er im Zusammenhang mit der Expression von Transgenen hierin beschrieben verwendet wird.

Ebenfalls offenbart ist die Verwendung eines Virus, insbesondere der Adenovirus, zur Replikation in Zellen, die YB-1 im Kern aufweisen, wobei der Virus, insbesondere der Adenovirus, so ausgebildet ist, dass die Replikation durch YB-1 über die Aktivierung des E2-Late-Promotors gesteuert wird, bevorzugterweise überwiegend über die Aktivierung des E2-Late-Promotors gesteuert wird. In einer Ausführungsform ist dabei vorgesehen, dass das YB-1 entweder ein transgenes YB-1 ist oder ein zelluläres, insbesondere zelluläres dereguliertes YB-1 ist. Unter einem transgenen YB-1 wird dabei bevorzugterweise ein solches verstanden, das durch einen Vektor, insbesondere einen oder den Adenovirus in einer Zelle zur Expression gebracht wird. Bei dem E2-Late-Promotor handelt es sich bevorzugterweise um den adenoviralen E2-Late-Promotors, wie er im Wildtyp-Adenovirus vorhanden ist, oder um einen E2-Late-Promotor, wie er im Zusammenhang mit der Expression von Transgenen hierin beschrieben verwendet wird.

Im Zusammenhang mit den erfindungsgemäßen Verwendungen und Anwendungen ist der Adenovirus so ausgebildet wie hierin offenbart, insbesondere so, wie er ausgestaltet ist, um erfindungsgemäß verwendet zu werden.

Des Weiteren wird offenbart ein virales Onkogenprotein, insbesondere ein isoliertes virales Onkogenprotein, wobei dieses die folgenden Eigenschaften aufweist:

a) Transaktivierung mindestens eines viralen Gens in YB-1 Kern-positiven Zellen, das ausgewählt ist aus der Gruppe, die E1B-55K, E3ADP und E4orf6 und E4orf3 umfasst; und

b) keine Induktion von YB-1 in einem Zellkern, insbesondere in dem Zellkern der Zelle, in der das virale Onkoprotein vorhanden ist.

In einer Ausführungsform ist vorgesehen, dass das virale Onkoprotein E1A ist.

Es ist vorgesehen, dass das virale Onkogenprotein gegenüber dem Wildtyp-Onkogenprotein eine oder mehrere Mutationen oder Deletionen aufweist, wobei die Deletion bevorzugt eine solche ist, die ausgewählt ist aus der Gruppe, die Deletion des Bereichs CR3, Deletion des N-Terminus und Deletion des C-Terminus umfasst.

Es ist vorgesehen, dass die Induktion von YB-1 durch das virale Onkogenprotein unterbleibt, wenn E4orf6 und/oder E1B 55 kD nicht in der den Zellkern aufweisenden Zelle vorhanden ist/sind.

Es ist vorgesehen, dass das virale Onkogenprodukt an Rb zu binden in der Lage ist.

Es ist alternative vorgesehen, dass das virale Onkogenprotein eine oder mehrer Mutationen oder Deletionen aufweist, wobei die Deletion bevorzugt eine solche in der CR1-Region und/oder der CR2-Region des E1A-Onkogenproteins ist. Dabei ist vorgesehen, dass das virale Onkogenprotein nicht in der Lage ist an Rb zu binden.

In einer Ausführungsform des vierten Aspektes ist vorgesehen, dass die adenovirale Nukleinsäure und/oder die Nukleinsäure des Helfervirus als replizierbarer Vektor vorliegt.

In einer Ausführungsform des ersten zweiten, dritten, vierten, fünften, sechsten, siebten und achten Aspektes ist vorgesehen, dass die Zellen, insbesondere die den Tumor oder Teile davon ausbildenden Zellen, eine Resistenz, insbesondere eine Mehrfachresistenz gegen pharmakologische Wirkstoffe, bevorzugterweise Antitumormittel, und bevorzugtererweise Zytostatika, aufweisen.

Des Weiteren wird offenbart die Verwendung einer Nukleinsäure, die für einen Virus, insbesondere einen Adenovirus, wie er erfindungsgemäß verwendet wird, codiert zur Herstellung eines Medikamentes, wobei der Virus so ausgebildet ist, dass die Replikation durch YB-1 über die Aktivierung des E2-Late-Promotors gesteuert wird, bevorzugterweise überwiegend über die Aktivierung des E2-Late-Promotors. In einer Ausführungsform ist dabei vorgesehen, dass das YB-1 entweder ein transgenes YB-1 ist oder ein zelluläres, insbesondere zelluläres dereguliertes YB-1 ist. Unter einem transgenen YB-1 wird dabei bevorzugterweise ein solches verstanden, das durch einen Vektor, insbesondere einen oder den Adenovirus in einer Zelle zur Expression gebracht wird. Bei dem E2-Late-Promotor handelt es sich bevorzugterweise um den adenoviralen E2-Late-Promotors, wie er im Wildtyp-Adenovirus vorhanden ist, oder um einen E2-Late-Promotor, wie er im Zusammenhang mit der Expression von Transgenen hierin beschrieben verwendet wird.

Ebenfalls offenbart wird die Verwendung einer Nukleinsäure, die für einen Virus, insbesondere einen Adenovirus, wie er erfindungsgemäß verwendet wird, codiert, zur Replikation in Zellen, wobei der Virus so ausgebildet ist, dass die Replikation durch YB-1 über die Aktivierung des E2-Late-Promotors gesteuert wird, bevorzugterweise überwiegend über die Aktivierung des E2-Late-Promötors gesteuert wird. In einer Ausführungsform ist dabei vorgesehen, dass das YB-1 entweder ein transgenes YB-1 ist oder ein zelluläres, insbesondere zelluläres dereguliertes YB-1 ist. Unter einem transgenen YB-1 wird dabei bevorzugterweise ein solches verstanden, das durch einen Vektor, insbesondere einen oder den Adenovirus in einer Zelle zur Expression gebracht wird. Bei dem E2-Late-Promotor handelt es sich bevorzugterweise um den adenoviralen E2-Late-Promotors, wie er im Wildtyp-Adenovirus vorhanden ist, oder um einen E2-Late-Promotor, wie er im Zusammenhang mit der Expression von Transgenen hierin beschrieben verwendet wird.

Des Weiteren offenbart ist die Verwendung des erfindungsgemäßen viralen Onkogenproteins oder einer dafür codierenden Nukleinsäure zur Herstellung eines Virus, insbesondere eines Adenovirus, wie er im Rahmen der Verwendungen gemäß dem ersten und zweiten Aspekt der vorliegenden Erfindung verwendet wird.

In einer Ausführungsform des ersten bis achten Aspektes ist dabei vorgesehen, dass der Virus eine für ein Transgen codierende Nukleinsäure umfasst.

In einer weiteren Ausführungsform des ersten bis achten Aspektes ist dabei vorgesehen, dass der Virus das Translations- und/oder das Transkriptionsprodukt eines Transgens umfasst.

In einer bevorzugten Ausführungsform des vierten Aspektes ist dabei vorgesehen, dass die Nukleinsäure des adenoviralen Replikationssystems und/oder die Nukleinsäure des Helfervirus ein Transgen oder eine für ein Transgen codierende Nukleinsäure umfasst.

In einer noch weiteren Ausführungsform des ersten bis achten Aspektes ist dabei vorgesehen, dass die Nukleinsäure ein Transgen oder eine für ein Transgen codierende Nukleinsäure umfasst.

In einer alternativen Ausführungsform des ersten bis achten Aspektes ist dabei vorgesehen, dass das Transgen ausgewählt ist aus der Gruppe, die Prodruggene, Zytokine, Apoptose-induzierende Gene, Tumorsuppressorgene, Gene für Metalloproteinasen-Inhibitoren und Gene für Angiogene-Inhibitoren umfasst.

In einer Ausführungsform ist dabei vorgesehen, dass das Transgen ausgewählt ist aus der Gruppe, die Nukleinsäuren für siRNA, für Aptamere, für Antisense-Moleküle und für Ribozyme umfasst, wobei die siRNA, die Aptamere, die Antisense-Moleküle und/oder die Ribozyme gegen ein Zielmolekül gerichtet ist.

In einer weiteren Ausführungsform des ersten bis achten Aspektes ist dabei vorgesehen, dass das Zielmolekül ausgewählt ist aus der Gruppe, die Resistenz-relevante Faktoren, Anti-Apoptose-Faktoren, Onkogene, Angiogenese-Faktoren, DNA-Synthese-Enzyme, DNA-Reperaturenzyme, Wachstumsfaktoren und deren Rezeptoren, Transkriptionsfaktoren, Metalloproteinasen, insbesondere Matrix-Metalloproteinasen, und Plasminogenaktivator vom Urokinase-Typ umfasst. In einer Ausführungsform sind die Resistenz-relevanten Faktoren bevorzugt aus der Gruppe ausgewählt, die P-Glykoprotein, MRP und GST umfasst, und umfassen auch die dafür codierenden Nukleinsäuren. In einer Ausführungsform sind die Anti-Apopotose-Faktoren ausgewählt sind aus der Gruppe, die BCL2 umfasst, und umfassen auch die dafür codierenden Nukleinsäuren. In einer Ausführungsform sind die Onkogene ausgewählt aus der Gruppe, die Ras, insbesondere mutiertes Ras, Rb und Myc umfasst, und umfassen auch die dafür codierenden Nukleinsäuren. In einer Ausführungsform sind die Angiogenese-Faktoren ausgewählt aus der Gruppe, die VEGF und HMG-Proteine umfasst, und umfassen auch die dafür codierenden Nukleinsäuren. In einer Ausführungsform sind die DNA-Synthese-Enzyme ausgewählt aus der Gruppe, die Telomerase umfasst, und umfassen auch die dafür codierenden Nukleinsäuren. In einer Ausführungsform sind die DNA-Reparaturenzyme ausgewählt aus der Gruppe, die Ku-80 umfasst, und umfassen auch die dafür codierenden Nukleinsäuren. In einer Ausfiihrungsform sind die Wachstumsfaktoren ausgewählt aus der Gruppe, die PDGF, EGF und M-CSF umfasst, und umfassen auch die dafür codierenden Nukleinsäuren In einer weiteren Ausführungsform ist vorgesehen, dass die Rezeptoren insbesondere solche der Wachstumsfaktoren sind, wobei bevorzugterweise die Wachstumsfaktoren aus der Gruppe ausgewählt sind, die PDGF, EGF und M-CSF umfasst, und umfassen auch die dafür codierenden Nukleinsäuren. In einer Ausführungsform sind die Transkriptionsfaktor ausgewählt aus der Gruppe, die YB-1 umfasst, und umfassen auch die dafür codierenden Nukleinsäuren. In einer Ausführungsform sind die Metalloproteinasen insbesondere Matrix-Metalloproteinasen. In einer bevorzugten Ausführungsform sind die Matrix-Metalloproteinasen ausgewählt aus der Gruppe, die MMP-1 und MMP-2 umfasst, und umfassen auch die dafür codierenden Nukleinsäuren. In einer Ausführungsform sind die Plasminogenaktivatoren vom Urokinase-Typ ausgewählt aus der Gruppe, die uPa-R umfasst, und umfassen auch die dafür codierenden Nukleinsäuren.

In einer noch weiteren Ausführungsform ist dabei vorgesehen, dass das Medikament weiterhin mindestens eine pharmazeutisch wirksame Verbindung enthält.

In einer bevorzugten Ausführungsform ist dabei vorgesehen, dass die pharmazeutisch wirksame Verbindung ausgewählt ist aus der Gruppe, die Zytokine, Metalloproteinase-Inhibitoren, Angiogenese-Inhibitoren, Cytostatika und Zellzyklus-Inhibitoren umfasst.

Der vorliegenden Erfindung liegt die überraschende Erkenntnis zugrunde, dass die DNA-Replikation von E1A-modifizierten Adenoviren in YB-1 Kern-positiven Tumorzellen auf die Aktivierung des E2-late Promotors zurückzuführen ist. Unter E1A-modifizierten Adenoviren sind dabei solche Adenoviren zu verstehen, die (a) keine Replikation oder eine verglichen mit dem jeweiligen Wildtyp verringerte, bevorzugterweise stark verringerte Replikation, in YB-1-Kern-negativen Zellen zeigen, (b) transaktivierend auf mindestens ein virales Gen wirken, wobei das Gen insbesondere ausgewählt ist aus der Gruppe, die E1B-55kDa, E4orf6, E4orf3 und E3ADP umfasst, und/oder (c) zelluläres YB-1 durch den Adenovirus nicht in den Kern transloziert. Optional weisen die erfindungsgemäß verwendeten Adenoviren die weitere Eigenschaft auf, dass nämlich die Bindung des von dem Adenovirus kodierten E1A-Proteins die Bindung von E2F an RB stört bzw. den entsprechenden Komplex aus E2F und Rb aufzulösen in der Lage ist. Adenoviren, die eines oder mehrere der vorstehend genannten Merkmale a) bis c), bevorzugterweise alle Merkmale a) bis c) aufweisen, sind replikationsdefizient in Zellen, die YB-1 nicht im Kern aufweisen.

In einer Ausführungsform wird unter einer stark verringerten Replikation hierin insbesondere eine solche Replikation verstanden, die gegenüber dem Wildtyp um den Faktor 2, bevorzugterweise um den Faktor 5, bevorzugterweise um den Faktor 10 und am bevorzugtesten um den Faktor 100 verringert ist. In einer bevorzugten Ausführungsform ist vorgesehen, dass der Vergleich der Replikation bei Verwendung gleicher oder ähnlicher Zellinien, gleicher oder ähnlicher Virustiter für die Infektion (engl. multiplicity of infection, MOI, oder engl. plaque forming unit, pfu) und/oder gleicher oder ähnlicher allgemeiner Versuchsbedingungen durchgeführt wird. Dabei wird unter Replikation insbesondere die Partikelbildung verstanden. In weiteren Ausführungsformen kann jedoch als Mass für die Replikation der Umfang der Synthese viraler Nukleinsäure verstanden sein. Verfahren zur Bestimmung des Umfanges der Synthese viraler Nukleinsäuren sind ebenso wie Verfahren zur Bestimmung der Partikelbildung den Fachleuten auf dem Gebiet bekannt.

Die hierin beschriebenen Erkenntnisse bzw. die Verfahren, Verwendungen oder Nukleinsäuren, Proteine, Replikationssysteme und dergleichen sind nicht notwendigerweise auf Adenoviren beschränkt. Grundsätzlich gibt es auch bei anderen Viren derartige Systeme, die hiermit ebenfalls umfasst sind.

Bei Verwendung der erfindungsgemäßen Viren oder bei der erfindungsgemäßen Verwendung der hierin beschriebenen Viren kann eine dem Wildtyp vergleichbare Replikation bereits bei einer Infektionsrate von 1 bis 10 pfu/Zelle erreicht werden gegenüber 10 bis 100 pfu/Zelle gemäß dem Stand der Technik.

Unter zellulärem YB-1 soll hierin ein jedes YB-1 verstanden werden, welches von einer Zelle codiert und bevorzugterweise auch exprimiert wird, wobei dieses YB-1 in der Zelle insbesondere vor der Infektion der betreffenden Zelle mit einem Adenovirus, bevorzugterweise einem Adenovirus, und/oder einem Helfervirus, wie hierin beschrieben, vorhanden ist. Es ist jedoch auch im Rahmen der vorliegenden Erfindung, dass zelluläres YB-1 auch ein solches ist, welches erst durch exogene Maßnahmen, wie beispielsweise durch Infektion mit einem Virus, insbesondere mit einem Adenovirus, in die Zelle eingeführt bzw. von dieser produziert wird.

Ohne im folgenden darauf festgelegt sein zu wollen, geht der vorliegende Erfinder davon aus, dass der E2-early Promotor, d. h. der frühe E2-Promotor, im Rahmen der Replikation der hierin erfindungsgemäß verwendeten Viren nicht über den humanen zellulären E2F-Transkriptionsfaktor eingeschaltet wird. Das Einschalten der Replikation ist dabei unabhängig vom Rb-Status der Zellen, d.h. dass die Tumorzellen, die unter Anwendung der hierin offenbarten Viren infiziert und in der Folge bevorzugt lysiert werden, sowohl funktionelle wie auch inaktive Rb-Proteine besitzen können. Zudem benötigt die adenovirale Replikation unter Verwendung der hierin offenbarten Adenoviren bzw. den hierin offenbarten Bedingungen kein funktionelles p53 Protein, wird jedoch auch durch dessen Vorhandensein nicht nachteilig beeinflusst. Insoweit wendet sich die technische Lehre von dem mit der Anwendung der onkolytischen oder tumorlytischen Adenoviren vom Typ AdΔ24, dl922-947, E1Ad/01/07, CB016 oder jener Adenoviren, wie sie beispielsweise im europäischen Patent EP 0 931 830 beschrieben sind, verfolgten Prinzip ab, bei denen eine und/oder mehrere Deletion(en) im E1A-Protein vorgenommen worden war(en) unter der Annahme, dass intakte funktionelle Rb-Proteine einer effizienten Replikation in vivo entgegenwirken und somit eine adenovirale Replikation in vivo nur in Rb-negativen bzw. Rb-mutierten Zellen sicher zu stellen. Diese adenoviralen Systeme nach dem Stand der Technik gehen auf E1A zurück, um mittels des frühen E2-Promotors (E2-early Promotor) und "freiem E2F" die in vivo Replikation von Adenoviren zu steuern. Gleichwohl können diese im Stand der Technik bekannten Viren erfindungsgemäß verwendet werden, d. h. zur Replikation in Zellen, die YB-1 unabhängig vom Zellzyklus im Kern enthalten.

Die in dem besagten europäischen Patent EP 0 931 830 beschriebenen Viren und insbesondere Adenoviren können dabei erfindungsgemäß verwendet werden. Konkret handelt es sich bei den in besagtem Patent beschriebenen Viren um solche, die eine Replikaktionsdefizienz aufweisen und denen ein exprimiertes virales Onkoprotein fehlt, das zur Bindung eines funktionellen Rb-Tumorsuppressor-Genprodukts fähig ist. Der Adenovirus kann dabei insbesondere ein solcher sein, dem exprimiertes virales E1A-Onkoprotein fehlt, das zur Bindung eines funktionellen Tumorsuppressor-Genprodukt, insbesondere Rb, fähig ist. Das virale E1A-Onkoprotein kann dabei eine inaktivierende Mutation aufweisen, beispielsweise in der CR1-Domäne an den Aminosäuren 30 bis 85 in Ad 5, den Nukleotidpositionen 697-790, und/oder der CR2-Domäne an den Aminosäuren 120 bis 139 in Ad 5, den Nukleotidpositionen 920 bis 967, welche an der Bindung von p105 Rb Protein, p130 und p107 Protein beteiligt sind. Dabei kann auch vorgesehen sein, dass der Adenovirus vom Typ 2 dl 312 oder Adenovirus vom Typ 5 NT dl 1010 ist.

Bei der erfindungsgemäßen Verwendung von Adenoviren zur Herstellung eines Medikamentes, insbesondere zur Herstellung eines Medikamentes für die Behandlung von Tumorerkrankungen, und bei der erfindungsgemäßen Verwendung von Adenoviren zur Replikation in Zellen, die YB-1 im Kern aufweisen, erfolgt die Replikation letzten Endes in solchen Zellen, die YB-1 im Kern, bevorzugterweise unabhängig vom Zellzyklus, aufweisen, mithin YB-1-Kern-positiv sind. Dabei ist besonders beachtlich, dass die Adenoviren als solche in Zellen, die YB-1 nicht im Kern, sondern im Wesentlichen nur im Zytoplasma enthalten, nicht oder stark verringert replizieren. Insoweit ist es erforderlich, dass für eine erfolgreiche Replikation dieser Viren YB-1 im Kern vorhanden ist. Dies kann beispielsweise, wie auch im Folgenden noch ausgeführt werden wird, durch Anlegen solcher Bedingungen an die Zellen realisiert werden, dass es zur Expression oder dem Vorhandensein von YB-1 im Kern kommt. Eine entsprechende Maßnahmen kann z. B. die Codierung bzw. Exprimierung von YB-1 durch die erfindungsgemäß verwendeten Adenoviren sein, die in Ergänzung zu den adenoviralen Genen auch eine genetische Information in sich tragen, die für YB-1 und insbesondere dessen Expression codiert. Andere Maßnahmen, die zum Transport, zur Induktion oder Expression von YB-1 im Kern der Zelle führen, sind das Anlegen von Stressbedingungen wie beispielsweise die Applikation von Zytostatika, Bestrahlung, Hyperthermie und dergleichen an die Zelle bzw. den eine - solche - Zelle enthaltenden Organismus.

Die im Rahmen der vorliegenden Erfindung, insbesondere zur Tumorlyse, verwendeten Adenoviren zeichnen sich weiterhin dadurch aus, dass sie in solchen Zellen, die YB-1 im Kern nicht aufweisen, mithin YB-1-Kern-negativ sind, nicht replizieren.

Ein weiteres Merkmal der erfindungsgemäß zu verwendenden Adenoviren besteht darin, dass sie für ein virales Onkoprotein, das hierin auch also Onkogenprotein bezeichnet wird, codieren, wobei es sich bei dem Onkogenprotein um E1A handelt, wobei das Onkogenprotein in der Lage ist, zumindest ein virales Gen zu aktivieren, welches einen Einfluss auf die Replikation des Virus und/oder die Zellyse der von dem Virus infizierten Zelle haben kann. Dabei ist bevorzugt, dass der Einfluss auf die Replikation dergestalt ist, das der Virus bei Vorhandensein des Onkogenproteins besser repliziert als bei Fehlen des Onkogenproteins des jeweilige Virus. Dieser Vorgang wird hierin auch als transaktivierend bezeichnet und insbesondere als E1A-transaktivierend bezeichnet, wenn die Transaktivierung von E1A vermittelt wird. Die Bezeichnung "transaktivieren" oder "Transaktivierung" beschreibt dabei bevorzugt den Vorgang, dass das in Frage stehende virale Onkoprotein auf die Expression und/oder auf die Transkription eines anderen oder mehrerer anderer Gene als das für das virale Onkoprotein selbst codierende Gen Einfluss nimmt, d. h. bevorzugterweise dessen Expression und/oder Translation steuert, und diese(s) insbesondere aktiviert. Derartige virale Gene sind bevorzugter Weise E1B55kDa, E4orf6, E4orf3 und E3ADP sowie beliebige Kombinationen der vorstehend genannten Gene bzw. Genprodukte.

Ein weiteres, wenngleich bevorzugterweise optionales, Merkmal der erfindungsgemäß zu verwendenden Adenoviren ist deren Bindungsverhalten zu bzw. mit dem Tumor-Suppressor Rb. Es ist grundsätzlich im Rahmen der vorliegenden Erfindung, dass die erfindungsgemäß verwendeten Adenoviren an Rb binden oder nicht binden können. Die Verwendung der beiden alternativen Ausgestaltungsformen der Adenoviren ist dabei unabhängig vom Rb-Status der behandelnden Zelle möglich.

Um E1A die Fähigkeit zu verleihen, nicht an Rb binden zu können, sind beispielsweise folgende Deletionen am E1A-Onkoprotein möglich: Deletion in der CR1-Region (Aminosäurepositionen 30-85 in Ad5) und Deletion der CR2-Region (Aminosäurepositionen 120-139 in Ad5). Dabei bleibt die CR3-Region erhalten und kann ihre transaktivierende Funktion auf die anderen frühen viralen Gene ausüben.

Um E1A die Fähigkeit zu verleihen, an Rb binden zu können, sind dagegen folgende Deletionen am E1A-Onkoprotein grundsätzlich möglich: Deletion der CR3-Region (Aminosäurepositionen 140-185); Deletion des N-Terminus (Aminosäurepositionen 1-29); Deletion der Aminosäurepositionen 85-119; und Deletion des C-Terminus (Aminosäurepositionen 186-289). Die hier aufgeführten Bereiche interferieren nicht mit der Bindung von E2F an Rb. Die transaktivierende Funktion bleibt erhalten, ist jedoch gegenüber vom Wildtyp-Ad5 verringert.

Derartige im Stand der Technik grundsätzlich bereits bekannte Viren gelten allgemein als replikationsdefizient. Es ist jedoch das Verdienst des vorliegenden Erfindes, erkannt zu haben, dass sie dennoch zur Replikation in einem geeigneten Hintergrund, insbesondere einem zellulären Hintergrund geeignet sind. Ein derartiger geeigneter zellulärer Hintergrund wird durch das Vorhandensein von YB-1 im Kern, bevorzugterweise eine Zellzyklus-unabhängige Anwesenheit von YB-1 im Kern, bedingt oder bereitgestellt. Der Begriff der Zellen oder zellulären Systeme, wie hierin verwendet, umfasst dabei Fragmente oder Fraktionen von Zellaufschlüssen ebenso wie Zellen, die in vitro, in vivo oder in situ vorliegen. Insoweit umfasst der Begriff zelluläre Systeme oder Zellen auch solche Zellen, die in einer Zellkultur, Gewebekultur, Organkultur oder in einem Gewebe oder Organ in vivo bzw. in situ, isoliert, in Gruppen oder als Teil von Geweben, Organen oder Organismen oder aber auch als solches in einem bevorzugterweise lebenden Organismus vorliegen. Bei dem Organismus handelt es sich bevorzugter Weise um einen Wirbeltier-Organismus und bevorzugterer Weise um ein Säugetier. Besonders bevorzugterweise ist der Organismus dabei ein menschlicher Organismus.

Darüber hinaus ist es im Rahmen der vorliegenden Erfindung, dass auf der Grundlage der hierin gegebenen technischen Lehre neue Viren erzeugt werden, die das Replikationsverhalten der hierin beschriebenen und im Stand der Technik bekannten Adenoviren in solchen Zellen zeigen, die YB-1-Kern-positiv sind. Mit anderen Worten, insbesondere bevorzugterweise ausgehend von den bereits bekannten Adenoviren können weitere Viren konzipiert werden, die die hierin definierten, für die erfindungsgemäße Verwendung erforderlichen Merkmale aufweisen.

Im Zusammenhang mit der vorliegenden Erfindung ist das modifizierte E1A-Onkoprotein der verschiedenen, erfindungsgemäß zu verwendenden Adenoviren in der Lage transaktivierend auf die frühen virale Gene, wie beispielsweise E1B55K, E4orf3, E4orf6, E3ADP, in YB-1 kernpositiven Zellen zu wirken. Dabei liegt sonst bevorzugterweise keine Veränderung im viralen Genom vor und der entsprechende Adenovirus kann insoweit ansonsten einen Adenoviren vom Wildtyp oder Abwandlung davon entsprechen.

Zu den hierin offenbarten Viren, die für ein transaktivierendes Onkogenprotein im Sinne der vorliegenden Erfindung codieren oder ein solches umfassen, gehören beispielsweise die Adenoviren AdΔ24, d1922-947, E1Ad/01/07, CB106 und/oder die im europäischen Patent EP 0931 830 beschriebenen Adenoviren, die jeweils in der Lage sind, transaktivierend auf die frühen Gene z.B. E1B, E2, E3 und/oder E4 zu wirken, und ist vergleichbar mit Adenovirus vom Wildtyp, insbesondere vom Wildtyp Ad5 aufweisen. Verantwortlich für die Transaktivierung ist in diesen Fällen ein bestimmter Bereich des E1A Proteins. Innerhalb von verschiedenen Adenovirus-Serotypen kommen 3 hoch konservierte Bereiche im E1A Protein vor. Der Bereich CR1 von 41-80 AS, CR2 von 120-139 und CR3 von 140-188 AS. Die transaktivierende Funktion beruht hauptsächlich auf das Vorhandensein der CR3-Region im E1A Protein. Die AS-Sequenz von CR3 liegt in den oben genannten Adenoviren unverändert vor. Dies führt dazu, dass die Transaktivierung der frühen Gene E1B, E2, E3 und E4 unabhängig davon erfolgt, ob YB-1 im Kern oder im Zytoplasma vorhanden ist.

Im rekombinanten Adenovirus dl520 ist dahingegen der CR3-Bereich deletiert worden. Damit exprimiert dl520 ein sogenanntes E1A12S Protein, welches nicht die AS-Sequenz der CR3-Region aufweist. Dies führt dazu, dass dl520 nur eine ganz schwache transaktivierende Funktion ausüben kann, insbesondere auf die E2-Region, und somit in YB-1 Kern-negativen Zellen nicht repliziert. In YB-1 Kern-positiven Zellen übernimmt YB-1 die Transaktivierung der E2-Region, und ermöglicht somit eine effiziente Replikation dl520. Darauf beruht die Verwendung von Systemen wie dl520 bzw. auf der Grundlage von dl520 zu den hierin offenbarten Zwecken. Ein weiterer wichtiger Unterschied zwischen beiden vorstehend beschriebenen Gruppen von Adenoviren, d. h. delta 24 (hierin auch als AdΔ24 bezeichnet) und dl520 besteht darin, dass bei dl520 die frühen Gene E1B, E3 und E4 stärker in YB-1 Kern-positiven Zellen als in YB-1 Kern-negativen Zellen transaktiviert werden. Bei delta 24 bestehen hingegen keine bzw. nur geringfügige Unterschiede. Die Transaktivierung von dl520 bzw. genauer des E1A12S Proteins ist allerdings erheblich schwächer im Vergleich zum Wildtyp-Adenovirus. Diese Transaktivierung reicht allerdings aus, um eine effiziente Replikation in YB-1 kernpositiven Zellen durchzuführen, wie dies auch in Beispiel 10 gezeigt ist. Die hierin und speziell in diesem Zusammenhang beschriebene Ausgestaltung des E1A-Proteins bzw. der dafür codierenden Nukleinsäure in der Form, dass das E1A-Protein gegenüber dem Wildtyp-Onkogenprotein E1A eine oder mehrere Deletionen und/oder Mutationen aufweist, wobei die Deletion bevorzugt eine solche ist, die ausgewählt ist aus der Gruppe, die Deletionen des Bereiches CR3 und Deletionen des N-Terminus und Deletionen des C-Terminus umfasst, einschließlich und besonders bevorzugt jener Ausgestaltungen des E1A-Proteins wie im Zusammenhang mit dl520 oder AdΔ24, dl922 bis 947, E1Ad/01/07, CB106 und/oder die im europäischen Patent EP 0 931 830 beschriebenen Adenoviren, stellen Ausführungsformen von Viren, insbesondere von Adenoviren dar, deren Replikation durch YB-1 über die Aktivierung des E2-late-Promotors gesteuert wird, bevorzugterweise überwiegend über die Aktivierung des E2-late-Promotors. Weitere Ausgestaltungen des E1A-Proteins, die diese Form der Replikation von Adenoviren erlauben, können auf der Grundlage der hierin gemachten Offenbarung von den Fachleuten auf dem Gebiet hergestellt werden.

Bei weiteren, neu zu konstruierenden rekombinanten Adenoviren, die hierin auch als Abwandlungen bezeichnet werden und die erfindungsgemäß verwendet werden können, liegt typischerweise eine E1-Deletion, eine E1/E3-Deletion und/oder eine E4-Deletion vor, d. h. die ensprechenden Adenoviren sind nicht in der Lage, funktional aktive E1- und/oder E3- und/oder E4-Expressionsprodukte bzw. entsprechende Produkte zu erzeugen, oder mit anderen Worten, diese Adenoviren lediglich in der Lage sind, funktional inaktive E1-, E3-und/oder E4-Expressionsprodukt zu erzeugen, wobei ein funktional inaktives E1-, E3- und/oder E4-Expressionsprodukt ein solches, welches entweder gar nicht als Expressionsprodukt, sei es auf der Ebene der Transkription und/oder der Translation, oder in einer Form vorliegt, bei der es zumindest eine der ihm im Adenovirus vom Wildtyp zukommende Funktion nicht aufweist. Diese dem Expressionsprodukt im Adenovirus vom Wildtyp zukommenden Funktion(en) ist/sind den Fachleuten auf dem Gebiet bekannt und beispielsweise beschrieben in Russell, W. C., Journal of Virology, 81, 2573-2604, 2000. Russell (a.a.O.) beschreibt im übrigen auch Prinzipien der Konstruktion von Adenoviren und adenoviralen Vektoren. Es ist auch im Rahmen der vorliegenden Erfindung, dass das modifizierte E1A-Onkoprotein, E1B-55K, E4orf6 und/oder E3ADP (adenoviral death protein (ADP)) (Tollefson, A. et al., J. Virology, 70, 2296-2306, 1996.) in einem solchen Vektor einzeln oder in beliebiger Kombination zur Expression gebracht wird/werden. Dabei können die einzelnen genannten Gene ebenso wie die hierin offenbarten Transgene unabhängig von einander entweder in der E1 und /oder E3 und/oder E4-Region einkloniert und mit Hilfe eines geeigneten Promoters oder unter Kontrolle eines geeigneten Promotors zur Expression gebracht werden. Grundsätzlich sind die Regionen E1, E3 und E4 als Klonierungsstellen innerhalb der adenoviralen Nukleinsäure gleichermaßen geeignet. Geeignete Promotoren sind u. a. jene, wie sie hierin im Zusammenhang mit der Steuerung bzw. Expression von E1A, insbesondere des modifizierten E1A, offenbart sind.

Schließlich ist in einer Ausführungsform vorgesehen, dass die erfindungsgemäß verwendeten Adenoviren hinsichtlich E1B defizient, insbesondere hinsichtlich E1B 19 kDa-defizient sind. Dabei wird hierin allgemein unter dem Begriff defizient ein Zustand verstanden, bei dem E1B die im Wildtyp inhärente Gesamtheit der Eigenschaften nicht aufweist und mindestens eine dieser Eigenschaften fehlen.

Die Adenoviren, wie sie gemäß der hierin offenbarten Erfindung verwendet werden, sind grundsätzlich im Stand der Technik zumindest in einigen Ausführungsformen bekannt. Bei den erfindungsgemäß verwendeten Adenoviren handelt es sich bevorzugter Weise um rekombinante Adenoviren, insbesondere auch dann, wenn eine Veränderung gegenüber dem Wildtyp vorgenommen wurde im Sinne der hierin gegebenen technischen Lehre. Es ist im Rahmen der Kenntnisse der Fachleute auf diesem Gebiet, für die Erfindung unwesentliche adenoviralen Nukleinsäuresequenzen zu deletieren bzw. zu mutieren. Derartige Deletionen können z. B die für einen Teil der E3 und E4 codierende Nukleinsäure betreffen wie hierin auch beschrieben. Bei einer Deletion von E4 ist dabei besonders bevorzugt, wenn sich diese nicht auf das Protein E4orf6 erstreckt, mithin der erfindungsgemäß zu verwendende Adenovirus E4orf6 codiert. In bevorzugten Ausführungsformen können diese adenoviralen Nukleinsäuren noch in das virale Kapsid verpackt werden und damit infektiöse Partikel ausbilden. Gleiches gilt für die erfmdungsgemäße Verwendung der Nukleinsäuren. Generell gilt es auch noch festzuhalten, dass die adenoviralen Systeme hinsichtlich einzelner oder mehrerer Expressionsprodukte defizient sein können. Dabei ist zu berücksichtigen, dass dies zum einen darauf beruhen kann, dass die für das Expressionsprodukt codierende Nukleinsäure vollständig oder in dem Maße mutiert oder deletiert ist, dass im wesentlichen kein Expressionsprodukt mehr gebildet wird, oder darauf, dass regulative bzw. die Expression steuernde Elemente wie Promotoren oder Transkriptionsfaktoren fehlen oder anders als im Wildtyp aktiv sind, sei es auf der Ebene der Nukleinsäure (Fehlen eines Promotors; cis-wirkende Element) oder auf der Ebene des Translations- bzw. Transkriptionssystems (trans-wirkende Elemente). Gerade der letztere Aspekt kann dabei vom jeweiligen zellulären Hintergrund abhängen.

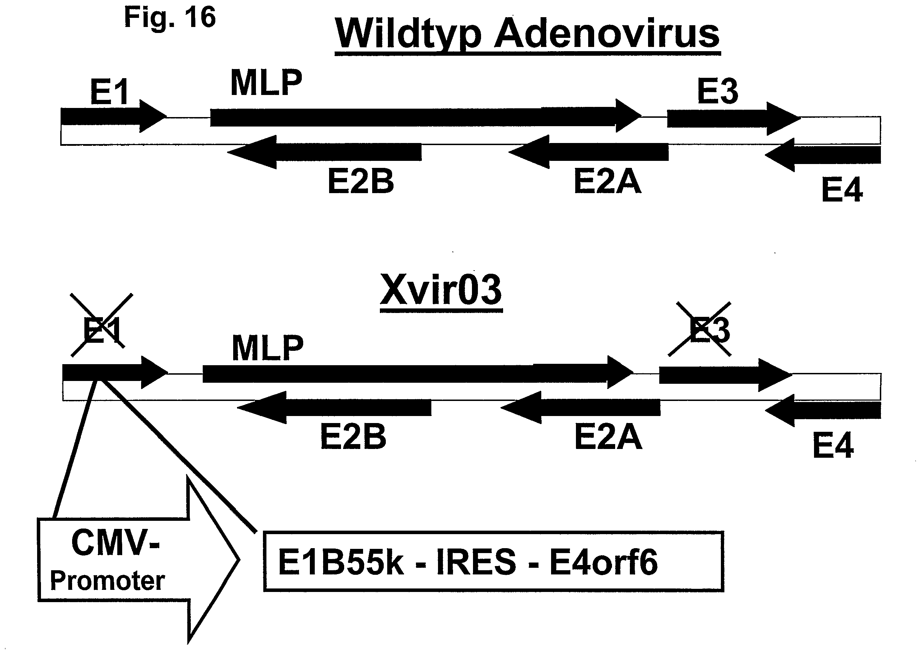

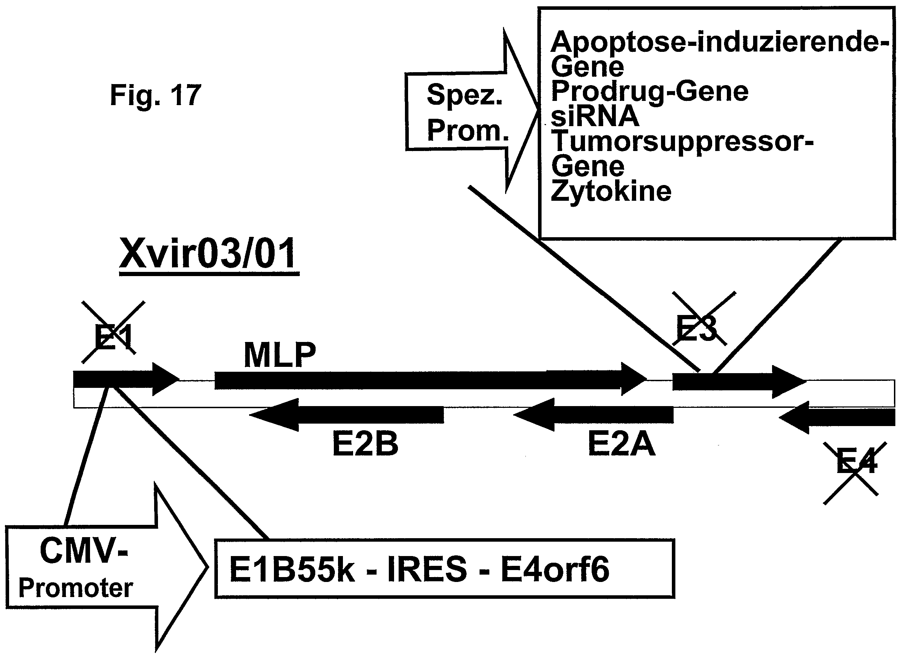

Neben der Verwendung von an und für sich bereits bekannten Adenoviren gemäß der vorliegenden Erfindung können auch neue Adenoviren in dem Umfang verwendet werden, wie dies für die anderen hierin beschriebenen Adenoviren bereits offenbart ist. Die erfindungsgemäßen neuen Adenoviren ergeben sich aus der hierin offenbarten technischen Lehre. Besonders bevorzugte Vertreter sind dabei beispielsweise die hierin in Fig. 16 und Fig. 17 dargestellten Viren Xvir03 und Xvir03/01, deren Konstruktionsprinzip auch in den Beispielen 11 und 12 weiter veranschaulicht ist.

Im Falle des Vektors Xvir03 wurde in der E1-Region ein CMV-Promotor kloniert, der die durch eine IRES-Sequenz getrennten Nukleinsäuren für E1B 55k und E4ORF6 codiert. Infolge Einklonierung dieser beiden Gene bzw. der daraus hergestellten Genprodukte kommt es zu einer Replikationseffizienz, die praktisch derjenigen von Wildtyp-Viren entspricht, wobei die Selektivität der Replikation in Zellen, insbesondere Tumorzellen, insoweit beibehalten wird, als dass eine Replikation, insbesondere in YB-1-Kern-positiven Zellen und insbesondere in solchen Zellen erfolgt, bei denen YB-1 dereguliert vorliegt. Zellen, in denen YB-1 dereguliert vorliegt, sind dabei bevorzugt solche, die eine erhöhte Expression von YB-1, bevorzugt Kompartimentunabhängig, aufweisen verglichen mit normalen oder Nicht-Tumorzellen.

Eine Weiterentwicklung des Virus Xvir03 stellt der Virus Xvir03/01 dar, bei dem unter der Kontrolle eines spezifischen Promotors, insbesondere eines Tumor- oder Gewebe-spezifischen Promotors, therapeutische Gene oder Transgene in einer bevorzugten Ausführungsform einkloniert sind. Weiterhin ist es im Rahmen eines derartigen Virus, dass auch die Region E4 funktional inaktiv ist, bevorzugterweise deletiert ist. Die hierin beschriebenen Transgene können dabei auch in die E4-Region kloniert werden, wobei dies alternativ oder ergänzend zum Klonieren der Transgene in die E3-Region erfolgen kann.

Derartige therapeutische Gene können dabei Prodrug-Gene, Gene für Zytokine, Apoptose-induzierende Gene, Tumorsuppressorgene, Gene für Metalloproteinasen-Inhibitoren und/oder Angiogenese-Inhibitoren sein. Ferner können siRNA, Aptameren, Antisense und Ribozyme exprimiert werden die gegen Krebs-relevante Zielmoleküle gerichtet sind. Bevorzugterweise ist das einzelne oder die mehreren Zielmoleküle aus der Gruppe ausgewählt, die Resistenz-relevante Faktoren, Anti-Apoptose-Faktoren, Onkogene, Angiogenese-Faktoren, DNA-Synthese-Enzyme, DNA-Reperaturenzyme, Wachstumsfaktoren und deren Rezeptoren, Transkriptionsfaktoren, Metalloproteinasen, insbesondere Matrix-Metalloproteinasen, und Plasminogenaktivator vom Urokinase-Typ umfasst. Bevorzugte Ausführungsformen davon sind hierin bereits offenbart.

Mögliche Prodrug-Gene, wie sie in bevorzugten Ausführungsformen verwendet werden können, sind beispielsweise *Cytosine deaminase, Thymidin kinase, Carboxypeptidase, Uracil phosphoribosyltransferase; Purin Nukleosid Phosphorylase (PNP);* Kirn et al, Trends in Molecular Medicine, Volume 8, No.4 (Suppl), 2002; Wybranietz W.A. et al., Gene Therapy, 8, 1654-1664, 2001; Niculescu-Duvaz et al., Curr. Opin. Mol. Therapy, 1, 480.486, 1999; Koyama et al., Cancer Gene Therapy, 7, 1015-1022, 2000; Rogers et al., Human Gene Therapy, 7, 2235-2245, 1996; Lockett et al., Clinical Cancer Res., 3, 2075-2080, 1997; Vijayakrishna et al., J. Pharmacol. And Exp. Therapeutics, 304, 1280-1284, 2003.

Mögliche Zytokine, wie sie in bevorzugten Ausführungsformen verwendet werden können, sind beispielsweise *GM-CSF, TNF-alpha, Il-12, Il-2, Il-6, CSF, Interferon-Gamma;* Gene Therapy, Advances in Pharmacology, Volume 40, Editor: J. Thomas August, Academic Press; Zhang und Degroot, Endocrinology, 144, 1393-1398, 2003; Descamps et al., J. Mol. Med., 74, 183-189, 1996; Majumdar et al., Cancer Gene Therapy, 7, 1086-1099, 2000.

Mögliche Apoptose-induzierende Gene, wie sie in bevorzugten Ausführungsformen verwendet werden können, sind beispielsweise Decorin: Tralhao et al., FASEB J, 17, 464-466, 2003; Retinoblastoma 94: Zhang et al., Cancer Res.,63, 760-765, 2003; Bax und Bad: Zhang et al, Hum. Gene Ther., 20, 2051-2064, 2002; Apoptin: Noteborn und Pietersen, Adv. Exp. Med. Biol., 465, 153-161, 2000); ADP: Toth et al., Cancer Gene Therapy, 10, 193-200, 2003; bcl-xs: Sumantran et al., Cancer Res, 55, 2507-2512, 1995; E4orf4: Braithwaite und Russell, Apoptosis, 6, 359-370, 2001; FasL, Apo-1 und Trail: Boehringer Manheim, Guide to Apoptotic Pathways, Arai et al., PNAC, 94, 13862-13867, 1997; Bims; Yamaguchi et al., Gene Therapy, 10, 375-385, 2003; GNR163: Oncology News, 17 Juni, 2000, sind.

Mögliche Tumorsuppressor-Gene, wie sie in bevorzugten Ausführungsformen verwendet werden können, sind beispielsweise *E1A, p53, p16, p21, p27, MDA-7.* Opalka et al., Cell Tissues Organs, 172, 126-132, 2002, Ji et al., Cancer Res., 59, 3333-3339, 1999, Su et al., Oncogene, 22, 1164-1180, 2003.

Mögliche Angiogenese-Inhibitoren, wie sie in bevorzugten Ausführungsformen verwendet werden können, sind beispielsweise *Endostatin, angiostatin* : Hajitou et al., FASEB J., 16, 1802-1804, 2002, und Antikörper gegen VEGF (Ferrara, N., Semin Oncol 2002 Dec; 29 (6 Suppl 16): 10-4.

Mögliche Metalloproteinase-Inhibitoren, wie sie in bevorzugten Ausführungsformen verwendet werden können, sind beispielsweise *Timp-3,* Ahonen et al., Mol Therapy, 5, 705-715, 2002; *PAI-1;* Soff et al., J. Clin. Invest., 96, 2593-2600, 1995 ; *Timp-1,* Brandt K. Curr. Gene Therapy, 2, 255-271, 2002.

siRNA (short interfering RNA) besteht aus zwei, bevorzugt zwei getrennten RNA-Strängen, die infolge Basenkomplementarität miteinander hybridisieren, d.h. im wesentlichen basengepaart vorliegen, und weist bevorzugterweise eine Länge von bis zu 50 Nukleotiden auf, bevorzugterweise zwischen 18 und 30 Nukleotiden, bevorzugtererweise weiniger als 25 Nukleotide und am bevorzugtesten 21, 22 oder 23 Nukleotide, wobei sich diese Zahlenwerte auf einen Einzelstrang der siRNA, insbesondere auf die Länge des Bereiches eines Einzelstranges, der mit einem, genauer dem zweiten Einzelstrang hybridisiert bzw. basenpaart, beziehen. siRNA induziert oder vermittelt spezifisch den Abbau von mRNA. Die dazu erforderliche Spezifität wird durch die Sequenz der siRNA und damit ihren Bindungsort vermittelt. Die abzubauende Zielsequenz ist dabei im wesentlichen komplementär zu dem ersten oder zu dem zweiten die siRNA aufbauenden Strang. Obwohl die genauen Wirkmechanismen noch unklar sind, wird vermutet, dass siRNA für Zellen eine biologische Strategie darstellt, während der Entwicklung bestimmte Allele zu hemmen und sich vor Viren zu schützen. Die durch siRNA vermittelte RNA-Interferenz wird als Verfahren zur spezifischen Unterdrückung oder gar völligen Ausschaltung der Expression eines Proteins durch Einbringen einer genspezifischen doppelsträngigen RNA benutzt. Für höhere Organismen ist eine 19 bis 23 Nukleotid lange siRNA deshalb besonders geeignet, weil sie nicht zur Aktivierung der unspezifischen Abwehrreaktion, zur sogenannten Interleukin-Antwort führt. Die direkte Transfektion von doppelsträngiger RNA aus 21 Nukleotiden mit symmetrischen 2-nt 3' Überhängen konnte eine RNA-Interferenz in Säugetierzellen vermitteln und zeigte im Vergleich zu anderen Technologien wie Ribozymen und Antisense-Molekülen eine hohe Effizienz (Elbashir, S. Harborth J. Lendeckel W. Yalvcin, A. Weber K Tuschl T: Duplexes of 21-nucleotide RNAs mediate RNA interference in cultured mammalian cells. Nature 2001, 411: 494-498). Nur wenige siRNA-Moleküle genügten, um die Expression des Zielgens zu unterdrücken. Um die Limitierungen exogen zugeführter siRNA, die insbesondere in der transienten Natur des Interferenz-Phänomens und der spezifischen Abgabe (engl. delivery) der siRNA-Moleküle liegen, zu umgehen, werden im Stand der Technik auch Vektoren eingesetzt, die eine endogene siRNA-Expression erlauben. Hierfür werden beispielsweise Oligonukleotide mit einer Länge von 64 Nukleotiden, die die 19 Nukleotide lange Zielsequenz einschließen, sowohl in sense- als auch in antisense- Orientierung, getrennt durch eine beispielsweise 9 Nukleotide lange Spacer-Sequenz, in den Vektor eingebaut. Das resultierende Transkript faltet sich zu einer Haarnadelstruktur mit einer Stammstruktur (engl.: stem) von beispielsweise 19 Basenpaaren. In der Zelle wird die Schleife rasch abgespaltet, so daß eine funktionelle siRNA entsteht (Brummelkamp et al., Science, 296, 550-553, 2002)

Die Aktivität von pRb bzw. E2F wird durch Phosphorylierung reguliert. Die hypophosphorylierte Form von pRb tritt hauptsächlich in der G1- und M-Phase auf. Dagegen tritt die hyperphosphorylierte Form von pRb in der S- und G2-Phase auf. Durch die Phosphorylierung von pRb wird E2F aus dem Komplex aus E2F und hypophosphoryliertem pRb freigesetzt. Die Freisetzung von E2F aus dem Komplex aus E2F und hypophosphoryliertem pRb führt zur Transkription von E2F-abhängigen Genen. Das E1A-Protein bindet nur an die hypophosphorylierte Form des pRb, wobei die Bindung von E1A an pRb hauptsächlich über die CR2-Region des E1A-Proteins erfolgt. Zudem bindet es auch an die CR1-Region, allerdings mit einer schwächeren Affinität (Ben-Israel and Kleiberger, Frontiers in Bioscience, 7, 1369-1395, 2002; Helt und Galloway, Carcinogenesis, 24, 159-169, 2003).

Die für YB-1 codierende Nukleinsäure, die in einer Ausführungsform der erfindungsgemäß zu verwendenden Adenoviren Bestandteil der Adenoviren sein kann, kann dabei eine einen Kerntransport von YB-1 vermittelnde Nukleinsäuresequenz umfassen. Als Adenoviren bzw. adenovirale Systeme und damit die entsprechenden Nukleinsäuren können im Zusammenhang damit und in Kombination mit diesen erfindungsgemäßen Nukleinsäuren die erfindungsgemäßen Nukleinsäuren, Adenoviren und adenoviralen Systeme sowie die im Stand der Technik bekannten Adenoviren wie beispielsweise Onyx-015, AdΔ24, dl922-947, E1Ad/01/07, CB016, dl 520 und die im Patent EP 0931 830 beschriebenen Adenoviren verwendet werden. Geeignete, den Kerntransport vermittelnde Nukleinsäuresequenzen sind den Fachleuten auf dem Gebiet bekannt und beispielsweise beschrieben in (Whittaker, G.R. et al., Virology, 246, 1-23, 1998; Friedberg, E.C., TIBS 17, 347, 1992; Jans, D.A. et al., Bioessays 2000 Jun; 22(6): 532-44; Yoneda, Y., J. Biocehm. (Tokyo) 1997 May; 121(5): 811-7; Boulikas, T., Crit. Rev. Eukaryot. Gene Expr. 1993; 3(3): 193-227; Lyons RH, Mol. Cell Biol., 7, 2451-2456, 1987). Bei den Kerntransport vermittelnden Nukleinsäuresequenzen können verschiedene Prinzipien verwendet werden. Ein derartiges Prinzip besteht beispielsweise darin, dass, dass YB-1 als Fusionsprotein mit einem Signalpeptid ausgebildet wird und infolge des Signalpeptids YB-1 in den Zellkern geschleust wird und damit die erfindungsgemäße Replikation der Adenoviren erfolgt.

Ein weiteres Prinzip, welches bei der Ausgestaltung der erfindungsgemäß verwendeten Adenoviren zur Anwendung gelangen kann, besteht darin, dass YB-1 mit einer Transportsequenz versehen wird, die dazu führt, dass YB-1, bevorzugter Weise ausgehend von einer Synthese im Cytoplasma, in den Zellkern geschleust oder transloziert wird und dort die virale Replikation befördert. Ein Beispiel für eine besonders wirksame, den Kerntransport vermittelnde Nukleinsäuresequenz stellt die TAT-Sequenz von HIV dar, die neben weiteren geeigneten derartigen Nukleinsäuresequenzen beispielsweise beschrieben ist in Efthymiadis, A., Briggs, LJ, Jans, DA., JBC 273, 1623-1628, 1998. Dabei ist es Rahmen der vorliegenden Erfindung, dass die erfindungsgemäß verwendeten Adenoviren die Nukleinsäuresequenzen umfassen, die für die den Kerntransport codierenden Peptide codieren.

Es ist im Rahmen der vorliegenden Erfindung, dass YB-1 in vollständiger Länge vorliegt, insbesondere in einer Form, die dem Wildtyp von YB-1 entspricht. Es ist weiterhin im Rahmen der vorliegenden Erfindung, dass YB-1 als Derivat, zum Beispiel, in verkürzter oder trunkierter Form verwendet wird oder vorliegt. Ein YB-1-Derivat, wie es im Rahmen der vorliegenden Erfindung verwendet wird, oder vorliegt, ist dabei ein solches, das an den E2 late-Promotor zu binden in der Lage ist und dadurch die Genexpression von der adenoviralen E2-Region aktiviert. Derartige Derivate umfassen insbesondere die hierin offenbarten YB-1-Derivate. Weitere Derivate können durch Deletion einzelner oder mehrerer Aminosäuren am N-Terminus, am C-Terminus oder innerhalb der Aminosäuresequenz erzeugt werden.

Hinsichtlich der vorstehend genannten verschiedenen weiteren, von den Adenoviren codierten bzw. exprimierten Genen und Genprodukten ist es auch möglich, dass diese in beliebiger Kombination codiert bzw. exprimiert werden.

Im Rahmen der vorliegenden Erfindung sollen hierin die Begriffe Adenovirus und adenovirale Systeme als im wesentlichen die gleiche Bedeutung aufweisend verstanden werden. Unter Adenovirus soll dabei insbesondere das vollständige Viruspartikel verstanden werden umfassend das Kapsid und die Nukleinsäure. Der Begriff adenovirales System stellt insbesondere darauf ab, dass die Nukleinsäure gegenüber dem Wildtyp verändert ist. Bevorzugt umfassen derartige Änderungen solche im Aufbau des Genoms des Adenovirus wie sie durch Deletieren und/oder Hinzufügen und/oder Mutieren von Promotoren, regulativen Sequenzen und/oder codierende Sequenzen wie beispielsweise Leserahmen entstehen. Der Begriff adenovirale Systeme wird darüber hinaus bevorzugt in dem Zusammenhang verwendet, dass es sich dabei um einen Vektor handelt, der beispielsweise in der Gentherapie verwendet werden kann.

Die vorstehend gemachten Ausführungen, einschließlich jeglicher Verwendungen sowie die Ausbildungen der Adenoviren bzw. adenoviralen Systeme gelten im gleichen Maße für die dafür codierenden Nukleinsäuren und umgekehrt.

Im Zusammenhang mit der vorliegenden Erfindung ist es möglich, dass die erfindungsgemäß verwendeten Adenoviren bzw. die für sie codierenden Nukleinsäuren eine jede entsprechende adenovirale Nukleinsäure ist, die zu einem Replikationsereignis für sich oder in Verbindung mit weiteren Nukleinsäuresequenzen führt. Dabei ist es möglich, wie hierin ausgeführt, dass mittels Helferviren die für die Replikation erforderlichen Sequenzen und/oder Genprodukte bereitgestellt werden. Sofern hierin auf codierende Nukleinsäuresequenzen Bezug genommen wird und es sich dabei um solche Nukleinsäuresequenzen handelt, die bekannt sind, ist es im Rahmen der Erfindung, dass nicht nur die identische Sequenz verwendet wird, sondern auch hiervon abgeleitete Sequenzen. Unter abgeleiteten Sequenzen sollen hierin insbesondere solche Sequenzen verstanden sein, die noch zu einem Genprodukt, sei es eine Nukleinsäure oder ein Polypeptid, führen, das eine Funktion aufweist, die einer oder der Funktion der nicht abgeleiteten Sequenz entspricht. Dies kann durch einfache, dem Fachmann geläufige Routinetests festgestellt werden. Ein Beispiel für derartige abgeleitete Nukleinsäuresequenzen sind jene Nukleinsäuresequenzen, die für das gleiche Genprodukt, insbesondere für die gleiche Aminosäuresequenz kodieren, jedoch infolge der Degeneriertheit des genetischen Codes eine andere Basenabfolge aufweisen.

Hinsichtlich des erfindungsgemäßen Adenovirus und/oder des erfindungsgemäßen adenoviralen Replikationssystems, bzw. deren erfindungsgemäße Verwendung ist dabei in einer Ausführungsform vorgesehen, dass die adenovirale Nukleinsäure für die Expression des Onkogenproteins, insbesondere des E1A-Proteins defizient ist, d.h. entweder für das 12S E1A-Protein nicht codiert oder für das 13S E1A-Protein nicht codiert oder für sowohl das 12S E1A-Protein als auch das 13S E1A-Protein nicht codiert, oder modifiziert ist, wie hierin definiert, und das adenovirale Replikationssystem weiter eine Nukleinsäure eines Helfervirus umfasst, wobei die Nukleinsäure des Helfervirus eine Nukleinsäuresequenz umfasst, die für das Onkogenprotein, insbesondere das E1A-Protein codiert, welches die folgenden Eigenschaften aufweist bzw. dem Adenovirus die folgenden Eigenschaften verleiht, nämlich dass dieser bevorzugterweise nicht replizierend in YB-Kern-negativen Zellen aber sehr wohl in vom Zellzyklus unabhängig YB-1-Kern-positiven Zellen replizierend ist, transaktivierend auf mindestens ein virales Gen, insbesondere E1B55kDa, E4orf6, E4orf3 und/oder E3ADP, in YB-1 kernpositiven Zellen wirkt, und/oder zelluläres YB-1 nicht in den Kern transloziert. Es ist im Rahmen der vorliegenden Erfindung, dass die hierin beschriebenen Transgene einzeln oder gemeinsam von dem Helfervirus codiert und/oder exprimiert werden.

Weiterhin ist bei einem derartigen erfindungsgemäßen adenoviralen Replikationssystem in einer Ausführungsform vorgesehen, dass die adenovirale Nukleinsäure und/oder die Nukleinsäure des Helfervirus als replizierbarer Vektor vorliegt.

Dabei ist es weiter im Rahmen der vorliegenden Erfindung, dass die für die Adenoviren, wie sie erfindungsgemäß verwendet werden, codierende Nukleinsäure(n) in einem Vektor, bevorzugter Weise in einem Expressionsvektor vorliegt/vorliegen und dieser Expressionsvektor erfindungsgemäß verwendet wird.

In einem weiteren Aspekt betrifft die vorliegende Erfindung auch eine Vektorgruppe umfassend mindestens zwei Vektoren, wobei die Vektorgruppe insgesamt ein adenovirales Replikationssystem umfasst, wie hierin beschrieben, und die Vektorgruppe erfindungsgemäß verwendet wird. Dabei kann vorgesehen sein, dass eine jede Komponente des adenoviralen Replikationssystems auf einem eigenen Vektor, bevorzugter Weise einem Expressionsvektor angeordnet ist.

Schließlich betrifft die vorliegende Erfindung in einem weiteren Aspekt auch die Verwendung einer Zelle, die eine oder mehrere der Nukleinsäuren, wie sie für die erfindungsgemäße Verwendung der hierin beschriebenen Adenoviren, die erfindungsgemäß verwendet werden sollen, codiert, und/oder ein entsprechendes adenovirales Replikationssystem und/oder einen entsprechenden Vektor und/oder eine erfindungsgemäße Vektorgruppe umfasst, zu denselben Zwecken, wie hierin für die Adenoviren beschrieben

Die vorstehend beschriebenen Konstrukte von Adenoviren und insbesondere deren Nukleinsäuren bzw. die dafür codierenden Nukleinsäuren können auch in eine Zelle, insbesondere eine Tumorzelle, in Teilen eingebracht werden, wobei dann infolge der Anwesenheit der verschiedenen Einzelkomponenten diese so zusammenwirken, als stammten die Einzelkomponenten von einer einzelnen Nukleinsäure bzw. einem einzelnen oder mehreren Adenoviren.

Die erfindungsgemäß verwendeten, für Adenoviren, adenovirale Systeme oder Teile davon codierenden Nukleinsäuren können als Vektoren vorliegen.

Bevorzugter Weise handelt es sich um virale Vektoren. Im Falle der adenovirale Nukleinsäuren umfassenden Nukleinsäuren ist das Viruspartikel dabei bevorzugterweise der Vektor. Es ist jedoch auch im Rahmen der vorliegenden Erfindung, dass die besagten Nukleinsäuren in einem Plasmidvektor vorliegen. In einem jeden Fall weist der Vektor Elemente auf, die für die Vermehrung der inserierten Nukleinsäure, d. h. Replikation und ggf. Expression der inserierten Nukleinsäure sorgen bzw. diese steuern. Geeignete Vektoren, insbesondere auch Expressionsvektoren, und entsprechende Elemente sind den Fachleuten auf dem Gebiet bekannt und beispielsweise beschrieben in Grunhaus, A., Horwitz, M.S., 1994, Adenoviruses as cloning vectors. In Rice, C., Hrsg., Seminars in Virology, London: Saunders Scientific Publications.

Der oben beschriebenen Ausführungsform, dass die verschiedenen Elemente der besagten Nukleinsäure nicht notwendigerweise auf nur einem Vektor enthalten sein müssen, trägt der Aspekt der Erfindung Rechnung, der die Vektorgruppe betrifft. Eine Vektorgruppe umfasst entsprechend mindestens zwei Vektoren. Ansonsten gilt betreffend die Vektoren bzw. die Vektorengruppe das hierin allgemein zu Vektoren Ausgeführte.

Die erfindungsgemäß verwendeten Adenoviren sind durch die verschiedenen hierin offenbarten Nukleinsäuren bzw. Genprodukte charakterisiert und können ansonsten all jene den Fachleuten auf dem Gebiet bekannten Elemente umfassen, wie dies auch bei Adenoviren vom Wildtyp der Fall ist (Shenk, T.: Adenoviridae: The virus and their replication. Fields Virology, 3. Auflage, Hrsg. Fields, B.N., Knipe, D.M., Howley, P.M. et al., Lippincott-Raven Publishers, Philadelphia, 1996, Kapitel 67).

Die Replikation von Adenoviren ist ein ausgesprochen komplexer Vorgang und greift im Regelfalle auf den humanen Transkriptionsfaktor E2F zurück. Während einer viralen Infektion werden zunächst die "frühen Gene" E1, E2, E3 und E4 exprimiert. Die Gruppe der "späten Gene" ist für die Synthese der viralen Strukturproteine verantwortlich. Für die Aktivierung sowohl der frühen wie auch der späten Gene spielt die E1-Region bestehend aus zwei Transkriptionseinheiten E1A und E1B, welche für verschiedene E1A- und E1B-Proteine codieren, eine entscheidende Rolle, da sie die Transkription der E2, E3, E4-Gene induzieren (Nevins, J. R., Cell 26, 213-220, 1981). Zudem können die E1A-Proteine in ruhenden Zellen die DNA-Synthese induzieren und so deren Eintritt in die S-Phase einleiten (siehe Boulanger and Blair, 1991). Darüber hinaus interagieren sie mit den Tumorsuppressoren der Rb-Klasse (Whyte, P. et al., Nature 334, 124-127, 1988). Dabei wird der zelluläre Transkriptionsfaktor E2F freigesetzt. Die E2F-Faktoren können dann an die entsprechenden Promotorbereiche sowohl zellulärer wie auch viraler Gene binden (insbesonder an den adenoviralen E2 early Promotor) und die Transkription und somit die Replikation einleiten (Nevins, J.

R., Science 258, 424-429, 1992).

Für das Einleiten bzw. die Durchführung der Replikation werden insbesondere die Genprodukte der E2-Region benötigt, da sie für drei essentielle Proteine kodieren. Die Transkription der E2-Proteine wird durch zwei Promotoren gesteuert, den "E2-Early E2F-abhängigen", hierin auch als E2-early Promotor oder früher E2-Promotor bezeichnet, und den "E2-late" Promoter (Swaminathan und Thimmapaya, The Molecular Repertoire of Adenoviruses III: Current Topics in Microbiology and Immunology, Vol 199, 177-194, Springer Verlag 1995). Zudem spielen die Produkte der E4-Region zusammen mit dem E1A- und E1B-55kDa-Protein eine wichtige Rolle für die Aktivität von E2F bzw. die Stabilität von p53. Zum Beispiel wird durch eine direkte Interaktion des von der E4-Region codierten E4orf6/7-Proteins mit dem Heterodimer bestehend aus E2F und DP1 der E2-Promoter noch stärker transaktiviert (Swaminathan und Thimmapaya, JBC 258, 736-746, 1996). Weiterhin wird p53 durch den Komplex bestehend aus E1B-55kDa und E4orf6 inaktiviert (Steegenga, W. T. et al., Oncogene 16, 349-357, 1998), um einen erfolgreichen lytischen Infektionszyklus durchlaufen zu können. Ferner besitzt das E1B-55kDa Protein eine weitere wichtige Funktion insoweit, als dass es in Wechselwirkung mit dem E4orf6 Protein den Export der viralen RNA aus dem Zellkern fördert, wohingegen die zelleigenen RNAs im Kern zurückgehalten werden (Bridge und Ketner, Virology 174, 345-353, 1990). Eine weitere wichtige Beobachtung ist die, dass der Proteinkomplex bestehend aus E1B-55kDa/E4orf6 in den sogenannten "viral inclusion bodies" lokalisiert ist. Es wird angenommen, dass diese Strukturen Orte der Replikation und Transkription darstellen (Ornelles und Shenk, J. Virology 65, 424-429, 1991).

Eine weitere für die Replikation und insbesondere für die Freisetzung von Adenoviren besonders wichtige Region ist die E3-Region. Die E3-Region enthält genauer die genetische Information für eine Vielzahl von relativ kleinen Proteinen, die für den adenoviralen Infektionszyklus in vitro, d.h. in der Zellkultur nicht essentiell sind. Sie spielen jedoch für das Überleben des Virus während einer akuten und/oder latenten Infektion in vivo eine bedeutende Rolle, da sie unter anderem immunregulatorische und apoptotische Funktion(en) besitzen (Marshall S. Horwitz, Virololgie, 279, 1-8, 2001; Russell, a.a.O.). Es konnte gezeigt werden, dass ein Protein mit einer Größe von ca. 11,6 kDa den Zelltod induziert. Das Protein wurde infolge seiner Funktion als ADP - für den englischen Begriff adenovirus death protein - bezeichnet (Tollefson, J. Virology, 70, 2296-2306, 1996). Das Protein wird vornehmlich in der späten Phase des Infektionszyklus gebildet. Ferner führt die Überexpression des Proteins zu einer besseren Lyse der infizierten Zellen (Doronin et al., J. Virology, 74, 6147-6155, 2000).

Weiterhin ist dem vorliegenden Erfinder bekannt, dass E1A-deletierte Viren, d.h. insbesondere solche Viren, die kein 12S E1A-Protein und auch kein 13S E1A-Protein exprimieren, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Ferner war bekannt, dass von den von E1A insgesamt 5 codierten Proteinen zwei Proteine, nämlich das 12S- und das 13S-Protein, die Expression der anderen adenoviralen Gene steuern bzw. induzieren (Nevins, J. R., Cell 26, 213-220, 1981; Boulanger, P. und Blair, E.; Biochem. J. 275, 281-299, 1991). Dabei hat sich gezeigt, dass hauptsächlich die CR3-Region des 13S-Proteins die transaktivierende Funktion ausübt (Wong HK und Ziff EB., J Virol., 68, 4910-20, 1994). Adenoviren, die bestimmte Deletionen in der CR1- und/oder CR2-Region und/oder CR3-Region des 13S-Proteins aufweisen, sind weitestgehend replikationsdefekt, wirken aber noch bei einzelnen Zelllinien transaktivierend auf die viralen Gene bzw. Promotoren, insbesondere auf die E2 Region (Wong HK, Ziff EB., J Virol. 68, 4910-20, 1994; Mymryk, J. S. und Bayley, S. T., Virus Research 33, 89-97, 1994).

Nach Infektion einer Zelle, typischerweise einer Tumorzelle, mit einem Wildtyp-Adenovirus wird YB-1 vermittelt durch E1A, E1B-55K und E4orf6 in den Kern induziert und co-lokalisiert mit E1B-55K im Kern in den viral inclusion bodies, was eine effektive Replikation des Virus im Zellkern sowohl in vitro als auch in vivo erlaubt. Dabei war bereits früher festgestellt worden, dass auch E4orf6 an E1B-55 K bindet (Weigel, S. und Dobbelstein , M. J. Virology, 74, 764-772, 2000; Keith N. Leppard, Seminars in Virology, 8, 301-307, 1998.) und somit den Transport bzw. die Verteilung von E1B-55K in den Kern vermittelt, was eine optimale Virusproduktion bzw adenovirale Replikation gewährleistet. Durch das Zusammenwirken von E1A, E1B-55K und YB-1 bzw. durch den Komplex aus E1B-55K/E4orf6 mit YB-1 und der Kolokalisation von YB-1 und E1B-55K im Kern in den sogenannten viral inclusion bodies ist eine erfindungsgemäße effiziente Replikation des Virus, und damit die Verwendung der hierin beschriebenen Viren zur Replikation in Zellen, die YB-1-Kern-positiv sind, bzw. zur Herstellung eines Medikamentes zur Behandlung von Erkrankungen, bei denen YB-1-Kern-positive Zellen beteiligt sind, möglich. Die dadurch vor diesem zellulären Hintergrund mögliche Replikation führt zu einer Lyse der Zelle, Freisetzung des Virus und Infektion und Lyse benachbarter Zellen, so dass im Falle der Infektion einer Tumorzelle bzw. eines Tumors letztlich eine Lyse des Tumors, d.h. eine Onkolyse, eintritt.

YB-1 gehört zu einer Gruppe hoch konservierter Faktoren, die an der invertierten CAAT-Sequenz, der sogenannten Y-Box, binden. Sie können sowohl auf der Ebene der Transkription als auch der Translation regulatorisch wirken (Wolffe, A. P. Trends in Cell Biology 8, 318-323, 1998). Es werden immer mehr Y-Box-abhängige Regulationswege bei der Aktivierung aber auch bei der Hemmung Wachstums- und Apoptose-assoziierter Gene aufgefunden (Swamynathan, S. K. et al.,FASEB J. 12, 515-522, 1998). So interagiert YB-1 direkt mit p53 (Okamoto, T. et al., Oncogene 19, 6194-6202, 2000), spielt eine wichtige Rolle bei der Fas-Genexpression (Lasham, A. et al., Gene 252, 1-13, 2000), MDR und MRP-Genexpression (Stein, U. et al., JBC 276, 28562-69, 2001; Bargou, R. C. et al., Nature Medicine 3, 447-450, 1997) und bei der Aktivierung von Topoisomerasen und Metalloproteinasen (Mertens. P. R. et al., JBC 272, 22905-22912, 1997; Shibao, K. et al., Int. J. Cancer 83, 732-737, 1999). Zudem ist YB-1 an der Regulation der mRNA-Stabilität (Chen, C-Y. et al., Genes & Development 14, 1236-1248, 2000) und an Reparaturvorgängen beteiligt (Ohga, T. et al.,Cancer Res. 56, 4224-4228, 1996;).

Die nukleäre Lokalisation von YB-1 in Tumorzellen führt zu einer E1A-unabhängigen viralen Replikation, bei der insbesondere weder ein 12S E1A-Protein noch ein 13S E1A-Protein exprimiert vorliegt bzw. verwendet wird, (Holm, P. S. et al. JBC 277, 10427-10434, 2002) und im Falle der Überexpression des Proteins YB-1 zu einer multidrug resistance (Vielfachresistenz). Zudem ist bekannt, dass die adenoviralen Proteine wie z. B. E4orf6 und E1B-55K einen positiven Effekt auf die virale Replikation ausüben (Goodrum, F. D. und Ornelles, D. A, J. Virology 73, 7474-7488, 1999), wobei ein funktionelles E1A Protein für das Einschalten der anderen viralen Genprodukte (z. B. E4orf6, E3ADP und E1B-55K) verantwortlich ist (Nevins J. R., Cell 26, 213-220, 1981). Dies unterbleibt jedoch bei den im Stand der Technik bekannten E1A-minus Adenoviren, bei denen das 13S E1A-Protein nicht vorhanden ist. Die Kernlokalisation von YB-1 in multidrug resistenten Zellen, die YB-1 im Kern aufweisen, erlaubt die Replikation bzw. Partikelbildung derartiger E1A-minus Viren. Hierbei ist jedoch die Effizienz der viralen Replikation bzw. Partikelbildung im Vergleich zum Wildtyp Ad5 um ein Vielfaches geringer. Eine Kombination von YB-1, welches entweder bereits im Zellkern der Tumorzelle enthalten ist, oder durch äußere Faktoren (z.B. Applikation von Zytostatika oder Bestrahlung oder Hyperthermie) in den Zellkern induziert, d.h. veranlasst wird, im Zellkern vorhanden zu sein, insbesondere unabhängig vom Zellzyklus vorhanden zu sein, oder als Transgen durch einen Vektor eingeführt wird, mit einem System, bevorzugterweise mit einem adenoviralen System, welches die adenoviralen Gene einschaltet, aber nicht zur viralen Replikation fähig ist, stellt demgegenüber überraschenderweise ein System dar, welches eine sehr effektive virale Replikation bzw. Partikelbildung durch YB-1 vermittelt und damit eine Onkolyse erlaubt. Geeignete Zytostatike sind beispielsweise solche, die zu den folgenden Gruppen gehören: Anthracycline, wie beispieleweise Daunomycin und Adriamycin; Alkylanzien, wie beispeislweise Cyclophosphamid; Alkaloide, wie beispielsweise Etoposid; Vin-Alkaloide, wie beispielsweise Vincristin und Vinblastin; Antimetabolite wie beispielsweise 5-Fluorouracil und Methrothrexat; Platin-Derivate, wie beispielsweise cis-Platin; Topoisomerase-Inhibitoren, wie beispielsweise Camphothecin; und Taxane, wie beispielsweise Taxol. Die hierin offenbarten Adenoviren, insbesondere rekombinanten Adenoviren, welche nur in YB-1-Kern-positiven Zellen zur Replikation befähigt sind, sind in ihrer Fähigkeit, transaktivierend auf die viralen Gene E1B-55K, E4orf6, E4orf3 und E3ADP zu wirken, beschränkt, verglichen mit den diesbezüglichen transaktivierenden Fähigkeiten von Adenoviren vom Wildtyp, insbesondere vom Wildtyp Ad5. Der vorliegende Erfinder hat nun überraschenderweise festgestellt, dass diese beschränkte transaktivierende Fähigkeit dadurch aufgehoben werden kann, dass die entsprechenden Gene und insbesondere E1B-55K und E4orf6 in Verbindung mit der Kernlokalisation von YB-1 zur Expression gebracht werden. Wie in den Beispielen hierin gezeigt wird, erhöht sich die virale Replikation bzw. Partikelbildung unter diesen Umständen auf ein Niveau, welches vergleichbar ist mit dem Replikationsverhalten bzw. Partikelbildungsverhalten von Adenoviren vom Wildtyp.

Bei dem Medikament, im Rahmen dessen oder bei dessen Herstellung die hierin beschriebenen Adenoviren erfindungsgemäß verwendet werden, ist vorgesehen, dass dieses in der Regel systemisch appliziert wird, gleichwohl es auch im Rahmen der vorliegenden Erfindung ist, wenn dieses lokal appliziert oder abgegeben wird. Die Applikation erfolgt mit der Absicht, dass insbesondere jene Zellen mit dem Adenovirus infiziert werden und insbesondere darin eine Replikation der Adenoviren erfolgt, bei denen eine Beteiligung, bevorzugter Weise kausal, an der Ausbildung eines Zustandes, typischerweise einer Erkrankung, vorliegt, zu deren Diagnose und/oder Prävention und/oder Behandlung das erfindungsgemäße Medikament verwendet wird.

Ein derartiges Medikament ist bevorzugter Weise für die Behandlung von Tumorerkrankungen vorgesehen. Dabei sind jene Tumorerkrankungen besonders bevorzugt, bei denen entweder YB-1 bereits im Zellkern infolge des der Tumorerkrankung zugrundeliegenden Mechanismus, insbesondere des zugrundeliegenden pathologischen Mechanismus, vorliegt, oder aber durch äußere Maßnahmen die Anwesenheit von YB-1 im Zellkern bedingt wird, wobei die Maßnahmen geeignet sind, YB-1 in den Zellkern zu transferieren, dort zu induzieren oder dort zu exprimieren. Der Begriff Tumor oder Tumorerkrankung soll hierbei sowohl maligne wie benigne Tumoren und entsprechende Erkrankungen bezeichnen. Dabei kann vorgesehen sein, dass das Medikament mindestens eine weitere pharmazeutisch wirksame Verbindung enthält. Die Art und der Umfang dieser weiteren pharmazeutisch aktiven Verbindungen wird dabei von der Art der Indikation abhängen, für die das Medikament eingesetzt wird. Im Falle der Verwendung des Medikamentes für die Behandlung und/oder die Prophylaxe von Tumorerkrankungen werden typischerweise Zytostatika, wie beispielsweise cis-Platin und Taxol, Daunoblastin, Daunorubicin, Adriamycin und/oder Mitoxantron oder andere der hierin beschriebenen Zytostatika oder Gruppen von Zytostatika verwendet.

Das erfindungsgemäße Medikament kann dabei in verschiedenen Formulierungen vorliegen, bevorzugter Weise in einer flüssigen Form. Weiterhin wird das Medikament Hilfsstoffe wie Stabilisatoren, Puffer, Konservierungsstoffe und dergleichen enthalten, die dem Fachmann auf dem Gebiet der Galenik bekannt sind.

Der vorliegende Erfinder hat überraschenderweise festgestellt, dass die erfindungsgemäße Verwendung der hierin beschriebenen Viren mit besonders großer Erfolgsrate bei solchen Tumoren verwendet werden kann, bei denen YB-1 unabhängig vom Zellzyklus im Zellkern vorkommt. Normalerweise ist YB-1 im Cytoplasma, insbesondere auch im perinukleären Plasma, vorhanden. In der S-Phase des Zellzyklus findet sich YB-1 im Zellkern von sowohl normalen wie auch Tumorzellen. Dies ist jedoch nicht ausreichend, um eine virale Onkolyse unter Verwendung derartiger modifizierten Adenoviren zu bewerkstelligen. Die im Stand der Technik beschriebene vergleichsweise geringe Wirksamkeit von derartigen attenuierten Adenoviren beruht letztlich auf deren fehlerhaften Anwendung. Mit anderen Worten, es können derartige adenovirale Systeme, insbesondere auch mit einer größeren Wirksamkeit dort eingesetzt werden, wo die molekularbiologischen Voraussetzungen für eine virale Onkolyse unter Verwendung dieser, hierin beschriebenen attenuierten oder modifizierten Adenoviren gegeben sind. Im Falle der hierin erfindungsgemäß zu verwendend_beschriebenen Adenoviren, wie beispielsweise AdΔ24, d1922-947, E1Ad/01/07, CB016, dl520 und die im europäischen Patent EP 0 931 830 beschriebenen rekombinanten Adenoviren, liegen diese Voraussetzungen bei solchen Tumorerkrankungen vor, deren Zellen eine vom Zellzyklus unabhängige Kernlokalisation von YB-1 aufweisen. Diese Form der Kernlokalisation kann dabei durch die Art des Tumors selbst bedingt sein, oder aber durch die hierin beschriebenen erfindungsgemäßen Agenzien oder Maßnahmen bewirkt werden. Die vorliegende Erfindung definiert somit eine neue Gruppe von Tumoren bzw. Tumorerkrankungen und damit auch von Patienten, die mit den erfindungsgemäßen Viren, besonders aber auch mit den im Stand der Technik bereits beschriebenen attenuierten oder modifizierten Adenoviren, noch wirksam behandelt werden können.

Eine weitere Gruppe von Patienten, die erfindungsgemäß unter Verwendung der hierin als erfindungsgemäß zu verwenden beschriebenen, im Stand der Technik als solches wenigstens zum Teil bekannten Adenoviren, oder unter Verwendung der hierin erstmalig beschriebenen Adenoviren behandelt werden können, insbesondere unter Verwendung solcher Adenoviren, die Mutationen bzw. Deletionen im E1A-Protein aufweisen, welche die Bindungen von Rb/E2f nicht stören oder aber in YB-1-Kern-negativen Zellen nicht replizieren oder eine stark verringerte Replikation, wie hierin definiert und/oder ein deletiertes Onkoprotein, insbesondere E1A, aufweisen bzw. zeigen, wie beispielsweise im Falle der Viren AdΔ24, dl922-947, E1Ad/01/07, CB106 und der im europäischen Patent EP 0931 830 beschriebenen Adenoviren, sind jene Patienten, bei denen durch Anlegen oder Realisieren bestimmter Bedingungen gewährleistet wird, dass YB-1 in den Kern wandert oder dort induziert oder dorthin transportiert wird. Die Verwendung derartiger Adenoviren bei dieser Patientengruppe beruht insoweit auf der Erkenntnis, dass die Induktion der viralen Replikation auf der Kernlokalisation von YB-1 mit anschließender Bindung von YB-1 an den E2-late-Promotor beruht. Infolge der hierin offenbarten Erkenntnisse können Adenoviren wie beispielsweise AdΔ24, dl922-947, E1Ad/01/07, CB106 und/oder die im europäischen Patent EP 0931 830 beschriebenen, auch in solchen Zellen replizieren, die YB-1 Kern-positiv sind und/oder in Zellen, in den YB-1 dereguliert im Sinne der vorliegenden Erfindung vorliegt. Insoweit können diese Adenoviren erfindungsgemäß für die Behandlung von Erkrankungen bzw. Patientengruppen verwendet werden, die Zellen mit diesen Eigenschaften aufweisen, insbesondere dann, wenn diese Zellen an der Ausbildung der jeweiligen zu behandelnden Erkrankung beteiligt sind. Dies begründet den Erfolg von AdΔ24, dl922-947, E1Ad/01/07, CB016 und der im Patent EP 0931 830 beschriebenen Adenoviren bei der erfindungsgemäßen Behandlung solcher Tumoren, die YB-1 zellzyklusunabhängig im Kern oder YB-1 dereguliert im Sinne der vorliegenden Offenbarung aufweisen. Eine weitere Gruppe von Patienten, die erfindungsgemäß unter Verwendung der hierin als erfindungsgemäß zu verwenden beschriebenen Viren bzw. unter Verwendung der hierin erstmalig beschriebenen Viren, insbesondere Adenoviren, behandelbar sind, sind somit solche, die YB-1 Kern-positiv sind und/oder YB-1 Kern-positiv sind als Ergebnis der nachfolgend beschriebenen Behandlungen und/oder solche Patienten, die eine der nachfolgenden Maßnahmen, bevorzugt im Sinne einer Behandlung, erfahren haben oder gleichzeitig mit der Gabe der entsprechenden Viren erfahren. Dabei ist es im Rahmen der vorliegenden Erfindung, dass YB-1 Kern-positive Patienten solche Patienten sind, die insbesondere in einer Anzahl der einen Tumor ausbildenden Zellen YB-1 unabhängig vom Zellzyklus im Kern aufweisen. Zu diesen Maßnahmen gehört die Verabreichung von Cytostatika, wie sie hierin insgesamt beschrieben sind und/oder im Rahmen einer Tumortherapie verwendet werden. Weiterhin gehört zu dieser Gruppe von Maßnahmen Bestrahlung, insbesondere eine Bestrahlung, wie sie im Rahmen einer Tumortherapie angewandt wird. Bestrahlung bedeutet dabei insbesondere die Bestrahlung mit energiereicher Strahlung, bevorzugterweise mit radioaktiver Strahlung, bevorzugtererweise wie sie im Rahmen einer Tumortherapie verwendet wird. Eine weitere Maßnahme stellt Hyperthermie bzw. das Anlegen von Hyperthermie dar, bevorzugterweise Hyperthermie, wie sie im Rahmen einer Tumortherapie verwendet wird. In einer ganz besonders bevorzugten Ausführungsform ist dabei vorgesehen, dass die Hyperthermie lokal angewandt wird. Schließlich ist eine weitere Maßnahme die Hormonbehandlung, insbesondere die Hormonbehandlung, wie sie bei der Tumorbehandlung zur Anwendung gelangt. Im Rahmen einer derartigen Hormonbehandlung werden Anti-Östrogene und/oder Anti-Androgene verwendet. Dabei werden Anti-Östrogene, wie beispielsweise Tamoxifen, insbesondere bei der Therapie von Brustkrebs verwendet und Anti-Androgene, wie beispielsweise Flutamid oder Cyproteronacetat, bei der Therapie von Prostatakrebs verwendet.

Dabei ist es im Rahmen der vorliegenden Erfindung, wenn einige der den Tumor aufbauenden Zellen YB-1 entweder inhärent oder nach Induktion bzw. aktivem Einführen in den Kern dort aufweisen oder aber hinsichtlich YB-1 dereguliert im Sinne der vorliegenden Offenbarung vorliegt. Bevorzugterweise sind etwa 5 % oder ein jeglicher Prozentsatz darüber, d. h. 6 %, 7 %, 8 % etc. der den Tumor aufbauenden Zellen derartige YB-1 Kern-positive Zellen oder Zellen, in denen YB-1 dereguliert vorliegt. Die Kernlokalisation von YB-1 kann durch Stress von außen bzw. lokal appliziertem Stress induziert werden. Diese Induzierung kann beispielsweise durch Bestrahlung, insbesondere UV-Bestrahlung, Anwendung von Zytostatika, wie sie unter anderem hierin ebenfalls offenbart sind, und Hyperthermie erfolgen. Im Zusammenhang mit Hyperthermie ist beachtlich, dass diese zwischenzeitlich sehr spezifisch, insbesondere örtlich spezifisch, realisiert werden kann und somit ebenfalls spezifisch einen Kerntransport von YB-1 in den Zellkern bedingen kann und infolgedessen die Voraussetzungen für eine Replikation des Adenovirus und damit einer Zell- und Tumorlyse, die bevorzugt lokal beschränkt erfolgt, gegeben sind (Stein U, Jurchott K, Walther W, Bergmann S, Schlag PM, Royer HD. J Biol Chem. 2001,276(30):28562-9; Hu Z, Jin S, Scotto KW. J Biol Chem. 2000 Jan 28; 275(4):2979-85; Ohga T, Uchiumi T, Makino Y, Koike K, Wada M, Kuwano M, Kohno K. J Biol Chem. 1998, 273(11):5997-6000).

Das erfindungsgemäße Medikament würde somit auch an solche Patienten und Patientengruppen verabreicht werden bzw. wäre für solche bestimmt, bei denen durch geeignete Vorbehandlungen oder gleichzeitige Behandlung ein Transport von YB-1, insbesondere in die entsprechenden Tumorzellen, bedingt werden würde.

Auf der Grundlage dieser technischen Lehre ist es für den Fachmann im Rahmen seiner Fähigkeiten, geeignete Modifikationen insbesondere von E1A vorzunehmen, die beispielsweise Deletionen oder Punktmutationen umfassen können, um so verschiedene Ausführungsformen der im Rahmen der erfindungsgemäßen Verwendung einsetzbaren Adenoviren zu erzeugen.

Wie bereits vorstehend ausgeführt, sind die erfindungsgemäß verwendeten Adenoviren in der Lage, in solchen Zellen bzw. zellulären Systemen zu replizieren, die YB-1 im Kern aufweisen. Für die Frage, inwieweit die erfindungsgemäß verwendeten Adenoviren zur Replikation und damit zur Tumorlyse in der Lage sind, ist der Status der Zellen hinsichtlich des Vorhandenseins oder Nichtvorhandenseins von Rb, d. h. des Retinoblastom-Tumorsupressor-Produktes, unabhängig. Weiterhin ist es im Rahmen der erfindungsgemäßen Verwendung der besagten Adenoviren nicht erforderlich, auf den p53-Status der infizierten, zu infizierenden oder zu behandelnden Zellen abzustellen, da bei Verwendung der hierin offenbarten adenoviralen Systeme im Zusammenhang mit YB-1-Kern-positiven Zellen, d.h. Zellen, die unabhängig vom Zellzyklus YB-1 im Kern aufweisen, dieser ebenso wie der Rb-Status auf das Replikationsgeschehen des Adenovirus keinen Einfluss für die Ausführung der hierin offenbarten technischen Lehre hat.

Das Onkogen bzw. Onkogenprotein, insbesondere E1A, kann dabei entweder unter der Kontrolle der eigenen natürlichen adenoviralen Promotoren stehen und/oder aber über einen Tumor- oder Gewebe-spezifischen Promotor gesteuert werden. Geeignete nicht-adenovirale Promotoren können ausgewählt sein aus der Gruppe, die Cytomegalovirus-Promotor, RSV-(Rous sarcoma Virus) - Promotor, Adenovirus-basierender Promotor Va I und den nicht-viralen YB-1-Promotor (Makino Y. et al., Nucleic Acids Res. 1996, 15, 1873-1878) umfasst. Weitere Promotoren, die im Zusammenhang mit einem jeden Aspekt der hierin offenbarten Erfindung verwendet werden können, stellen der Telomerase-Promotor, der Alpha-Fetoprotein (AFP)-Promotor, der Caecinoembryonic Antigen Promotor (CEA) (Cao, G., Kuriyama, S., Gao, J., Mitoro, A., Cui, L., Nakatani, T., Zhang, X., Kikukawa, M., Pan, X., Fukui, H., Qi, Z. Int. J. Cancer, 78, 242-247, 1998), der L-Plastin-Promotor (Chung, I., Schwartz, PE., Crystal, RC., Pizzorno, G, Leavitt, J., Deisseroth, AB. Cancer Gene Therapy, 6, 99-106, 1999), Argenin-Vasopressin-Promotor (Coulson, JM, Staley, J., Woll, PJ. British J. Cancer, 80, 1935-1944, 1999), E2f-Promotor (Tsukada et al. Cancer Res., 62, 3428 - 3477), Uroplakin II Promotor (Zhang et al., Cancer Res., 62, 3743-3750, 2002) und der PSA-Promotor (Hallenbeck PL, Chang, YN, Hay, C, Golightly, D., Stewart, D., Lin, J., Phipps, S., Chiang, YL. Human Gene Therapy, 10, 1721-1733, 1999) dar. Ferner stellt der in der deutschen Patentanmeldung DE 101 50 984.7 beschriebenen YB-1 abhängigen E2-late-Promotor von Adenoviren einen Promotor dar, wie er im Rahmen der vorliegenden Erfindung verwendet werden kann.

Hinsichtlich des Telomerase-Promotors ist bekannt, dass dieser in humanen Zellen von zentraler Bedeutung ist. So wird die Telomeraseaktivität durch die transkriptionelle Kontrolle des Telomerase reverse Transkriptase-Gens (hTERT), welches die katalytische Untereinheit des Enzyms darstellt, reguliert. Die Expression der Telomerase ist in 85% der humanen Tumorzellen aktiv. Im Unterschied dazu ist sie in den meisten normalen Zellen inaktiv. Ausgenommen davon sind Keimzellen und embryonales Gewebe (Braunstein, I. et al., Cancer Research, 61, 5529-5536, 2001; Majumdar, A. S. et al., Gene Therapy 8, 568-578, 2001). Genaue Untersuchungen am hTERT-Promotor haben gezeigt, dass Fragmente des Promotors von 283 bp bzw. 82 bp vom Initiationscodon entfernt für eine spezifische Expression in Tumorzellen ausreichend sind (Braunstein I. et al.; Majumdar AS et al., aaO). Daher eignet sich dieser Promotor bzw. die spezifischen Fragmente, um eine spezifische Expression eines Gens und insbesondere eines Transgens, bevorzugterweise eines hierin offenbarten Transgens, nur in Tumorzellen zu erzielen. Der Promotor soll die Expression des modifizierten Onkogens, bevorzugt des E1A-Onkogenproteins, nur in Tumorzellen ermöglichen. Auch ist die Expression eines Transgens, insbesondere eines solchen, das ausgewählt ist aus der Gruppe, die E4orf6, E1B55kD, ADP und YB-1 umfasst, in einem adenoviralen Vektor unter einem dieser Promotoren in einer Ausführungsform vorgesehen. Es ist auch im Rahmen der vorliegenden Erfindung, dass der Leserahmen des transaktivierenden Onkogenproteins, insbesondere des E1A-Proteins im Leserahmen (engl. "in frame") mit einem oder mehreren der Genprodukte des adenoviralen Systems ist. Der Leserahmen des transaktivierenden E1A-Proteins kann jedoch auch unabhängig davon sein.

Hinsichtlich der Eigenschaft der Zellen, zu deren Lyse die hierin beschriebenen Adenoviren erfindungsgemäß verwendet werden, ist vorgesehen, dass diese in einer Ausführungsform eine Resistenz, bevorzugterweise eine Mehrfach- oder Vielfach-Resistenz zeigen. Resistenz, wie hierin verwendet, bezeichnet dabei bevorzugterweise eine Resistenz gegen die hierin beschriebenen Zytostatika. Diese Mehrfach-Resistenz geht bevorzugterweise mit der Expression, bevorzugter Weise einer Überexpression, des membranständigen Transportproteins P-Glycoprotein einher, welches als Marker für die Bestimmung entsprechender Zellen und damit auch von diesem aufweisenden Tumoren bzw. entsprechende Patientengruppen herangezogen werden kann. Der Begriff Resistenz, wie er hierin verwendet wird, umfasst dabei sowohl die auch als klassische Resistenz bezeichnete, durch das P-Glycoprotein vermittelte Resistenz, wie auch die auch als atypische Resistenz bezeichnete Resistenz, die durch MRP vermittelte oder andere, nicht-P-Glycoprotein vermittelte Resistenzen umfasst. Ein weiterer Marker, der mit der Expression von YB-1 korreliert, ist die Topoisomerase II alpha. Insoweit kann in einem Screenen-Verfahren, um zu bestimmen, ob ein Patient mit den Adenoviren erfindungsgemäß mit Aussicht auf Erfolg behandelt werden kann, an Stelle von der bzw. in Ergänzung zur Bestimmung von YB-1 im Kern die Expression von Topoisomerase II alpha herangezogen werden. Ein Marker, der grundsätzlich ähnlich wie das P-Glycoprotein verwendet werden kann, ist MRP. Ein weiterer Marker, zumindest in dem Umfang, als dass colorectrale Karzinomzellen oder Patienten mit einem Colorectalcarcinom betroffen sind, ist PCNA (engl. proliferating cell nuclear antigen (Hasan S. et al., Nature, 15, 387-391, 2001.), wie beispielsweise beschrieben von Shibao K. et al. (Shibao K et al., Int. Cancer, 83, 732-737, 1999). Schließlich ist, zumindest für den Bereich der Brustkrebs- und Osteosarcoma-Zellen die Expression von MDR (engl. multiple drug resistance) ein Marker im vorstehend beschriebenen Sinne (Oda Y et al., Clin. Cancer Res., 4, 2273-2277, 1998). Ein weiterer möglicher Marker, der erfindungsgemäß verwendet werden kann, ist p73 (Kamiya, M., Nakazatp, Y., J Neurooncology 59, 143-149 (2002); Stiewe et al., J. Biol. Chem., 278, 14230-14236, 2003).

Es ist somit ein besonderer Vorteil der vorliegenden Erfindung, dass auch jene Patienten unter Anwendung der erfindungsgemäßen Verwendung der Adenoviren, wie hierin beschrieben, therapiert werden können, die ansonsten im medizinisch-klinischen Sinne als "austherapiert" gelten und somit eine weitgehende Behandlung der Tumorerkrankung nach den Methoden des Standes der Technik mit Aussicht auf Erfolg nicht mehr möglich ist, insbesondere die Verwendung von Zytostatika sinnvoller Weise nicht mehr möglich ist bzw. nicht mehr erfolgreich durchgeführt werden kann im Sinne einer Beeinflussung bzw. Verringerung des Tumors. Der Begriff des Tumors bezeichnet hierin allgemein auch eine jegliche Tumor- oder Krebserkrankung, die entweder inhärent YB-1 im Zellkern enthält oder aber durch Realisieren exogener Maßnahmen, wie hierin offenbart, YB-1 im Zellkern, bevorzugterweise unabhängig vom Zellzyklus, aufweist.

Weiterhin können die hierin beschriebenen Viren zur Behandlung grundsätzlich von Tumoren verwendet werden. Bevorzugterweise sind diese Tumoren ausgewählt aus der Gruppe, die Brustkrebs, Ovarialkarzinom, Prostatakarzinom, Osteosarkom, Glioblastom, Melanom, kleinzelliges Lungenkarzinom und Kolorektalkarzinom umfasst. Weitere Tumoren sind jene, die resistent, wie hierin beschrieben, sind, bevorzugt Gene, die mehrfach resistent sind, insbesondere auch solche Tumoren der vorstehend beschriebenen Gruppe.

Die Erfindung betrifft in einem weiteren Aspekt auch die Verwendung von einem mit YB-1 wechselwirkenden Mittels in einem Verfahren zum Screenen von Patienten, die mit einem modifizierten Adenovirus, d.h. einem Adenovirus, wie er erfindungsgemäß verwendet wird, wie beispielsweise AdΔ24, d1922-947, E1Ad/01/07, CB016 oder die im europäischen Patent EP 0931 830 beschriebenen Viren, behandelbar sind, wobei das Verfahren die folgenden Schritte umfasst:

- Untersuchen einer Probe des Tumorgewebes und
- Festellen, ob YB-1 im Kern Zellzyklus-unabhängig lokalisiert ist.

Anstelle von oder in Ergänzung zu YB-1 kann auch das Vorhandensein der vorstehend beschriebenen Marker festgestellt werden.

In dem Falle, dass das Tumorgewebe oder ein Teil davon YB-1 im Zellkern, insbesondere Zellzyklus-unabhängig, lokalisiert aufweist, können die hierin offenbarten Adenoviren erfindungsgemäß verwendet werden.

In einer Ausführungsform des Verfahrens ist vorgesehen, dass die Untersuchung des Tumorgewebes unter Verwendung eines Mittels erfolgt, das ausgewählt ist aus der Gruppe, die Antikörper gegen YB-1, Aptamere gegen YB-1, Spiegelmere gegen YB-1 sowie Anticaline gegen YB-1 umfasst. Grundsätzlich die gleichen Mittel können für die entsprechenden Marker hergestellt bzw. verwendet werden. Die Herstellung von Antikörpern, insbesondere monoklonalen Antikörpern, ist den Fachleuten auf dem Gebiet bekannt. Ein weiteres Mittel zum spezifischen Nachweis von YB-1 oder den Markern stellen Peptide dar, die mit hoher Affinität an ihre Zielstrukturen, im vorliegenden Falle YB-1 oder die besagten Marker, binden. Im Stand der Technik sind Verfahren bekannt, wie beispielsweise phage-display, um derartige Peptide zu erzeugen. Dabei wird typischerweise von einer Peptid-Bibliothek ausgegangen, wobei die einzelnen Peptide eine Länge von etwa 8 bis 20 Aminosäuren aufweisen und die Größe der Bibliothek etwa $10^2$ bis $10^{18}$, bevorzugterweise $10^8$ bis $10^{15}$ verschiedene Peptide beträgt. Eine spezielle Form von an Zielmolekülen bindenden Polypeptiden stellen die sogenannten Anticaline dar, wie sie beispielsweise in der deutschen Patentanmeldung DE 197 42 706 beschrieben sind.

Ein weiteres Mittel zum spezifischen Binden an YB-1 oder die entsprechenden, hierin offenbarten Marker und damit zum Nachweis einer Zellzyklus-unabhängigen Lokalisation von YB-1 im Zellkern sind die sogenannten Aptamere, d. h. D-Nukleinsäure, die auf RNA- oder DNA-Basis entweder als Einzelstrang oder als Doppelstrang vorliegen und spezifisch an ein Zielmolekül binden. Die Herstellung von Aptameren ist beispielsweise beschrieben im europäischen Patent EP 0 533 838. Eine Sonderform der Aptamere stellen die sogenannten Aptazyme dar, die beispielsweise beschrieben sind von Piganeau, N. et al. (2000), Angew. Chem. Int. Ed., 39, Nr. 29, Seiten 4369 - 4373. Dabei handelt es sich um eine spezielle Ausführungsform von Aptameren insoweit, als dass sie neben dem Aptameranteil noch einen Ribozymanteil aufweisen und nach Bindung oder Freisetzung des an den Aptamerteil bindenden Zielmoleküls der Ribozymanteil katalytisch aktiv wird und ein Nukleinsäuresubstrat spaltet, was mit der Erzeugung eines Signals einhergeht.

Eine weitere Form der Aptamere stellen sogenannte Spiegelmere dar, d. h. zielmolekülbindende Nukleinsäuren, die aus L-Nukleinsäuren hergestellt sind. Das Verfahren zur Herstellung dieser Spiegelmere ist beispielsweise beschrieben in WO 98/08856.

Die Probe des Tumorgewebes kann dabei durch Punktion oder durch einen chirurgischen Eingriff erhalten werden. Die Feststellung, ob im Kern YB-1 Zellzyklus-unabhängig lokalisiert ist, erfolgt dabei häufig unter Verwendung mikroskopischer Techniken und/oder mittels Immunhistoanalyse, typischerweise unter Verwendung von Antikörpern oder einem der weiteren vorstehenden Mitteln. Weitere Verfahren zum Nachweis, dass YB-1 im Kern und insbesondere dort Zellzyklus-unabhängig lokalisiert ist, sind dem Fachmann bekannt. Beispielsweise kann bei dem Durchmustern von gegen YB-1 gefärbten Gewebeschnitten die Lokalisation von YB-1 leicht erkannt werden. Dabei ergibt sich bereits infolge der Häufigkeit des Auftretens von YB-1 im Kern, dass es sich um eine Zellzyklus-unabhängige Lokalisation im Kern handelt. Eine weitere Möglichkeit zum Zellzyklus-unabhängigen Nachweis von YB-1 im Kern besteht in der Durchführung einer Färbung gegen YB-1 und Feststellen, ob YB-1 im Kern lokalisiert ist, und Durchführung der Bestimmung des Zellstadiums der Zellen. Dies bzw. die Detektion von YB-1 kann aber auch unter Verwendung der vorstehend genannten, gegen YB-1 gerichteten Mittel erfolgen. Der Nachweis der Mittel erfolgt dabei durch Verfahrensweisen, die den Fachleuten auf dem Gebiet bekannt sind. Dadurch, dass die besagten Mittel spezifisch gegen YB-1 gerichtet sind und insoweit nicht an andere Strukturen innerhalb der zu untersuchenden Probe, insbesondere der Zellen, binden, kann durch eine geeignete Markierung der Mittel deren Lokalisierung und infolge der spezifischen Bindung an YB-1 auch die Lokalisierung von YB-1 entsprechend nachgewiesen und festgestellt werden. Verfahren zum Markieren der Mittel sind den Fachleuten auf dem Gebiet bekannt.

Die vorliegende Erfindung soll im folgenden anhand der Figuren und Beispiele weiter veranschaulicht werden, wobei sich daraus neue Merkmale, Ausführungsformen und Vorteile der Erfindung ergeben. Dabei zeigt

Fig. 1 den strukturellen Aufbau der darin als AdEl/E3-minus Adenovirus bezeichneten adenoviralen Vektoren, die E1/E3-deletierte Adenoviren sind, Wildtyp-Adenovirus und Adenovirus dl520;

Fig. 2 die Bindungsdomänen des E1A-Proteins hinsichtlich der Bindung von p300, p107 und p105;

Fig. 3 U2OS-Zellen, welche nicht YB-1 im Kern aufweisen, nach Infektion mit dem darin als E1/E3-minus Ad5 bezeichneten E1/E3-deletierten Adenoviren Ad5 und dl520;

Fig. 4 257RDB-Zellen, welche YB-1 im Kern aufweisen, nach Infektion mit dem darin als E1/E3-minus Ad5 bezeichneten E1/E3-deletierten Adenoviren Ad5 und Adenovirus dl520;

Fig. 5 257RDB-Zellen und U2OS-Zellen, nach Infektion mit dem Adenovirus dl1119/1131;

Fig. 6 das Ergebnis einer EMSA-Analyse, womit belegt wird, dass YB-1 in den vielfachresistenten Zellen bzw. Zelllinien 257RDB, 181 RDB, MCF-7Ad- im Zellkern vorhanden ist, wohingegen YB-1 in U2OS und HeLa-Zellen nicht im Kern vorhanden ist;

Fig. 7        den strukturellen Aufbau des E1A-Proteins vom Wildtyp-Adenovirus, von Adenovirus dl520 und Adenovirus dl1119/1131;

Fig. 8        ein Balkendiagramm, welches die Repli-
             kationseffizienz von Adenoviren bei An-
             wesenheit zusätzlich exprimierter viraler
             Proteine in Absolutzahlen zeigt; und

Fig. 9        ein Balkendiagramm, welches die Steige-
             rung der Replikationseffizienz von Adeno-
             viren bei Anwesenheit zusätzlich expri-
             mierter viraler Proteine zeigt;

Fig. 10       mit U2OS-Zellen bewachsene Näpfen
             nach Kristallviolett-Färbung und Infektion
             mit dl520 mit 10 bzw. 30 pfu/Zelle bzw.
             Kontrolle (K) ohne Gabe von Daunorubicin
             bzw. mit Gabe von 40 ng pro ml Daunorubicin;

Fig. 11       mit HeLa-Zellen bewachsene Näpfen
             nach Kristallviolett-Färbung und Infektion
             mit dl520 mit 10 bzw. 30 pfu/Zelle bzw.
             Kontrolle (K) ohne Gabe von Daunorubicin
             bzw. mit Gabe von 40 ng pro ml Daunorubicin;

Fig. 12       ein Diagramm des Tumorvolumens über
             die Zeit von Tumoren verschiedenen Ursprungs (RDB257 und HeLa) nach Behandlung mit PBS bzw. dl520;

Fig. 13       Aufnahmen von euthanasierten Mäusen,
             die einen Tumor auf der Grundlage von
             RDB257-Zellen entwickelten, nach Behandlung mit PBS bzw. mit 5 x 10[8] pfu
             dl520;

Fig. 14       das Ergebnis einer Southern Blot-Analyse
             eines Zellextraktes (von den sucutan ge-
             wachsenen Tumoren) von RDB257-Zel-
             len und HeLa-Zellen nach Infektion mit
             dl520;

Fig. 15       ein Balkendiagramm, welches die Replikationseffizienz bzw. die Partikelbildung
             von dl520 und Wildtyp-Adenovirus in YB-
             1 Kern-positiven Tumorzellen (257RDB
             und 181RDB) und YB-1 Kern-negativen
             Tumorzellen (HeLa, U2OS) zeigt;

Fig. 16       den strukturellen Aufbau des Adenovirus
             vom Wildtyp und des adenoviralen Vektors AdXvir03;

Fig. 17       den strukturellen Aufbau des adenoviralen
             Vektors AdXVir03/01; und

Fig. 18A/B    mit 181RDB-Zellen (Fig. 18A) und
             272RDB-Zellen (Fig. 18B) bewachsene
             Näpfe nach Kristallviolett-Färbung und Infektion mit Ad312 (20 pfu/Zelle), Xvir03 (5
             pfu/Zelle) und Kontrolle (nicht infiziert),
             wobei die Krisstallviolettfärbung fünf Tage
             nach Infektion erfolgte.

**Beispiel 1: Strukturen von E1A-Modifikationen, wie sie von den erfindungsgemäß verwendeten Adenoviren aufgewiesen werden können**

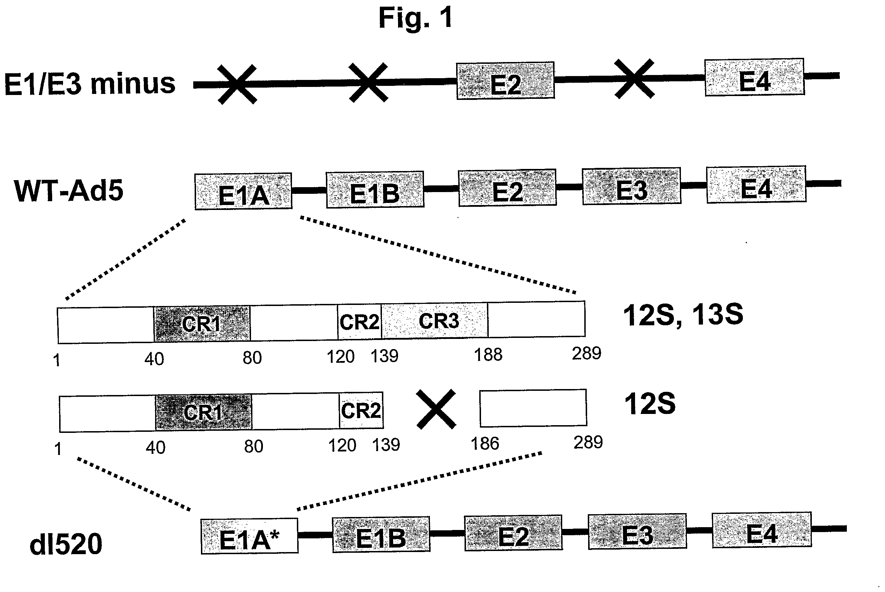

Fig. 1 zeigt den strukturellen Aufbau der adenoviralen Vektoren AdE1/E3-minus, d.h. des EI/E3-deletieren Adenovirus, Wildtyp-Adenovirus und Adenovirus dl520.

Das Adenovirus AdE1/E3-minus weist keine funktionalen für E1A und keine für E1B und keine für E3 codierenden Bereiche auf und dient im Rahmen der durchgeführten Experimente als Toxizitätskontrolle

Das Wildtyp-E1A-Gen kodiert für insgesamt 5 Proteine, die durch alternatives splicing der RNA von E1A hervorgehen. Dabei werden unter anderem zwei unterschiedliche Proteine, nämlich ein 289 Aminosäuren großes Protein und ein 243 Aminosäuren großes Protein hergestellt. Dl520 kodiert nicht für das 289 Aminosäure große Protein, da es eine Deletion im CR3-Bereich des E1A-Gens aufweist, das zu einem Fehlen des 13S-Genproduktes führt. Der erfindungsgemäß verwendbare Adenovirus dl520 wird unter Fachleuten als 12S-E1A Virus bezeichnet. Der im Stand der Technik bekannte Adenovirus dl347 (Wong und Ziff, J. Virol., 68, 4910-4920, 1994) ist ebenfalls ein 12S-E1A Virus, der erfindungsgemäß verwendet werden kann.

Innerhalb des 289 Aminosäuren großen Proteins, welches von 13S-E1A mRNA kodiert wird, gibt es 3 Bereiche, die bei den unterschiedlichen adenoviralen Subtypen konserviert vorliegen. Diese werden als CR1, CR2 und CR3 bezeichnet. Während CR1 und CR2 bei beiden E1A-Proteinen (E1A 12S und E1A 13S) vorkommt, d. h. sowohl bei dem 289 Aminosäure langen wie auch bei dem 243 Aminosäure langen Protein, ist der CR3-Bereich nur bei dem größeren der beiden vorstehend genannten Proteine zu finden.

Der CR3-Bereich wird für die Aktivierung der viralen Gene, insbesondere von E1B, E2, E3 und E4 benötigt. Viren, die nur das kleinere, 243 Aminosäuren lange Protein aufweisen, transaktivieren nur sehr schwach die viralen Gene und führen keine adenovirale Replikation in solchen Zellen durch, die YB-1 nicht im Kern aufweisen. Da YB-1 nur in Tumorzellen im Kern vorliegt bzw. nachweisbar ist, eignet sich dieser Vektor, um eine tumorspezifische Replikation zu induzieren.

Durch die Deletion von CR3 in dl520 ist dieser Adenovirus nicht in der Lage, zelluläres YB-1 in den Zellkern zu translokalisieren, hierin auch als Translozieren bezeichnet, und somit auch nicht in der Lage, in YB-1-Kern-negativen Zellen zu replizieren und stellt damit einen der erfindungsgemäß zu verwendenden Viren dar, wobei dieser Virus die erfindungsgemäß erforderliche Transaktivierung aufweist.

## Beispiel 2: Wirkmechanismus von Adenoviren in Abhängigkeit des Rb-Status von Zellen

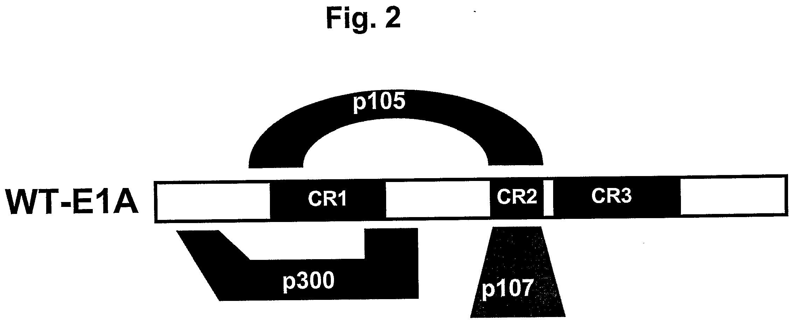

In Fig. 2 sind die Bindungsdomänen des E1A-Proteins hinsichtlich der Bindung von p300, p107 und p105 dargestellt. P300 ist dabei ebenso wie p107 ein zelluläres Bindungsprotein. Die Bindung des Retinoblastoma-Proteins (pRb), ein Tumorsuppressor-Protein, erfolgt über CR1 und CR2. Studien haben gezeigt, dass pRb und p107/p300 in Verbindung mit dem zellulären Transkriptionsfaktor E2F eine transkriptionelle Regulation ausüben. Das Wildtyp E1A-Protein unterbricht die Bindung von E2F an Rb. Das solchermaßen freigesetzte E2F bindet an den E2 early Promotor und induziert dadurch die adenovirale Replikation.

Es ist im Stand der Technik bekannt, dass bestimmte Deletionen im E1A-Onkoprotein dazu führen, dass rekombinante adenovirale Vektoren wie die nachstehend genannten vornehmlich in Rb-negativen Zellen zur Replikation befähigt ist und erfindungsgemäß verwendet werden können. Zum Beispiel weist der adenovirale Vektor dl922-947 eine Deletion in der CR2-Region auf (Aminoäurepositionen 122-129) und der Vektor CB016 Deletionen in den Bereichen CR1 (Aminoäurepositionen 27-80) und CR2 (Aminoäurepositionen 122-129). Der Vektor E1A*dl*/01/07 weist eine Deletion im CR2-Bereich auf (Aminoäurepositionen 111-123). Durch eine zusätzliche Deletion am N-Terminus (Aminoäurepositionen 4-25), erfolgt zudem keine Bindung an das Protein p300. Der adenovirale Vektor AdΔ24 weist eine Deletion in der CR2-Region auf (Aminoäurepositionen 120-127). Der im Patent EP 0 931 830 beschriebene adenovirale Vektor weist Deletionen in dem CR1 und CR2-Bereich auf.

Der Bindungsmechanismus von E2F/RB und die durch E1A vermittelte Freisetzung von E2F ist grundlegend verschieden von dem der vorliegenden Erfindung zugrunde liegenden Mechanismus. Nicht die Freisetzung von E2F vom Rb-Protein ist, wie im Stand der Technik angenommen, ein wichtiger, um nicht zu sagen der entscheidende Vorgang der adenoviralen Replikation, sondern die Kernlokalisation des humanen Transkriptionsfaktors YB-1. Dieser Transkriptionsfaktor kommt in normalen Zellen über den größten Teil des Zellzyklus lediglich im Zytoplasma vor. Nach Infektion mit einem Adenovirus wird dieser unter bestimmten Bedingungen in den Kern induziert oder liegt bei bestimmten zellulären Systemen wie bestimmten Tumorerkrankungen, wie z.B. aber nicht darauf beschränkt, Brustkrebs, Ovarialkarzinom, Prostatakarzinom, Osteosarkom, Glioblastom, Melanom, kleinzelliges Lungenkarzinom und Kolorektalkarzinom, bereits im Kern vor.

## Beispiel 3: Infektion von U2OS-Zellen

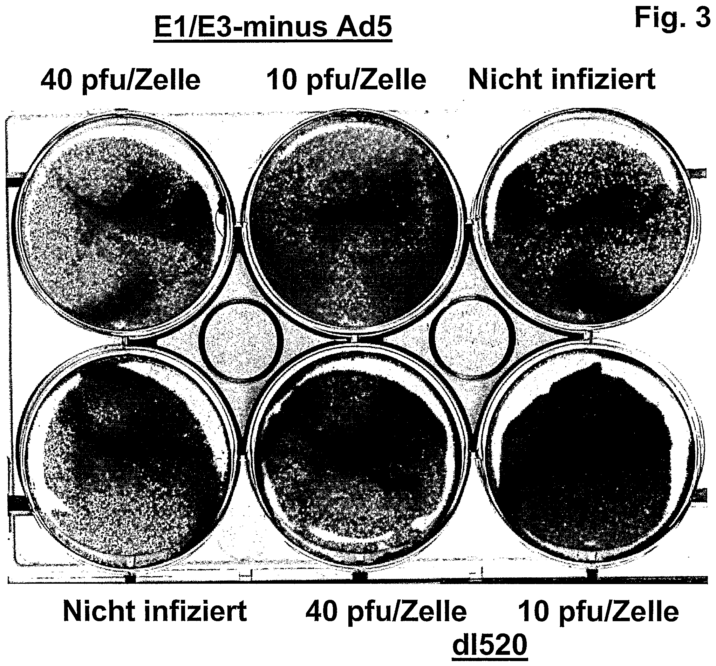

Pro Schale wurden 100.000 U2OS-Zellen ausplattiert. Am nächsten Tag wurden die Zellen wie in Fig. 3 dargestellt mit den verschiedene Adenoviren infiziert. Die Infektion erfolgte in 500 μL serumfreie DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Wie aus Fig. 3 ersichtlich zeigen die U2OS Zellen, welche YB-1 nicht im Kern aufweisen, nach Infektion mit zwei verschiedenen Adenoviren, nämlich dem als E1/E3-minus bezeichneten E1/E3-deletierten Adenovirus und Adenovirus d1520, der erfindungsgemäß verwendet werden kann, keine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert. Danach werden die Platten mit 6 Näpfen mit Wasser gründlich gewaschen und bei Raumtemperatur getrocknet.

Dies bestätigt die der vorliegenden Erfindung zugrundeliegende Erkenntnis, dass das Vorhandensein von YB-1 erforderlich ist, um die erfindungsgemäß zu verwendenden Viren zu einer Lyse von infizierten Zellen zu veranlassen.

## Beispiel 4: Infektion von 257RDB-Zellen

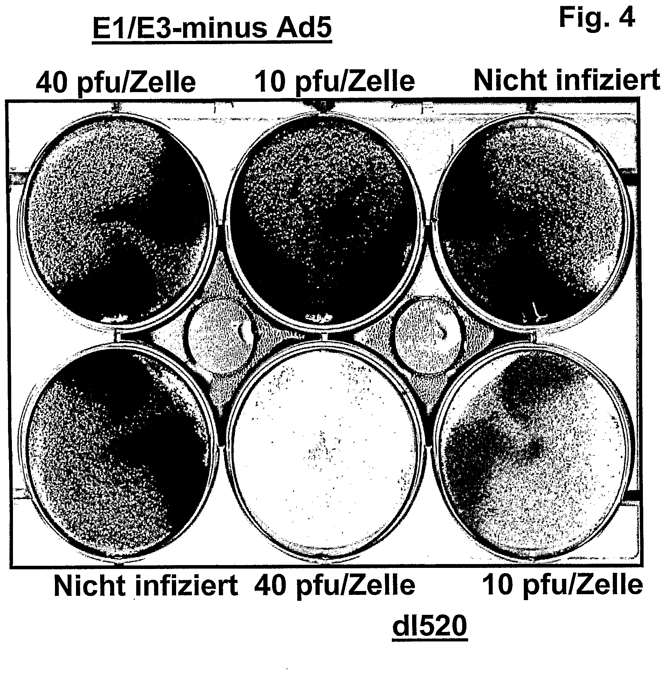

Pro Schale wurden 100.000 257RDB-Zellen ausplattiert. Am nächsten Tag wurden die Zellen wie in Fig. 4 dargestellt mit den verschiedene Adenoviren infiziert. Die Infektion erfolgte in 500 μL serumfreiem DMEM-Medium für 1 h bei 37° C. Anschließend wurde das Infektionsmedium entfernt und durch 2 ml Vollmedium (10 % FKS/DMEM) ersetzt. Nach 3 Tagen erfolgte die Auswertung mit Hilfe einer Kristallviolettfärbung.

Das Ergebnis dieses Versuchs ist in Fig. 4 dargestellt. Das Adenovirus als E1/E3-minus Ad5 bezeichnete E1/E3-deletierte Adenovirus zeigt keine Lyse bei niedrigen MOIs (pfu/Zelle) bei Infektion von 257RDB-Zellen, die YB-1 im Kern aufweisen. Im Gegensatz dazu zeigt dl520, welcher wie in Beispiel 3 belegt, in YB-1-Kern-negativen Zellen nicht repliziert und gleichzeitig mit E1A für ein im Sinne der vorliegenden Erfindung transaktivierendes Onkogenprotein codiert, eine praktisch vollständige Lyse bei einer MOI (engl. multiplicity of infection) von 40 pfu pro Zelle und eine noch überwiegende Lyse bei einer MOI von 10 pfu pro Zelle. Daraus ergibt sich, dass dl 520 und vergleichbare Viren, wie hierin beispielsweise mit dl1119/1131 oder AdXvir 03 beschrieben, eine gegenüber einem E1-deletierten oder einem E1/E3-deletierten Adenoviurs um etwa eine Größenordnung (etwa zehnfach) verringerte MOI erforderlich machen, was deren Eignung zur klinischen Anwendung begründet.

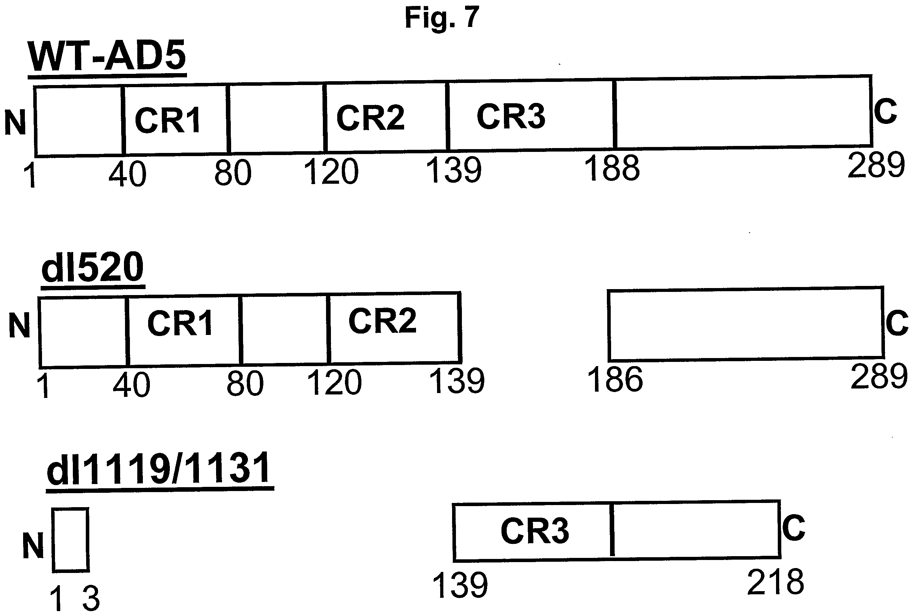

Wie in Fig. 7 dargestellt zeichnet sich das E1A-Protein von dl520 dadurch aus, dass der Bereich CR3 davon deletiert ist, was zu der für die erfindungsgemäße Verwendung des Adenovirus erforderlichen Transaktivierung und Replikation in YB-1-Kern-positiven Zellen führt.

**Beispiel 5: Infektion von 257RDB und U2OS-Zellen mit dl1119/1131**

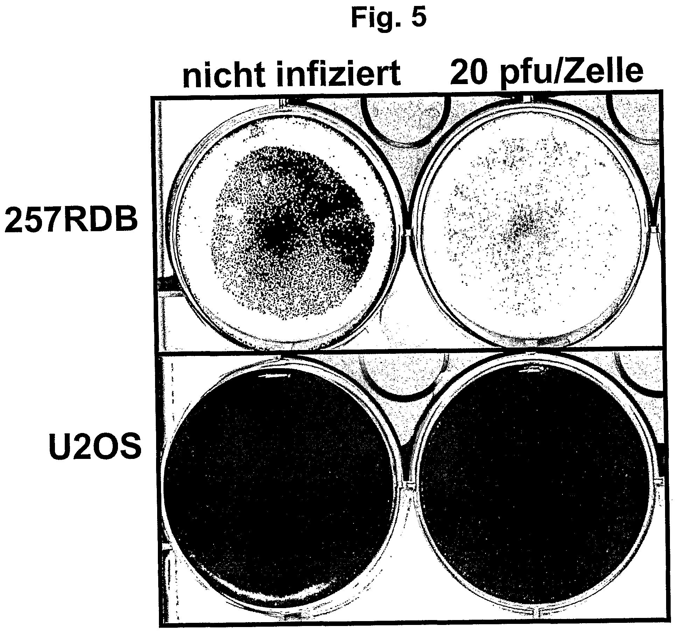

Wie in Fig. 5 dargestellt kommt es bei Infektion von YB-1-Kern-negativen U2OS-Zellen mit dem Adenovirus dl1119/1131, das eine Deletion der Aminosäuren 4-138 des E1A-Proteins bzw. der dafür codierenden Nukleinsäure und ein Stop-Codon nach Aminosäure 218 aufweist, wodurch das exprimierte verkürzte E1A-Protein die CR3-Region des vollständigen E1A-Proteins enthält, bei einer MOI von 20 pfu pro Zelle zu keiner Lyse. Als Negativkontrolle wurde ein nicht-infizierter Zellrasen herangezogen.

Im Gegensatz dazu zeigt sich unter dem Einfluss von Adenovirus dl1119/1131 in einem zellulären System wie 257RDB, welches YB-1 im Kern aufweist, d. h. YB-1-Kern-positiv ist, bereits bei einer MOI von 20 pfu pro Zelle eine praktisch vollständige Lyse des Zellrasens. Insoweit findet sich auch mit diesem Beispiel ein Beleg für die Aussage, dass ein modifiziertes E1A-Onkogenprotein, welches, wie in Fig. 7 dargestellt, beispielsweise lediglich den CR3-Bereich umfasst und dem der Bereich CR1 sowie CR2 fehlt, die für die erfindungsgemäße Verwendung von Adenoviren erforderliche Transaktivierung in YB-1-Kern-positiven Zellen zeigt, mit der Folge einer viralen Replikation. Der Adenovirus dl1119/1131 stellt somit einen weiteren, erfindungsgemäß verwendbaren Adenovirus dar. Dabei ist es im Rahmen der vorliegenden Erfindung, dass auch solche Viren verwendet werden können, die hinsichtlich des CR3-Bereiches wie dl1119/1131 ausgebildet sind, jedoch im Unterschied dazu den Bereich CR1 und/oder CR2 aufweisen.

**Beispiel 6: Nachweis von nuklärem YB-1 bei vielfachresistenten Zellen**

Dem Experiment liegt die Überlegung zugrunde, das nukläres YB-1 als Transkriptionsfaktor an die Y-Box (CAAT-Sequenz) innerhalb des mdr1-Promoters (engl. multiple drug resistance promoter) binden sollte. Um dies nachzuweisen, wurde eine sogenannte EMSA-Analyse (electrophoretic mobility shift assay) durchgeführt. Dabei wird Kernprotein isoliert und anschließend werden 1-10 μg Protein mit einem kurzen DNA-Fragment (Oligo) zusammen bei 37° C inkubiert. Um nukläres YB-1 zu bestimmen, wurde folgendes Oligonukleotid benutzt: *mdr1* promoter im Unterschied zu U20S (Position -86 bis -67): TGAGGCTGATTGGCTGGGCA (die Y-box ist unterstrichen).

Dieses DNA-Fragment wird zuvor mit einer Kinase am 5'-Ende mit $^{32}$P radioaktiv markiert. An-schließend erfolgt die Auftrennung in einem nativen Polyacrylamidgel. Falls das Protein YB-1 an einer Sequenz am Oligonucleotid bindet, ist dies zu erkennen, da ungebundenes Oligonukleotid im Gel schneller wandert als das gebundene Oligonucleotid (Holm, P. S. et al., JBC 277, 10427-10434, 2002; Bargou, R. C. et al., Nature Medicine 3, 447-450, 1997).

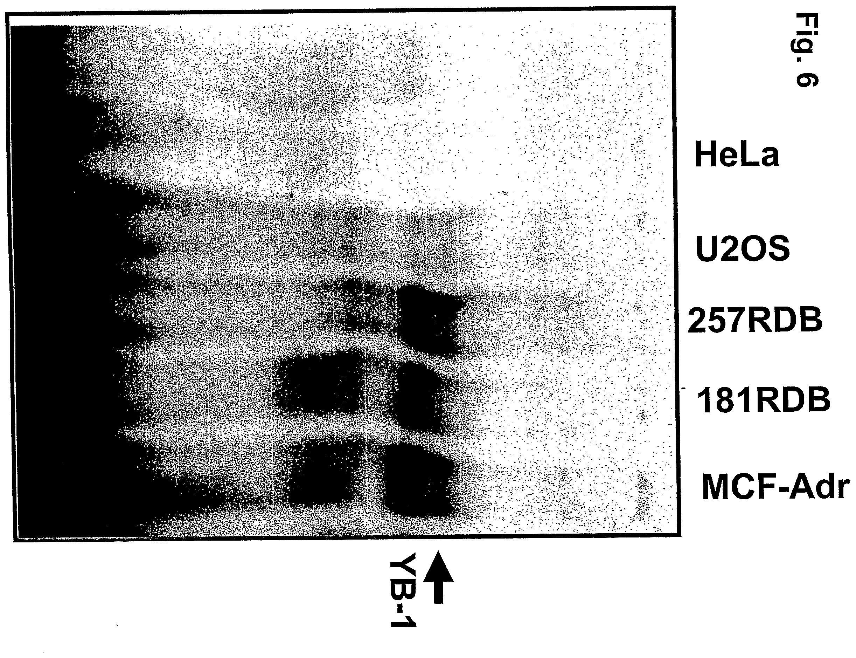

Wie in Fig. 6 dargestellt, konnte im Rahmen einer EMSA-Analyse gezeigt werden, dass YB-1 in den vielfachresistenten Zellen 257RDB, 181RDB und MCF-7Ad-Zellen im Kern im Gegensatz zu den Zelllinien U2OS und HeLa-Zellen vorhanden ist.

Die in Beispiel 4 und 5 gezeigten Ergebnisse belegen, dass die Adenoviren dl520 und dl1119/1131 in YB-1-Kern-positiven Zellen wie z. B. 257RDB im Unterschied zu U205 replizieren und eine Zelllyse induzieren. Dies belegt somit die Aussage der erfindungsgemäßen Verwendung der Adenoviren. Weiterhin belegen die Ergebnisse, dass bereits eine schwache Transaktivierung der viralen Gene in YB-1- Kern-positiven Zellen im Vergleich zum Wildtyp-Adenovirus durch die modifizierten oder deletierten E1A-Genprodukte bei Anwesenheit von YB-1 im Zellkern erfolgreich mit einer Replikation und Lyse von derartigen Zellen einhergeht, einschließlich beispielsweise von vielfachresistenten Zellen, und somit die hierin beschriebenen Adenoviren bei der Lyse derartiger Tumoren verwendet werden können.

**Beispiel 7: Steigerung der Replikationseffizienz von E1-minus Adenoviren**

In diesem Beispiel wird die Substitution der frühen viralen Gene E1B-55K und E4orf6 durch Transfektion mit dem Plasmid pE4orf6 und Infektion mit dem E1/E3-deletierten Adenovirus Ad-55K gezeigt. Ad-55k ist ein E1/E3 deletiertes Virus, wobei E1B-55k in die E1 kloniert wurde und unter CMV-Kontrolle steht. Diese Substitution wird mit Blick darauf erforderlich, dass AdYB-1, d. h. ein Adenovirus, der YB-1 exprimiert, diese frühen Gene nicht exprimiert und der vorliegende Erfinder erkannt hat, dass eine Substitution dieser frühen Gene in einem Replikationssystem, bei dem YB-1 im Kern vorhanden ist, in der Lage ist, die Replikationseffizienz bzw. die Partikelbildungseffizienz in einem Umfang vergleichbar derjenigen von Wildtyp-Adenoviren vom Typ Ad5 zu erhöhen.

Dabei wurde wie folgt vorgegangen:
Transfektion von je $10^5$ U2OS-Zellen mit dem Plasmid pE4orf6 mit Hilfe von Lipofectamin. Das Plasmid pE4orf6 trägt die für das frühe virale Gen E4orf6 codierende DNA-Sequenz unter CMV-Kontrolle.

24 h nach der Transfektion mit dem Plasmid pE4orf6 wurden die Zellen mit dem YB-1 exprimierenden E1/E3-deletierten Adenovirus AdYB-1 (50 pfu/Zelle) und dem E1/E3-deletierten E1B-55K Adenovirus Ad-55K (50 pfu/Zelle) infiziert. Ad-55K ist ein E1/E3-deletiertes Virus, welches als Transgen das virale Gen E1B-55K unter CMV-Kontrolle trägt.

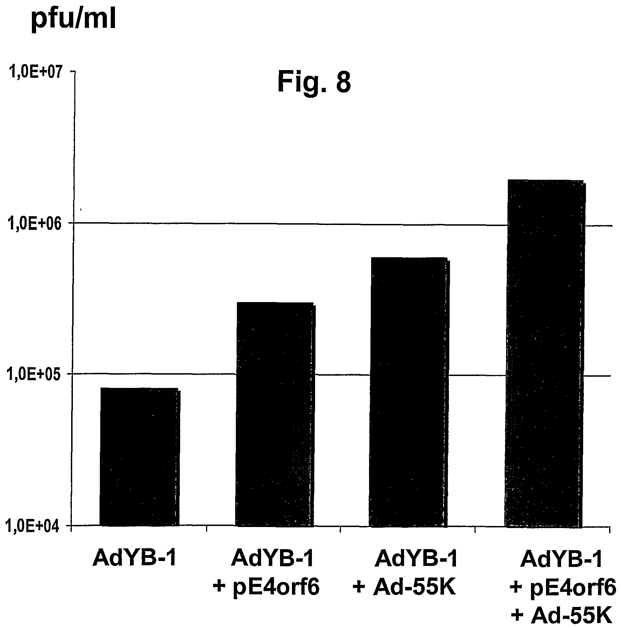

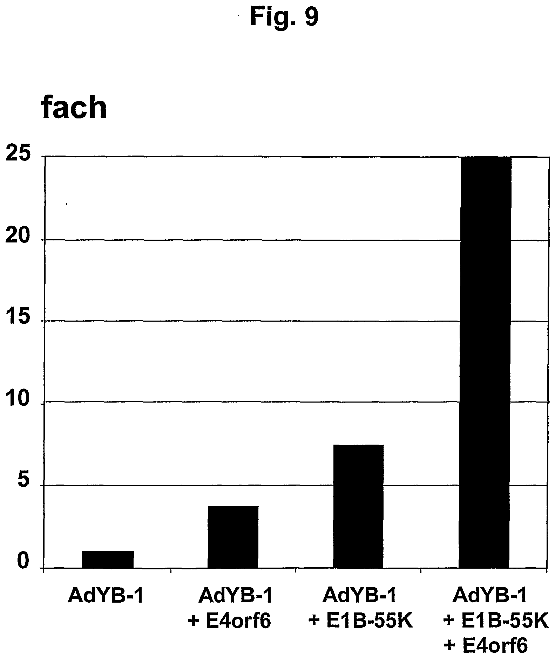

Anschließend wurden die Zellen vom Medium (2ml) 5 Tage nach der Infektion (= post infectionem) entfernt. Die Freisetzung der viralen Partikel aus den isolierten Zellen erfolgte durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in den Figs. 8 und 9 dargestellt. Dabei zeigt Figur 8 das Ergebnis des Plaque Assays, dargestellt in absoluten Zahlen. Die deutlichste Differenz zur Infektion mit AdYB-1 alleine zeigt hierbei die Kombination aus Transfektion mit dem Plasmid pE4orf6 und Co-Infektion mit den beiden Viren AdYB-1 und Ad-55K. Fig. 9 zeigt das Ergebnis von Fig. 8, wobei hier die Steigerung der Replikationseffizienz als Vielfaches der für AdYB-1 ermittelten Replikation dargestellt ist. Die mit Plasmid pE4orf6 transfizierten und anschließend mit AdYB-1 und E1B-55K (Ad-55K) infizierten Zellen produzierten bis zu 25 mal mehr pfu/ml.

Aufgrund dieser Ergebnisse kann gefolgert werden, dass die Substitution von E1B-55K und E4orf6 die Anzahl der gebildeten Viren (pfu/ml) nach Infektion mit dem E1/E3-deletierten Adenovirus AdYB-1 um einen Faktor von bis zu 25 erhöht. Dabei sind die additiven Effekte von E1B-55K und E4orf6 auf die Produktion von plaque forming units (pfu) signifikant größer als die Effekte eines der beiden Genprodukte alleine.

Kontrollversuche mit einem Plasmid, welches EGFP exprimierte, zeigten deutlich, dass in dem gewählten experimentellen Ansatz nur etwas 10 % der Zellen erfolgreich mit dem Plasmid pE4orf6 transfiziert werden konnten. Die Anzahl der in den Zellen gebildeten Partikel, die sowohl E1B-55K als auch E4orf6 exprimierten, ist mit der des humanen Adenovirustyp 5 (Wildtyp) vergleichbar. Dies bestätigt die der vorliegenden Erfindung zugrundeliegende Erkenntnis, dass die Expression von E4orf6 und E1B-55K in Verbindung mit der Kernlokalisation von YB-1 in der Lage ist, eine adenovirale Replikation bzw. Partikelbildung, insbesondere von E1A-deletiertes Adenoviren zu bewerkstelligen, die vergleichbar derjenigen von Wildtyp Ad5 ist.

**Beispiel 8: Verstärkung der Replikation von in YB1-Kern-negativen Zellen nicht replizierenden Adenoviren in YB-1-Kern-positiven Zellen nach Gabe von Zytostatika**

Im Stand der Technik ist bekannt, dass durch die Zugabe von verschiedenen Zytostatika die Kernlokalisation des humane Transkriptionsfaktor YB-1 induziert wird. Wie vom vorliegenden Erfinder gefunden, steuert kemlokalisiertes YB-1 die adenovirale Replikation mittels Aktivierung des adenoviralen E2-late Promoters. Die Kombination beider Effekte kann dazu genutzt werden, um eine spezifische Tumorlyse herbeizuführen.

Bei der Durchführung der onkolytischen Assays wurde wie folgt vorgegangen. 200.000 Zellen (HeLa bzw. U2OS) wurden je Napf in 6-Napf-Platten ausplattiert. Am nächsten Tag wurden 40 ng/ml (Endkonzentration) Daunorubicin zugegeben. Nach 3 Stunden Inkubationszeit wurden die Zellen mit 10 bzw. 30 pfu dl520/Zelle infiziert. Anschließend wurden die Zellen in Zytostatikafreiem Medium incubiert. Nach 3-5 Tagen wurden die Zellen mit Kristallviolett angefärbt.

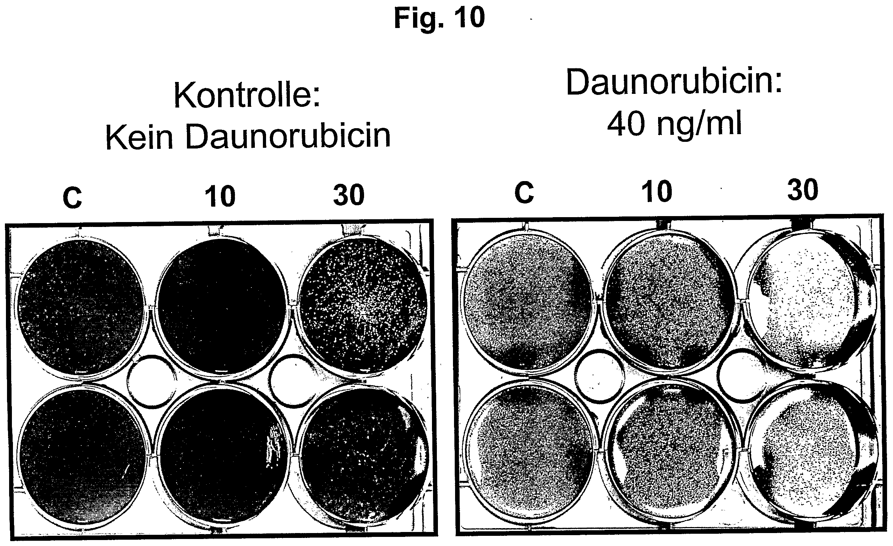

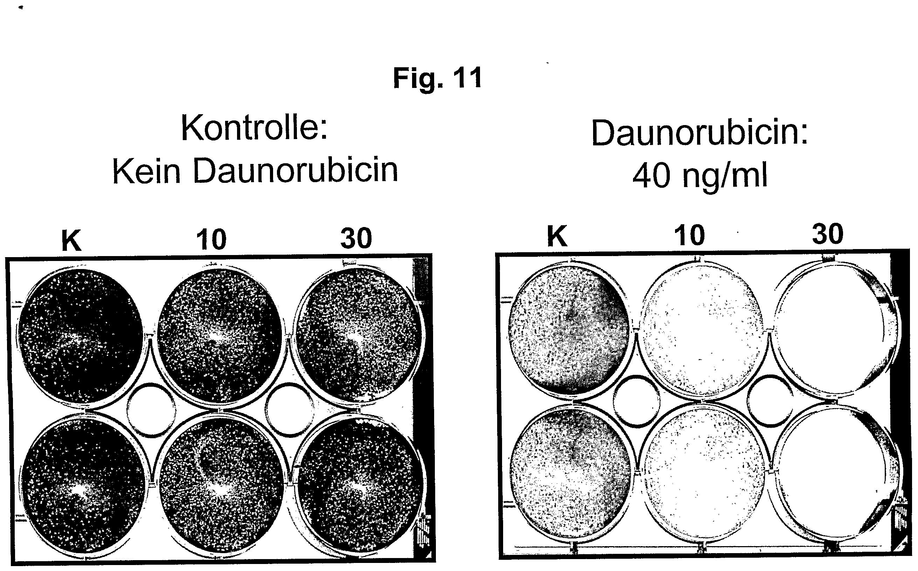

Wie aus Fig. 10 und 11 ersichtlich, induziert die Zugabe von Daunorubicin die Replikation von dl520 durch die Kernlokalisation von YB-1. Somit erzielt dl520 in Verbindung mit dem Zytostatikum Daunorubicin einen größeren tumorlytischen Effekt als Daunorubicin alleine.

**Beispiel 9: In vivo Tumorlyse durch dl520**

Die in dieser in vivo Studie verwendeten Zellen HeLa (YB-1 Kern-negativ) und 257RDB (YB-1 Kern-positiv) werden unter sterilen Zellkulturbedingungen expandiert. Kurz vor der Injektion der Zellen in die Mäuse (Stamm CD1NuNu), um einen Tumor subcutan zu setzen, werden diese durch Trypsinierung geerntet, in DMEM-Medium (10 % FKS) aufgenommen, gezählt und einmal mit PBS gespült. Anschließend werden die Zellen zentrifugiert, das PBS abgesaugt und in frischem PBS, der gewünschten Zellzahl entsprechen, portioniert. Die subcutan injizierte Zellzahl betrug in dieser Studie je $5 \times 10^6$ Zellen von beiden Zelllinien. Die Injektion erfolgt subcutan in eine Flanke der Tiere, wobei die HELA-Zellen in die rechte Seite und die 257RDB-Zellen in die linke Flankenseite zur besseren Unterscheidung injiziert wurden. Das Wachstum der Tumoren wurde zweimal wöchentlich kontrolliert und dabei die Länge und die Breite der Tumoren mit einer Schiebleere gemessen. Daraus wurde das Tumorvolumen über folgende mathematische Formel berechnet:

$$3/4\pi * a/2 * (b/2)^2 \quad a = \text{Länge, } b = \text{Breite}$$

Wenn der Tumor ein Volumen von 200 bis 520 $mm^3$ erreicht hat, wird das Virus bzw. PBS als Negativkontrolle intratumoral appliziert. Die zu injizierenden Volumina waren gleich und betrugen jeweils 50 µl. Dies wird an 3 aufeinanderfolgenden Tagen wiederholt. Die Gesamtdosis an appliziertem Virus betrug $5 \times 10^8$ pfu. Danach wird das Tumorwachstum weiterhin zweimal pro Woche dokumentiert und das Volumen berechnet. Am Ende der Studie werden die Mäuse euthanasiert und die Tumoren für weitere Analysen entnommen.

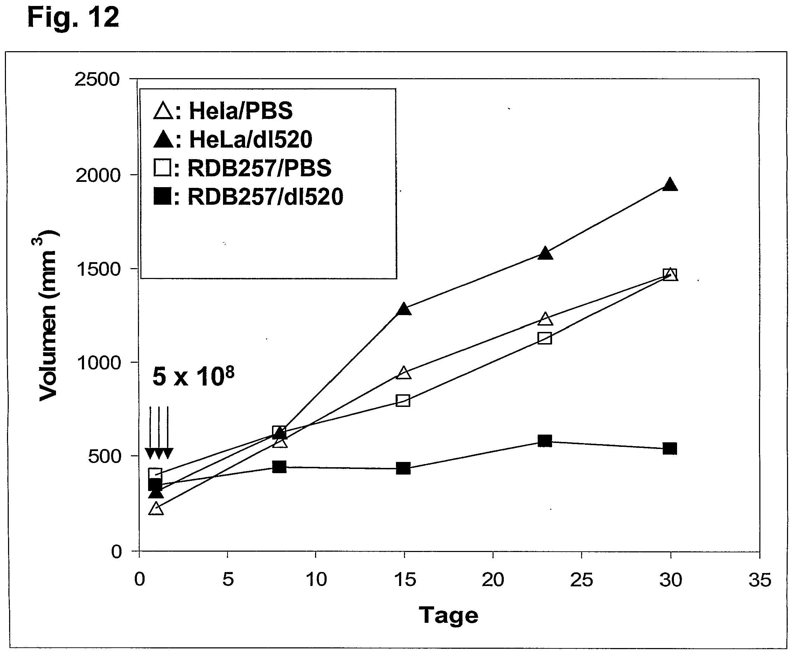

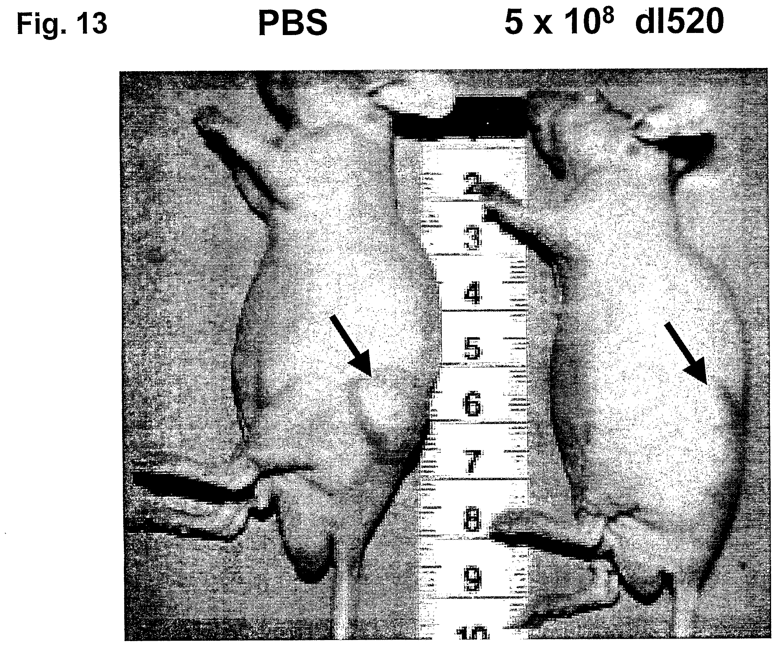

Die Ergebnisse sind in den Figuren 12 und 13 dargestellt.

Fig. 12 zeigt ein Diagramm, welches das Volumen des Tumors in Abhängigkeit von der Zeit und den verschiedenen Behandlungsschemata darstellt. Im Falle der durch RDB257 ausgebildeten Tumoren erfolgt bei Injektion von PBS ein signifikantes Wachstum des Tumors von ca. 438 $mm^3$ bis 1466 $mm^3$. Unter dem Einfluss des erfindungsgemäß verwendeten Vektors dl520 konnte das Tumorwachstum signifikant verringert werden. Ausgehend von einer durchschnittlichen Tumorgröße von 344 mm$^3$ erhöhte sich die Tumorgröße lediglich um 21 % auf insgesamt 543 mm$^3$.

Als Kontrolle wurde im vorliegenden Beispiel der Tumor aus HeLa-Zellen verwendet, der sich bei Gabe von PBS hinsichtlich seines Wachstums ähnlich verhielt, wie der auf RDB257 zurückgehende Tumor bei Gabe von PBS. Mit dl520 behandelte HeLa-Zellen basierte Tumoren zeigten einen noch wesentlicheren Anstieg des Tumorwachstums ausgehend 311 mm$^3$ bis 1954 mm$^3$.

Fig. 13 zeigt Aufnahmen von euthanasierten Nacktmäusen, denen unter Anwendung von RDB257 ein Tumor gesetzt wurde. Es ist deutlich zu erkennen, dass nach der erfindungsgemäßen Applikation des Adenovirus dl520 eine signifikante Rückbildung des Tumors eintrat. Im vorliegenden Falle wurde sogar eine Verringerung des Tumorvolumens erzielt (Tag 1 nach Applikation des Virus dl520: 515 mm$^3$; Tag 30 nach Applikation des Virus dl520: 350 mm$^3$.

**Beispiel 10: Southern Blot von Tumor-DNA**

Die DNA wird aus einem Tumorstück isoliert, welches aus der Mitte des in Beispiel 9 gesetzten Tumors entnommen worden ist. Für die Isolierung wird das Dneasy Tissue Kit von der Firma Qiagen benutzt. Die Durchführung der DNA-Isolierung erfolgte nach Herstellerangaben. Dabei wird die DNA aus den Zellen durch eine alkalische Lyse freigesetzt. Anschließend wird isolierte DNA über eine Säule gereinigt. Anschließend wird die Konzentration der isolierten DNA photometrisch bei 260 nm gemessen. Die Analyse erfolgte anhand von 2 μg der DNA-Proben, die mit dem Restriktionsenzym Kpn I 10 Units verdaut wurden. Dann erfolgt eine elektrophoretisch Auftrennung der Proben in einem 0,8%igen Agarosegel. Anschließend wurde die DNA auf eine Nylonmembran geblottet (Durchführung nach dem System von Schleicher & Schuell). Die auf der Membran geblottete DNA wird gegen eine spezifische 1501 bp große DNA-Sonde hybridisiert Die 1501 bp große DNA-Sonde bindet spezifisch an das 3369 bpgroßes Kpn I-Fragment innerhalb der E2A-codierenden Ad5-Sequenz. Die Sonde wurde zuvor per PCR (Primer:5'- GTC GGA GAT CAG ATC CGC GT, 5'- GAT CCT CGT CGT CTT CGC TT) hergestellt und mit $^{32}$P radioaktiv markiert. Anschließend wird die Membran gewaschen und auf eine Filmfolie exponiert.

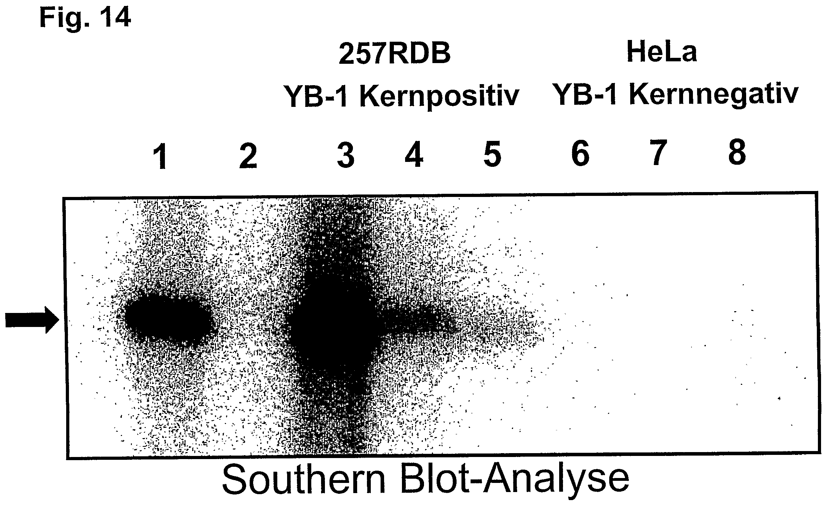

Das Ergebnis des Southern-Blots der Tumor-DNA ist in Fig. 14 dargestellt. Die Analyse belegt, dass nur dl520 in den resistenten Zellen RDB257 in vitro repliziert, wie in den Spuren 3, 4 und 5 dargestellt. Spur 1 zeigt als Positivkontrolle Ad-5d, Spur 6, 7 und 8 DNA von HeLa-Zellen, die mit dl520 infiziert wurden. Nachdem HeLa-Zellen keine YB-1-Kernpositivität zeigen, erfolgte darin keine Replikation des Virus dl520, so dass entsprechend die E2A-Sequenz nicht nachgewiesen werden konnte.

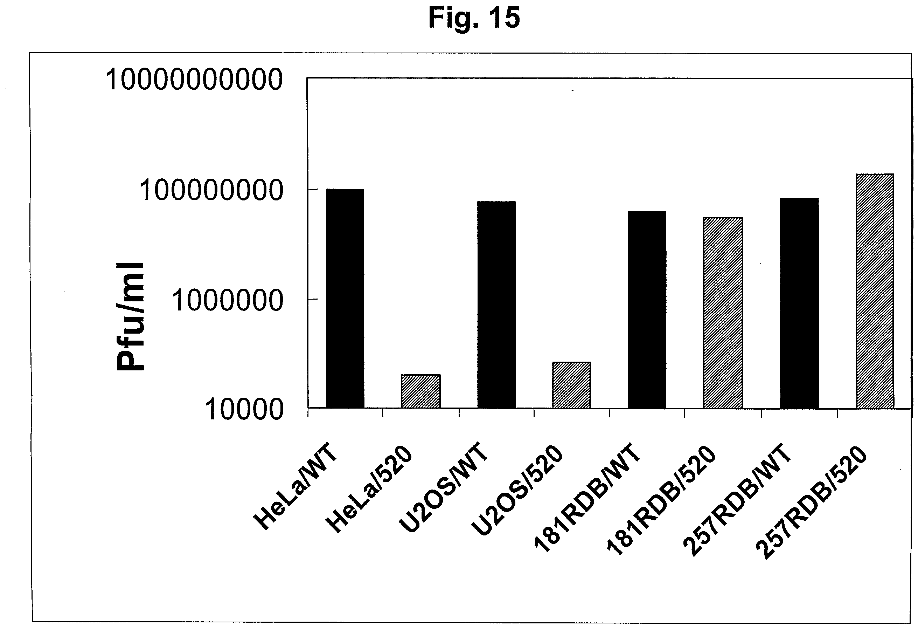

Ein weiteres Ergebnis mit dl520 ist in der Fig. 15 dargestellt. Anhand eines Plaque-assays wurde die Partikelbildung (pfu/ml) nach Infektion mit dl520 und Wildtyp-Adenovirus untersucht. Dabei werden verschiedene YB-1 Kern-positive (257RDB und 181RDB) Tumorzellen und YB-1 kernnegative Tumorzellen mit dl520 und Wildtyp-Adenovirus infiziert.

Dabei wurde wie folgt vorgegangen: Jeweils 100.000-200.000 Zellen werden in sog. Platten mit 6 Näpfen (engl. 6 well plates) in L 15-Medium (resistente Zellen) bzw. DMEM (nicht-resistente Zellen) jeweils mit 10 % FKS ausplattiert. Nach 24 h erfolgt die Infektion mit dl520 und WT-Adenovirus (10 pfu/Zelle) 3 Tage nach der Infektion (post infectionem) erfolgt die Freisetzung der viralen Partikel aus der Zellsuspension (3 ml) durch dreimaliges alternierendes Einfrieren und Auftauen (engl. thaw/freeze). Anschließend wurde ein Plaque Assay auf 293-Zellen zur Bestimmung der gebildeten infektiösen Partikel (plaque forming units pro ml (pfu/ml)) durchgeführt. Das Ergebnis ist in der Fig. 15 dargestellt. Dabei zeigt das Ergebnis des Plaque Assays, dass dl520 in den YB-1 Kern-positiven Zellen (257RDB und 181RDB) vergleichbar dem Wildtyp-Adenovirus repliziert. Insoweit wird bei der erfindungsgemäßen Verwendung der hierin beschriebenen Adenoviren eine Replikationseffizienz vergleichbar derjenigen von Wildtyp-Adenoviren erreicht.

**Beispiel 11: Struktureller Aufbau des adenoviralen Vektors Xvir03**

Fig.16 zeigt den strukturellen Aufbau des adenoviralen Vektors Xvir03. Das Adenovirus Xvir03 ist ein sogenanntes E1/E3-deletiertes Adenovirus. Das heisst, dass keine für die adenovirale Replikation funktionellen E1A, E1B und E3 Proteine hergestellt werden. Die Deletion der E1 Region erstreckt sich von 342 - 3528; die Deletion der E3 Region von Aminosäureposition 27865 - 30995. Wie hierin verwendet, bezeichnet der Begriff "E1-deletiertes Virus ein solches Virus, bei dem E1 funktional nicht mehr aktiv ist. Dies kann durch eine Inaktivierung bei ansonsten weitestgehend intakter Nukleinsäure- bzw. Aminosäuresequenz erfolgen, kann jedoch auch eine unterschiedlich große Deletion des für E1-Region kodierenden Proteine bedeuten. Durch das Fehlen des E1A und E1B Proteins bzw. der dafür codierenden Nukleinsäuren wird die E4-Region, z.B. E4orf6, nicht oder nur sehr schwach (ca. 1-5% im Vergleich zum Wildtyp-Adenovirus) exprimiert. In der E1-Region werden die viralen Gene E1B55k und E4orf6 vermittelt durch den in den Xvir 03 eingeführten, heterologen CMV-Promoter (Firma Clontech: Plasmid pShuttle) exprimiert. Anstelle des CMV-Promotors können auch jene hierin beschriebenen Promotoren verwendet werden, wie im Zusammenhang mit der Expression von E1A offenbart. Der offene Leserahmen beider Gene ist über eine sogenante IRES-Sequenz (engl.: internal ribosomal entry site) miteinander verbunden (Pelletier, J. and Sonenberg, N.

Nature, 1988, 334, 320-325). Dieses Element (Firma Novagen: pCITE) erlaubt die Expression von 2 Proteinen aus einer mRNA.

**Bei der Herstellung des Vektors wurde wie folgt vorgegangen:**

Das Plasmid E1B55k-pShuttle ging durch Umklonierung des offenen Leserahmen von E1B55k aus pCGNE1B von M. Dobelstein (Uni Marburg) mit XbaI und BfrI in den pShuttle-Vektor von Clontech hervor. Anschließend wurde E1B55k in pShuttle mit ApaI linearisiert, die Enden geglättet und mit NheI geschnitten.

In einem zweiten Vektor, dem pcDNA3.1(+) (Invitrogen), wurden nacheinander das IRES-Element als PCR-Produkt mit dem pCITE-4a(+) der Firma Novagen als Template über TA-Klonierung in die EcoRV-Schnittstelle und das E4orf6 aus dem Plasmid pCMV-E4orf6 (M. Dobelstein, Uni Marburg) über BamHI hineinkloniert = IRES-E4orf6-pcDNA3.1 (+). IRES-E4orf6 in pcDNA3.1 (+) wurde linearisiert mit NotI, die Enden geglättet und anschließend wurde das Fragment IRES-E4ORF6 mit NheI herausgeschnitten. Das Fragment IRES-E4orfF6 wurde mit dem geöffneten Vektor E1B55k-pShuttle (blunt, NheI) verknüpft. Die Kassette wurde anschließend aus dem E1B55k-IRES-E4orf6-pShuttle zusammen mit dem CMV-Promotor und dem *bovine growth hormone* (BGH)-PolyA in das ∆E1, ∆E3 Adeno-X-Plasmid (Clontech) mit I-Ceu I und PI-SceI kloniert und als AdcmvE1B/IRES/E4orf6 bezeichnet. Danach erfolgte die Adenovirusherstellung nach Herstellerangaben (Clontech). Das mit PacI linearisierte Adenoplasmid mit dem Expressionselement CMV-E1B55k-IRES-E4orf6-BGH polyA wurde mit in HEK293 Zellen transfiziert und nach 11 Tagen *post transfectionem* die sich ablösenden Zellen zusammen mit dem Medium abgenommen, um durch wiederholte Einfrier-Auftau-Zyklen die entstandenen Adenoviren freizusetzen.

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, in YB-1-Kern-positiven Zellen sowie Zellen, in denen YB-1 dereguliert, d.h. im Vergleich zu Normal-Zellen bzw. Nicht-Tumorzellen überexprimiert vorliegt, zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

**Beispiel 12: Struktureller Aufbau des adenoviralen Vektors Xvir03/01**

Wie aus Fig. 17 ersichtlich, ist XVIR03/01 eine Weiterentwicklung von XVIR03. Dabei können in der E3 Region therapeutische Gene wie beispielsweise die hierin beschriebenen Gene und Transgene kloniert werden. Zudem wurde eine Deletion in der E4-Region eingeführt, um eine homologe Rekombination mit dem E4orf6 aus der Expressionskassette von Xvir03 zu vermeiden. Dies führt auch dazu, dass bei diesem Konstrukt größere Transgene kloniert werden können. Die deletierte E3-Region enthält für das Einsetzen einer Kassette nutzbare SacI, NdeI und NheI- Schnittstellen, in die beispielsweise die therapeutischen Transgene einkloniert werden können.

**Vorbereitung eines Plasmids zur Klonierung therapeutischer Gene in die E3-Region sowie zur Deletion in der E4-Region:**

Das pAdenoX-Plasmid von Clontech verfügt über eine SfuI -Schnittstelle hinter der 3' ITR-Region, die im wildtyp Adenovirus fehlt. Die E3-E4-Region wurde mit SpeI (Position 23644) und SfuI aus pAdenoX (Clontech) in pcDNA3.1(+) (Invitrogen) übernommen = pcDNA3.1-E3∆27865-30995-E4. Der Großteil des E4ORF6, nämlich 33241-33875 wurde mittels PstI entfernt = pcDNA3.1-E3∆27865-30995,E4∆33241-33875. Für die Weiterentwicklung von XVIR03 wurde der deletierte E3/E4-Bereich aus pcDNA3.1-E3∆27865-30995,E4∆33241-33875 mittels SfuI und SpeI in das Plasmid pAdenoX kloniert = pAdenoX E3∆27865-30995,E4∆33241-33875.

Die Expressionskassette wurde anschließend wie für Xvir03 beschrieben aus dem E1B55k-IRES-E4orf6-pShuttle zusammen mit dem CMV-Promotor und dem *bovine growth hormone* (BGH)-PolyA in das pAdenoX E3∆27865-30995,E4∆33241-33875 mit I-Ceu I und PI-SceI kloniert und als AdcmvE1B/IRES/E4orf6-AE4 bezeichnet. Danach erfolgte die Adenovirusherstellung nach Herstellerangaben (Clontech).

Der vorstehend beschriebene Vektor eignet sich grundsätzlich wie die anderen hierin beschriebenen erfindungsgemäß zu verwendenden Viren. Insbesondere ist der vorstehend beschriebene Vektor dazu geeignet, bei YB-1-Kern-positiven Zellen sowie Zellen, in denen YB-1 dereguliert, d.h. im Vergleich zu Normal-Zellen bzw. Nicht-Tumorzellen überexprimiert vorliegt zu replizieren und insoweit eine Lyse herbeizuführen. Die Anwendung dieses Vektors erstreckt sich dabei auch insbesondere auf jene Krankheiten und Patientengruppen oder Patientenkollektive, die für die anderen hierin beschriebenen erfindungsgemäß zu verwendenden Adenoviren und erfindungsgemäßen Adenoviren offenbart sind.

**Beispiel 13: Onkolytische Wirkung von Xvir 03 in 257 RDB- und 181 RDB-Zellen**

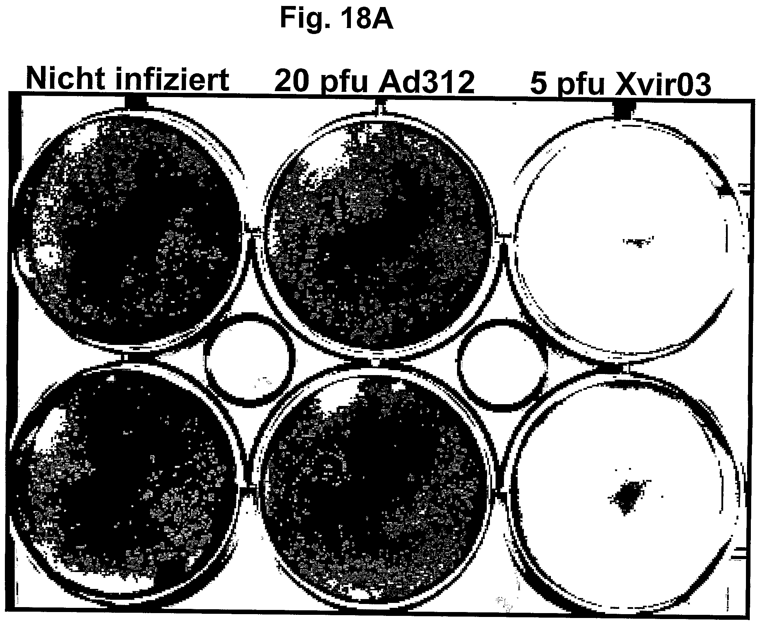

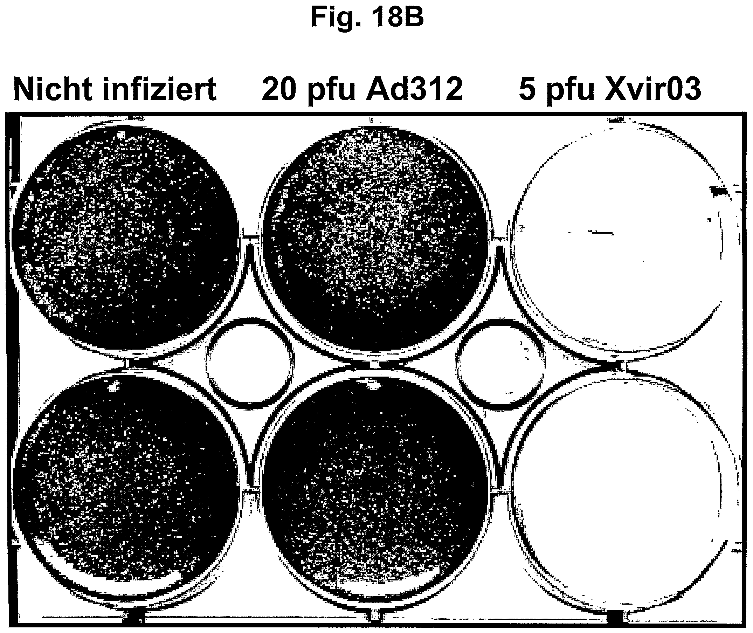

Pro Napf einer Platte mit sechs Näpfen (engl. 6 well plate) wurden jeweils 100.000 Zellen (257RDB und 181RDB) ausplattiert. Am nächsten Tag wurden die Zellen wie in Fig. 18 dargestellt mit Ad312 (20pfu/Zelle) und Xvir03 (5pfu/Zelle) infiziert. Die Infektion erfolgte in 500 μL serumfreie DMEM-Medium für 1 h bei 37°C. Anschließend wurde das Infektionsmedium entfernt und durch 2 ml Vollmedium (10 % FKS/DMEM) ersetzt. Nach 5 Tagen erfolgte die Auswertung mit Hilfe einer Kristallviolettfärbung. Das Ergebnis ist in Figs. 18A und 18B dargestellt.

Wie aus Fig. 18A und 18B ersichtlich, zeigen die vielfachresistenten Zellen, welche YB-1 im Kern aufweisen, nach Infektion mit Ad312 und Xvir03 nur bei Xvir03 eine Lyse, wie durch Kristallviolettfärbung der Zellen dargestellt. Dabei wird zunächst das Medium entfernt. Anschließend werden die Zellen mit Kristallviolett überschichtet (50 % ETOH, 3 % Formaldehyd, 5 % Essigsäure, 1 % Kristallviolett) und für 5-10 min bei Raumtemperatur inkubiert. Danach werden die Platten mit sechs Näpfen mit Wasser gründlich gewaschen und bei Raumtemperatur getrocknet.

Es ist dem vorliegenden Erfinder bekannt, dass E1A-deletierte Viren (z. B. Ad312), die jedoch keine transaktivierenden Adenoviren im Sinne der vorliegenden Erfindung sind, bei höheren MOI's sehr effizient replizieren können (Nevins J. R., Cell 26, 213-220, 1981), die jedoch in der klinischen Anwendung nicht zu realisieren sind. Dieses Phänomen wird in der Literatur als "E1A-like activity" bezeichnet. Der hier eingesetzte Adenovirus Ad312 ist ein E1A-deletiertes Virus. Bei der eingesetzten Menge (20 pfu/zelle), die noch oberhalb des klinisch wünschenswerten Titers liegt, werden die frühen adenoviralen Gene wie z. B. E1B55k und E4orf6 nicht oder nur sehr gering exprimiert (Nevins J. R., Cell 26, 213-220, 1981). Wie bereits beschrieben spielen diese Gene bzw. die Proteine eine bedeutende Rolle bei der viralen Replikation. Im Gegesatz dazu werden diese Gene bzw. Proteine beim Adenovirus Xvir03 exprimiert (Fig 16). Anhand der Fig. 18A und 18B wird ersichtlich, dass die Expression der Gene E1B55k und E4orf6 zur einer effizienten viralen Replikation und Zellyse führen bei gleichzeitig geringem erforderlichen Infektionstiter (augedrückt als pfu/Zelle). Dies bestätigt die der vorliegenden Erfindung zugrundeliegende Erkenntnis, dass die Expression von E4orf6 und E1B-55K (und das Nicht-Vorhandensen von E1A) in Verbindung mit der Kernlokalisation von YB-1 in der Lage ist, eine sehr effiziente adenovirale Replikation zu induzieren. Die dazu erforderliche Menge von nur 1 bis 5 pfu/Zelle lässt jetzt eine klinischen Anwendung zu.

Dies bestätigt die der vorliegenden Erfindung zugrundeliegende Erkenntnis, dass das Vorhandensein von YB-1 im Kern, insbesondere Zellzyklus-unabhängig, erforderlich ist, um die erfindungsgemäß zu verwendenden Viren zu einer Lyse von infizierten Zellen zu veranlassen.

**Patentansprüche**

1. Verwendung eines Adenovirus zur Herstellung eines Medikamentes für die Behandlung von Tumoren, **dadurch gekennzeichnet, dass** der Virus in Zellen repliziert, die YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, wobei das Onkogenprotein E1A ist und die den Tumor oder einen Teil davon ausbildenden Zellen YB-1 im Kern aufweisen.

2. *In vitro* Verwendung eines Adenovirus zur Replikation in Zellen, **dadurch gekennzeichnet, dass** die Zellen YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, und das Onkogenprotein E1A ist.

3. Adenovirus zur Verwendung als Medikament, **dadurch gekennzeichnet, dass** der Virus in Zellen repliziert, die YB-1 im Kern aufweisen, der Virus replikationsdefizient ist in Zellen, die YB-1 nicht im Kern aufweisen, der Virus für ein Onkogenprotein codiert, das zumindest ein virales Gen transaktiviert, wobei das Gen ausgewählt ist aus der Gruppe, die E1B55kDa, E4orf6, E4orf3 und E3ADP umfasst, wobei das Onkogenprotein E1A ist.

4. Adenovirus nach Anspruch 3 zur Verwendung bei der Behandlung von Tumoren, wobei die den Tumor oder einen Teil davon ausbildenden Zellen YB-1 im Kern aufweisen.

5. Verwendung nach Anspruch 1 und 2 und Adenovirus zur Verwendung nach Anspruch 3 und 4, **dadurch gekennzeichnet, dass** das virale Onkogenprotein E1A nicht die nukläre Lokalisation von YB-1 induziert.

6. Verwendung nach Anspruch 1 und 2 und Adenovirus zur Verwendung nach Anspruch 3 und 4, **dadurch gekennzeichnet, dass** die Zellen Rb-negativ und im Zellkern YB-1 positiv, insbesondere unabhängig vom Zellzyklus im Zellkern YB-1 positiv sind.

7. Verwendung nach einem der Ansprüche 1, 5 und 6 und Adenovirus zur Verwendung nach einem der Ansprüche 3 bis 6, **dadurch gekennzeichnet, dass** die den Tumor oder Teile davon ausbildenden Zellen eine Resistenz gegen pharmakologische Wirkstoffe aufweisen.

8. Verwendung nach Anspruch 7 und Adenovirus zur Verwendung nach Anspruch 7, **dadurch gekennzeichnet, dass** die Zellen eine Überexpression des membranständigen Transportproteins P-Glykoprotein zeigen.

9. Verwendung nach einem der Ansprüche 1, 2 und 5 bis 8 und Adenovirus zur Verwendung nach einem der Ansprüche 3 bis 8, **dadurch gekennzeichnet, dass** das virale Onkogenprotein unter der Kontrolle eines Gewebes- und/oder Tumor-spezifischen Promotors steht.

10. Verwendung nach einem der Ansprüche 1, 2 und 5 bis 9 und Adenovirus zur Verwendung nach einem der Ansprüche 3 bis 9, **dadurch gekennzeichnet, dass** der Virus für YB-1 codiert.

11. Verwendung nach Anspruch 10 und Adenovirus zur Verwendung nach Anspruch 10, **dadurch gekennzeichnet, dass** YB-1 unter der Kontrolle eines Gewebe- und/oder Tumor-spezifischen Promotors steht.

12. Verwendung nach einem der Ansprüche 1, 2 und 5 bis 11 und Adenovirus zur Verwendung nach einem der Ansprüche 3 bis 11, **dadurch gekennzeichnet, dass** der Virus für mindestens ein Protein codiert, das ausgewählt ist aus der Gruppe, die E4orf6, E4orf3, E1B55k und adenovirales E3ADP-Protein umfasst.

13. Verwendung nach Anspruch 12 und Adenovirus zur Verwendung nach Anspruch 12, **dadurch gekennzeichnet, dass** die den Tumor oder einen Teil davon ausbildenden Zellen YB-1 im Kern aufweisen.

14. Verwendung nach einem der Ansprüche 1 und 5 bis 13 und Adenovirus zur Verwendung nach einem der Ansprüche 4 bis 13, **dadurch gekennzeichnet, dass** der Tumor YB-1 im Kern nach Induktion des Transports von YB-1 in den Kern enthält.

15. Verwendung nach einem der Ansprüche 1, 2 und 5 bis 13 und Adenovirus zur Verwendung nach einem der Ansprüche 3 bis 14, **dadurch gekennzeichnet, dass** der Virus ausgewählt ist aus der Gruppe, die AdΔ24, dl922-947, E1Ad/01/07, dl1119/1131, CB 016, d1520, und Viren, denen ein exprimiertes virales Onkogen fehlt, das zur Bindung eines funktionellen Rb-Tumorsuppressor-Genprodukts fähig ist, umfasst.

16. Verwendung nach einem der Ansprüche 1, 2 und 5 bis 15 und Adenovirus zur Verwendung nach einem der Ansprüche 3 bis 15, wobei der Virus E1B 19 kDa-defizient ist.

17. Verwendung eines adenoviralen Replikationssystems umfassend eine Nukleinsäure, die für einen Adenovirus wie in einem der Ansprüche 1 bis 16 definiert codiert, und umfassend eine Nukleinsäure eines Helfervirus, wobei die Nukleinsäure des Helfervirus eine Nukleinsäuresequenz umfasst, die für YB-1 codiert, zur Herstellung eines Medikamentes für die Behandlung von Tumoren, wobei die den Tumor oder einen Teil davon ausbildenden Zellen YB-1 im Kern aufweisen, oder zur Replikation in Zellen, die YB-1 aufweisen.

18. Verwendung einer Nukleinsäure codierend für einen Adenovirus wie in einem der Ansprüche 1 bis 16 definiert, zur Herstellung eines Medikamentes für die Behandlung von Tumoren, wobei die den Tumor oder einen Teil davon ausbildenden Zellen YB-1 im Kern aufweisen.

19. Verwendung nach Anspruch 18, **dadurch gekennzeichnet, dass** die den Tumor oder Teile davon ausbildenden Zellen eine Resistenz gegen pharmakologische Wirkstoffe aufweisen.

20. Verwendung nach Anspruch 19, **dadurch gekennzeichnet, dass** die Resistenz eine Mehrfachresistenz ist.

21. Verwendung nach Anspruch 19 oder 20, **dadurch gekennzeichnet, dass** der pharmakologische Wirkstoff ein Antitumormittel ist.

22. Verwendung nach Anspruch 21, **dadurch gekennzeichnet, dass** das Antitumormittel ein Zytostatikum ist.

23. *In* vitro Verwendung einer Nukleinsäure, die für einen Adenovirus wie in einem der Ansprüche 1 bis 16 definiert codiert, zur Replikation in Zellen, **dadurch gekennzeichnet, dass** die Zellen YB-1 im Kern aufweisen, der Virus replikationsdefizient ist in Zellen, die YB-1 nicht im Kern aufweisen, und der Virus für ein Onkogen oder Onkogenprodukt codiert, das zumindest ein virales Gen transaktiviert, wobei das Gen ausgewählt ist aus der Gruppe, die E1B55kDa, E4orf6, E4orf3 und E3ADP umfasst.

24. Verwendung eines Vektors umfassend eine Nukleinsäure wie in einem der Ansprüche 18 bis 22 definiert zur Herstellung eines Medikamentes zur Behandlung von Tumoren, wobei die den Tumor oder einen Teil davon ausbildenden Zellen YB-1 im Kern aufweisen, oder zur Replikation in Zellen, die YB-1 im Kern aufweisen.

25. *In* vitro Verwendung eines mit YB-1 wechselwirkenden Mittels zur Charakterisierung von Zellen, Zellen eines Tumorgewebes oder Patienten, um zu bestimmen, ob diese(r) mit einem Virus wie in einem der Ansprüche 1 bis 16 definiert kontaktiert und/oder behandelt werden sollen, wobei das Mittel ausgewählt ist aus der Gruppe, die Antikörper, Antikaline, Aptamere, Aptazyme und Spiegelmere umfasst.

26. Verwendung nach einem der Ansprüche 1, 2 und 5 bis 16 und Adenovirus zur Verwendung nach einem der Ansprüche 3 bis 16, wobei der Virus eine für ein Transgen codierende Nukleinsäure umfasst.

27. Verwendung nach einem der Ansprüche 1, 2 und 5 bis 16 und Adenovirus zur Verwendung nach einem der Ansprüche 3 bis 16, wobei der Virus das Translations- und/oder das Transkriptionsprodukt eines Transgens umfasst.

28. Verwendung eines adenoviralen Replikationssystems nach Anspruch 17, wobei die Nukleinsäure des adenoviralen Replikationssystems und/oder die Nukleinsäure des Helfervirus ein Transgen oder eine für ein Transgen codierende Nukleinsäure umfasst.

29. Verwendung einer Nukleinsäure nach einem der Ansprüche 18 bis 23, wobei die Nukleinsäure ein Transgen oder eine für ein Transgen codierende Nukleinsäure umfasst.

30. Verwendung nach einem der Ansprüche 26 bis 29, wobei das Transgen ausgewählt ist aus der Gruppe, die Prodruggene, Zytokine, Apoptose-induzierende Gene, Tumorsuppressorgene, Gene für Metalloproteinasen-Inhibitoren und Gene für Angiogene-Inhibitoren umfasst.

31. Verwendung nach einem der Ansprüche 26 bis 29, wobei das Transgen ausgewählt ist aus der Gruppe, die Nukleinsäuren für siRNA, für Aptamere, für Antisense-Moleküle und für Ribozyme umfasst, wobei die siRNA, die Aptamere, die Antisense-Moleküle und/oder die Ribozyme gegen ein Zielmolekül gerichtet ist.

32. Verwendung nach Anspruch 31, wobei das Zielmolekül ausgewählt ist aus der Gruppe, die Resistenzrelevante Faktoren, Anti-Apoptose-Faktoren, Onkogene, Angiogenese-Faktoren, DNA-Synthese-Enzyme, DNA-Reparaturenzyme, Wachstumsfaktoren, Rezeptoren für Wachstumsfaktoren, Transkriptionsfaktoren, Metalloproteinasen, insbesondere Matrix-Metalloproteinkinasen, und Plasminogenaktivator vom Urokinase-Typ umfasst.

33. Verwendung nach einem der Ansprüche 1, 5 bis 16, 18 bis 22, 24 und 26 bis 32, wobei das Medikament weiterhin mindestens eine pharmazeutisch wirksame Verbindung enthält.

34. Adenovirus zur Verwendung nach einem der Ansprüche 4 bis 16 und 26 bis 27, wobei die Behandlung weiterhin die Verabreichung mindestens einer pharmazeutisch wirksamen Verbindung umfasst.

35. Verwendung nach Anspruch 33 und Adenovirus zur Verwendung nach Anspruch 34, wobei die pharmazeutisch wirksame Verbindung ausgewählt ist aus der Gruppe, die Zytokine, Metalloproteinasen-Inhibitoren, Angiogenese-Inhibitoren, Cytostatika und Zellzyklus-Inhibitoren umfasst.

**Claims**

1. Use of an adenovirus for the manufacture of a medicament for the treatment of tumors, **characterized in that** the virus replicates in cells having YB-1 in the nucleus, the virus is replication-deficient in cells not having YB-1 in the nucleus, the virus codes for an oncogene protein which transactivates at least one viral gene, wherein the gene is selected from the group comprising E1B55kDa, E4orf6, E4orf3 and E3ADP, wherein the oncogene protein is E1A and the cells forming the tumor or a part thereof have YB-1 in the nucleus.

2. *In vitro* use of an adenovirus for replication in cells, **characterized in that** the cells have YB-1 in the nucleus, the virus is replication-deficient in cells not having YB-1 in the nucleus, the virus codes for an oncogene protein which transactivates at least one viral gene, wherein the gene is selected from the group comprising EIB55kDa, E4orf6, E4orf3 and E3ADP, and wherein the oncogene protein is E1A.

3. Adenovirus for use as a medicament, **characterized in that** the virus replicates in cells having YB-1 in the nucleus, the virus is replication-deficient in cells not having YB-1 in the nucleus, the virus codes for an oncogene protein which transactivates at least one viral gene, wherein the gene is selected from the group comprising E1B55kDa, E4orf6, E4orf3 and E3ADP, wherein the oncogene protein is E1A.

4. Adenovirus of claim 3 for use in the treatment of tumors, wherein the cells forming the tumor or a part thereof have YB-1 in the nucleus.

5. Use of any one of claims 1 and 2 and adenovirus for use of any one of claims 3 and 4, **characterized in that** the viral oncogene protein E1A does not induce nuclear localization of YB-1.

6. Use of any one of claims 1 and 2 and adenovirus for use of any one of claims 3 and 4, **characterized in that** the cells are Rb negative and YB-1 positive in the nucleus, preferably are YB-1 positive in the nucleus independent of the cell cycle.

7. Use of any one of claims 1, 5 and 6 and adenovirus for use of any one of claims 3 to 6, **characterized in that** the cells forming the tumor or a part thereof are resistant against pharmacological agents.

8. Use of claim 7 and adenovirus for use of claim 7, **characterized in that** the cells overexpress membrane-bound transport protein P-glycoprotein.

9. Use of any one of claims 1, 2 and 5 to 8 and adenovirus for use of any one of claims 3 to 8, **characterized in that** the viral oncogene protein is under the control of a tissue-specific promoter and/or a tumor-specific promoter.

10. Use of any one of claims 1, 2 and 5 to 9 and adenovirus for use of any one of claims 3 to 9, **characterized in that** the virus codes for YB-1.

11. Use of claim 10 and adenovirus for use of claim 10, **characterized in that** YB-1 is under the control of a tissue-specific promoter and/or tumor-specific promoter.

12. Use of any one of claims 1, 2 and 5 to 11 and adenovirus for use of any one of claims 3 to 11, **characterized in that** the virus codes for at least one protein selected from the group comprising E4orf6, E4orf3, E1B55k and adenoviral E3ADP protein.

13. Use of claim 12 and adenovirus for use of claim 12, **characterized in that** the cells forming the tumor or a part thereof have YB-1 in the nucleus.

14. Use of any one of claims 1, 2 and 5 to 13 and adenovirus for use of any one of claims 4 to 13, **characterized in that** the tumor contains YB-1 in the nucleus after induction of YB-1 transport into the nucleus.

15. Use of any one of claims 1, 2 and 5 to 13 and adenovirus for use of any one of claims 3 to 14, **characterized in that** the virus is selected from the group comprising AdΔ24, dl922-947, E1Ad/01/07, dl1119/1131, CB 016, dl520, and viruses lacking an expressed viral oncogene which is capable of binding a functional Rb tumor suppressor gene product.

16. Use of any one of claims 1, 2 and 5 to 15 and adenovirus for use of any one of claims 3 to 15, wherein the virus is E1B 19 kDa deficient.

17. Use of an adenoviral replication system comprising a nucleic acid coding for an adenovirus as defined in any one of claims 1 to 16, and comprising a nucleic acid of a helper virus, wherein the nucleic acid of the helper virus comprises a nucleic acid sequence coding for YB-1, for the manufacture of a medicament for the treatment of tumors, wherein the cells forming the tumor or a part thereof have YB-1 in the nucleus, or for replication in cells having YB-1.

18. Use of a nucleic acid coding for an adenovirus as defined in any one of claims 1 to 16, for the manufacture of a medicament for the treatment of a tumor, wherein the cells forming the tumor or a part thereof have YB-1 in the nucleus.

19. Use of claim 18, **characterized in that** the cells forming the tumor or a part thereof are resistant to pharmacological agents.

20. Use of claim 19, **characterized in that** the resistance is a multiple resistance.

21. Use of any one of claims 19 and 20, **characterized in that** the pharmacological agent is an anti-tumor agent.

22. Use of claim 21, **characterized in that** the anti-tumor agent is a cytostatic.

23. *In vitro* use of a nucleic acid coding for an adenovirus as defined in any one of claims 1 to 16, for replication in cells, **characterized in that** the cells have YB-1 in the nucleus, the virus is replication-deficient in cells not having YB-1 in the nucleus, and the virus codes for an oncogene or oncogene product which transactivates at least one viral gene, wherein the gene is selected from the group comprising E1B55kDa, E4orf6, E4orf3 and E3ADP.

24. Use of a vector comprising a nucleic acid as defined in any one of claims 18 to 22, for the manufacture of a medicament for the treatment of tumors, wherein the cells forming the tumor or a part thereof have YB-1 in the nucleus, or for replication in cells which have YB-1 in the nucleus.

25. *In vitro* use of an agent interacting with YB-1 for characterizing cells, cells of a tumor tissue or patients for determining whether these may be contacted and/or treated with a virus as defined in any one of claims 1 to 16, wherein the agent is selected from the group comprising antibodies, anticalins, aptamers, aptazymes and Spiegelmers.

26. Use of any one of claims 1, 2 and 5 to 16 and adenovirus for use of any one of claims 3 to 16, wherein the virus comprises a nucleic acid coding for a transgene.

27. Use of any one of claims 1, 2 and 5 to 16 and adenovirus for use of any one of claims 3 to 16, wherein the virus comprises the translation product and/or the transcription product of a transgene.

28. Use of an adenoviral replication system of claim 17, wherein the nucleic acid of the adenoviral replication system and/or the nucleic acid of the helper virus comprises a transgene or a nucleic acid coding for a transgene.

29. Use of a nucleic acid of any one of claims 18 to 23, wherein the nucleic acid comprises a transgene or a nucleic acid coding for a transgene.

30. Use of any one of claims 26 to 29, wherein the transgene is selected from the group comprising prodrug genes, cytokines, apoptosis inducing genes, tumor suppressor genes, genes for metalloproteinase inhibitors and genes for angiogenesis inhibitors.

31. Use of any one of claims 26 to 29, wherein the transgene is selected from the group comprising nucleic acids for siRNA, for aptamers, for antisense molecules and for ribozymes, wherein the siRNA, aptamers, antisense molecules and/or ribozymes are directed to a target molecule.

32. Use of claim 31, wherein the target molecule is selected from the group comprising resistance-relevant factors, anti-apoptosis factors, oncogenes, angiogenesis factors, DNA synthesis enzymes, DNA repair enzymes, growth factors, receptors for growth factors, transcription factors, metalloproteinases, in particular matrix metalloproteinases, and urokinase-type plasminogen activator.

33. Use of any one of claims 1, 5 to 16, 18 to 22, 24 and 26 to 32, wherein the medicament further comprises at least one pharmaceutically active agent.

34. Adenovirus for use of any one of claims 4 to 16 and 26 to 27, wherein the treatment further comprises administering at least one pharmaceutically active agent.

35. Use of claim 33 and adenovirus for use of claim 34, wherein the pharmaceutically active agent is selected from the group comprising cytokines, metalloproteinase inhibitors, angiogenesis inhibitors, cytostatics and cell cycle inhibitors.

**Revendications**

1. Utilisation d'un adénovirus pour la production d'un médicament pour le traitement de tumeurs, **caractérisée en ce que** le virus se réplique dans des cellules qui présentent YB-1 dans le noyau, le virus est déficient pour la réplication dans des cellules qui ne présentent pas YB-1 dans le noyau, le virus code pour une protéine oncogène qui transactive au moins un gène viral, dans laquelle le gène est choisi dans le groupe qui comprend E1B55kDa, E4orf6, E4orf3 et E3ADP, dans laquelle la protéine oncogène est E1A et qui présentent des cellules YB-1 formant la tumeur ou une partie de celle-ci dans le noyau.

2. Utilisation *in vitro* d'un adénovirus pour la réplication dans des cellules, **caractérisée en ce que** les cellules présentent YB-1 dans le noyau, le virus est déficient pour la réplication dans des cellules qui ne présentent pas YB-1 dans le noyau, le virus code pour une protéine oncogène qui transactive au moins un gène viral, dans laquelle le gène est choisi dans le groupe qui comprend E1B55kDa, E4orf6, E4orf3 et E3ADP, et la protéine oncogène est E1A.

3. Adénovirus pour l'utilisation comme médicament, **caractérisé en ce que** le virus se réplique dans des cellules qui présentent YB-1 dans le noyau, le virus est déficient pour la réplication dans des cellules qui ne présentent pas YB-1 dans le noyau, le virus code pour une protéine oncogène qui transactive au moins un gène viral, dans lequel le gène est choisi dans le groupe qui comprend E1B55kDa, E4orf6, E4orf3 et E3ADP, et la protéine oncogène est E1A.

4. Adénovirus selon la revendication 3 pour l'utilisation dans le traitement de tumeurs, dans lequel les cellules formant la tumeur ou une partie de celle-ci présentent YB-1 dans le noyau.

5. Utilisation selon la revendication 1 et 2 et adénovirus pour l'utilisation selon la revendication 3 et 4, **caractérisés en ce que** la protéine oncogène virale EIA n'induit pas la localisation nucléaire d'YB-1.

6. Utilisation selon la revendication 1 et 2 et adénovirus pour l'utilisation selon la revendication 3 et 4, **caractérisés en ce que** les cellules sont Rb-négatives et dans le noyau cellulaire, sont YB-1 positives, en particulier indépendamment du cycle cellulaire, sont YB-1 positives dans le noyau cellulaire.

7. Utilisation selon l'une quelconque des revendications 1, 5 et 6 et adénovirus pour l'utilisation selon l'une quelconque des revendications 3 à 6, **caractérisés en ce que** les cellules formant la tumeur ou des parties de celle-ci présentent une résistance contre des substances pharmacologiques.

8. Utilisation selon la revendication 7 et adénovirus pour l'utilisation selon la revendication 7, **caractérisés en ce que** les cellules présentent une surexpression de la protéine de transport glycoprotéine P membranaire.

9. Utilisation selon l'une quelconque des revendications 1, 2 et 5 à 8 et adénovirus pour l'utilisation selon l'une quelconque des revendications 3 à 8, **caractérisés en ce que** la protéine oncogène virale est sous le contrôle d'un promoteur spécifique d'un tissu et/ou d'une tumeur.

**10.** Utilisation selon l'une quelconque des revendications 1, 2 et 5 à 9 et adénovirus pour l'utilisation selon l'une quelconque des revendications 3 à 9, **caractérisés en ce que** le virus code pour YB-1.

**11.** Utilisation selon la revendication 10 et adénovirus pour l'utilisation selon la revendication 10, **caractérisés en ce que** YB-1 est sous le contrôle d'un promoteur spécifique d'un tissu et/ou d'une tumeur.

**12.** Utilisation selon l'une quelconque des revendications 1, 2 et 5 à 11 et adénovirus pour l'utilisation selon l'une quelconque des revendications 3 à 11, **caractérisés en ce que** le virus code pour au moins une protéine qui est choisie dans le groupe qui comprend E4orf6, E4orf3, E1B55k et la protéine E3ADP adénovirale.

**13.** Utilisation selon la revendication 12 et adénovirus pour l'utilisation selon la revendication 12, **caractérisés en ce que** les cellules formant la tumeur ou une partie de celle-ci présentent YB-1 dans le noyau.

**14.** Utilisation selon l'une quelconque des revendications 1 et 5 à 13 et adénovirus pour l'utilisation selon l'une quelconque des revendications 4 à 13, **caractérisés en ce que** la tumeur contient YB-1 dans le noyau après induction du transport d'YB-1 dans le noyau.

**15.** Utilisation selon l'une quelconque des revendications 1, 2 et 5 à 13 et adénovirus pour l'utilisation selon l'une quelconque des revendications 3 à 14, **caractérisés en ce que** le virus est choisi dans le groupe qui comprend AdΔ24, dl922-947, E1Ad/01/07, dl1119/1131, CB 016, dl520, et parmi des virus auxquels manque un oncogène viral exprimé qui est capable de se lier à un produit de gène suppresseur de tumeur Rb fonctionnel.

**16.** Utilisation selon l'une quelconque des revendications 1, 2 et 5 à 15 et adénovirus pour l'utilisation selon l'une quelconque des revendications 3 à 15, dans lesquels le virus est déficient pour E1B de 19 kDa.

**17.** Utilisation d'un système de réplication adénoviral comprenant un acide nucléique qui code pour un adénovirus tel que défini dans l'une quelconque des revendications 1 à 16, et comprenant un acide nucléique d'un virus auxiliaire, dans laquelle l'acide nucléique du virus auxiliaire comprend une séquence d'acide nucléique qui code pour YB-1, pour la production d'un médicament pour le traitement de tumeurs, dans laquelle les cellules formant la tumeur ou des parties de celle-ci présentent YB-1 dans le noyau, ou pour la réplication dans des cellules qui présentent YB-1.

**18.** Utilisation d'un acide nucléique codant pour un adénovirus tel que défini dans l'une quelconque des revendications 1 à 16, pour la production d'un médicament pour le traitement de tumeurs, dans laquelle les cellules formant la tumeur ou une partie de celle-ci présentent YB-1 dans le noyau.

**19.** Utilisation selon la revendication 18, **caractérisée en ce que** les cellules formant la tumeur ou une partie de celle-ci présentent une résistance contre une substance pharmacologique.

**20.** Utilisation selon la revendication 19, **caractérisée en ce que** la résistance est une résistance multiple.

**21.** Utilisation selon la revendication 19 ou 20, **caractérisée en ce que** la substance pharmacologique est un agent antitumoral.

**22.** Utilisation selon la revendication 21, **caractérisée en ce que** l'agent antitumoral est un cytostatique.

**23.** Utilisation *in vitro* d'un acide nucléique qui code pour un adénovirus tel que défini dans l'une quelconque des revendications 1 à 16, pour la réplication dans des cellules, **caractérisée en ce que** les cellules présentent YB-1 dans le noyau, le virus est déficient pour la réplication dans des cellules qui ne présentent pas YB-1 dans le noyau, et le virus code pour un oncogène ou un produit oncogène qui transactive au moins un gène viral, dans lequel le gène est choisi dans le groupe qui comprend E1B55kDa, E4orf6, E4orf3 et E3ADP.

**24.** Utilisation d'un vecteur comprenant un acide nucléique tel que défini dans l'une quelconque des revendications 18 à 22, pour la production d'un médicament pour le traitement de tumeurs, dans laquelle les cellules formant la tumeur ou des parties de celle-ci présentent YB-1 dans le noyau, ou pour la réplication dans des cellules qui présentent YB-1.

**25.** Utilisation *in vitro* d'un agent interagissant avec YB-1 pour la caractérisation de cellules, de cellules d'un tissu tumoral ou de patients, pour déterminer si celles-ci/ceux-ci doivent être mis en contact et/ou traités avec un virus tel que défini dans l'une quelconque des revendications 1 à 16, dans laquelle l'agent est choisi dans le groupe qui comprend des anticorps, des anticalines, des aptamères, des aptazymes et des spiegelmères.

**26.** Utilisation selon l'une quelconque des revendications 1, 2 et 5 à 16 et adénovirus pour l'utilisation selon l'une quelconque des revendications 3 à 16, dans lesquels le virus comprend un acide nucléique codant pour un transgène.

27. Utilisation selon l'une quelconque des revendications 1, 2 et 5 à 16 et adénovirus pour l'utilisation selon l'une quelconque des revendications 3 à 16, dans lesquels le virus comprend le produit de traduction et/ou le produit de transcription d'un transgène.

28. Utilisation d'un système de réplication adénoviral selon la revendication 17, dans laquelle l'acide nucléique du système de réplication adénoviral et/ou l'acide nucléique du virus auxiliaire comprend un transgène ou un acide nucléique codant pour un transgène.

29. Utilisation d'un acide nucléique selon l'une quelconque des revendications 18 à 23, dans laquelle l'acide nucléique comprend un transgène ou un acide nucléique codant pour un transgène.

30. Utilisation selon l'une quelconque des revendications 26 à 29, dans laquelle le transgène est choisi dans le groupe qui comprend des gènes de promédicament, des cytokines, des gènes inducteurs de l'apoptose, des gènes suppresseurs de tumeurs, des gènes pour des inhibiteurs des métalloprotéinases et des gènes pour des inhibiteurs d'angiogènes.

31. Utilisation selon l'une quelconque des revendications 26 à 29, dans laquelle le transgène est choisi dans le groupe qui comprend des acides nucléiques pour des ARNsi, pour des aptamères, pour des molécules anti-sens et pour des ribozymes, dans laquelle les ARNsi, les aptamères, les molécules anti-sens et/ou les ribozymes sont dirigés comme une molécule cible.

32. Utilisation selon la revendication 31, dans laquelle la molécule cible est choisie dans le groupe qui comprend des facteurs pertinents pour la résistance, des facteurs anti-apoptose, des oncogènes, des facteurs de l'angiogenèse, des enzymes de synthèse de l'ADN, des enzymes de réparation de l'ADN, des facteurs de croissance, des récepteurs de facteurs de croissances, des facteurs de transcription, des métalloprotéinases, en particulier des métalloprotéinekinases de matrice, et un activateur de plasminogène du type urokinase.

33. Utilisation selon l'une quelconque des revendications 1, 5 à 16, 18 à 22, 24 et 26 à 32, dans laquelle le médicament contient en outre au moins un composé pharmaceutiquement efficace.

34. Adénovirus pour l'utilisation selon l'une quelconque des revendications 4 à 16 et 26 à 27, dans lequel le traitement comprend en outre l'administration d'au moins un composé pharmaceutiquement efficace.

35. Utilisation selon la revendication 33 et adénovirus pour l'utilisation selon la revendication 34, dans lesquels le composé pharmaceutiquement efficace est choisi dans le groupe qui comprend des cytokines, des inhibiteurs de métalloprotéinases, des inhibiteurs de l'angiogenèse, des cytostatiques et des inhibiteurs du cycle cellulaire.

Fig. 1

Fig. 2

Fig. 3

## E1/E3-minus Ad5

**40 pfu/Zelle**    **10 pfu/Zelle**    **Nicht infiziert**

**Nicht infiziert**    **40 pfu/Zelle**    **10 pfu/Zelle**

## dl520

EP 1 506 021 B1

**E1/E3-minus Ad5**

**Fig. 4**

40 pfu/Zelle     10 pfu/Zelle     Nicht infiziert

Nicht infiziert   40 pfu/Zelle     10 pfu/Zelle

**dl520**

EP 1 506 021 B1

## Fig. 5

nicht infiziert    20 pfu/Zelle

257RDB

U2OS

Fig. 6

**Fig. 7**

## WT-AD5

| | | CR1 | | CR2 | CR3 | | |
|---|---|---|---|---|---|---|---|

N ... C

1    40    80    120    139    188    289

## dl520

| | CR1 | | CR2 |
|---|---|---|---|

N ... C

1    40    80    120    139      186    289

## dl1119/1131

N □        | CR3 | |        C

1 3        139        218

EP 1 506 021 B1

pfu/ml

Fig. 8

## Fig. 9

fach

Bar chart with y-axis labeled in "fach" units from 0 to 25. Four bars: AdYB-1 (~1), AdYB-1 + E4orf6 (~3.5), AdYB-1 + E1B-55K (~7), AdYB-1 + E1B-55K + E4orf6 (~25, off scale).

## Fig. 10

**Kontrolle:**
Kein Daunorubicin

**Daunorubicin:**
40 ng/ml

C 10 30 C 10 30

Fig. 11

Kontrolle:
Kein Daunorubicin

Daunorubicin:
40 ng/ml

**Fig. 12**

**Fig. 13**   **PBS**   $5 \times 10^8$  dl520

EP 1 506 021 B1

Fig. 14

257RDB　　　HeLa

YB-1 Kernpositiv　YB-1 Kernnegativ

1　2　3　4　5　6　7　8

Southern Blot-Analyse

Fig. 15

Fig. 16

Wildtyp Adenovirus

E1 · MLP · E2B · E2A · E3 · E4

Xvir03

E1 · MLP · E2B · E2A · E3 · E4

E1B55k - IRES - E4orf6

CMV-Promoter

**Fig. 17**

**Xvir03/01**

Fig. 18A

Fig. 18B

Nicht infiziert   20 pfu Ad312   5 pfu Xvir03

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0931830 A
- DE 10150984
- DE 19742706
- EP 0533838 A
- WO 9808856 A

**In der Beschreibung aufgeführte Nicht-Patentliteratur**

- **GANLY I et al.** *Gene Therapy,* 2001, vol. 8, 369-375
- **KHURI, F. et al.** *Nature Medicine,* 2000, vol. 6, 879-885
- **KIRN, D. et al.** *Proc. Am. Soc. Clin. Oncol.,* 1998, vol. 17, 391a
- **ZAMBETTI, G.P. et al.** *FASEB J.,* 1993, vol. 7, 855-865
- **HOWE, J. A. et al.** *Molecular Therapy,* 2000, vol. 2, 485-495
- **FUEYO, J. et al.** *Oncogene,* 2000, vol. 19, 2-12
- **HEISE, C. et al.** *Nature Medicine,* 2001, vol. 6, 11341139
- **BALAGUE, C. et al.** *J. Virol.,* 2001, vol. 75, 7602-7611
- **DYSON, N.** *Genes & Development,* 1998, vol. 12, 2245-2262
- **RODRIGUEZ, R. et al.** *Cancer Res.,* 1997, vol. 57, 2559-2563
- **GOTTESMAN ; PASTAN.** *Annu. Rev. Biochem.,* 1993, vol. 62, 385-427
- **STEIN, U. et al.** *JBC,* 2001, vol. 276, 28562-69
- **J. WIJNHOLDS.** *Novartis Found Symp.,* 2002, vol. 243, 69-79
- **BARGOU, R. C. et al.** *Nature Medicine,* 1997, vol. 3, 447-450
- *Clin. Cancer Res.,* 1998, vol. 4, 2273-2277
- **KOIKE, K. et al.** *FEBS Lett,* 1997, vol. 17, 390-394
- **HOLM P S et al.** *JBC,* 2002, vol. 277, 10427-10434
- **HOWE J A et al.** *Molecular Therapy,* November 2000, vol. 2 (5), 485-494
- **WONG H K et al.** *Journal of Virology,* August 1994, vol. 68 (8), 4910-4920
- **RUSSELL, W. C.** *Journal of Virology,* 2000, vol. 81, 2573-2604
- **TOLLEFSON, A. et al.** *J. Virology,* 1996, vol. 70, 2296-2306
- **KIRN et al.** *Trends in Molecular Medicine,* 2002, vol. 8 (4
- **WYBRANIETZ W.A. et al.** *Gene Therapy,* 2001, vol. 8, 1654-1664
- **NICULESCU-DUVAZ et al.** *Curr. Opin. Mol. Therapy,* 1999, vol. 1, 480.486
- **KOYAMA et al.** *Cancer Gene Therapy,* 2000, vol. 7, 1015-1022
- **ROGERS et al.** *Human Gene Therapy,* 1996, vol. 7, 2235-2245
- **LOCKETT et al.** *Clinical Cancer Res.,* 1997, vol. 3, 2075-2080
- **VIJAYAKRISHNA et al.** *J. Pharmacol. And Exp. Therapeutics,* 2003, vol. 304, 1280-1284
- Gene Therapy, Advances in Pharmacology. Academic Press, vol. 40
- **ZHANG ; DEGROOT.** *Endocrinology,* 2003, vol. 144, 1393-1398
- **DESCAMPS et al.** *J. Mol. Med.,* 1996, vol. 74, 183-189
- **MAJUMDAR et al.** *Cancer Gene Therapy,* 2000, vol. 7, 1086-1099
- **TRALHAO et al.** *FASEB J,* 2003, vol. 17, 464-466
- **ZHANG et al.** *Cancer Res.,* 2003, vol. 63, 760-765
- **ZHANG et al.** *Hum. Gene Ther.,* 2002, vol. 20, 2051-2064
- **NOTEBORN ; PIETERSEN.** *Adv. Exp. Med. Biol.,* 2000, vol. 465, 153-161
- **TOTH et al.** *Cancer Gene Therapy,* 2003, vol. 10, 193-200
- **SUMANTRAN et al.** *Cancer Res,* 1995, vol. 55, 2507-2512
- **BRAITHWAITE ; RUSSELL.** *Apoptosis,* 2001, vol. 6, 359-370
- **ARAI et al.** *PNAC,* 1997, vol. 94, 13862-13867
- **YAMAGUCHI et al.** *Gene Therapy,* 2003, vol. 10, 375-385
- *Oncology News,* 17. Juni 2000
- **OPALKA et al.** *Cell Tissues Organs,* 2002, vol. 172, 126-132

- **JI et al.** *Cancer Res.,* 1999, vol. 59, 3333-3339
- **SU et al.** *Oncogene,* 2003, vol. 22, 1164-1180
- **HAJITOU et al.** *FASEB J.,* 2002, vol. 16, 1802-1804
- **FERRARA, N.** *Semin Oncol,* Dezember 2002, vol. 29 (16), 10-4
- **AHONEN et al.** *Mol Therapy,* 2002, vol. 5, 705-715
- **SOFF et al.** *J. Clin. Invest.,* 1995, vol. 96, 2593-2600
- **BRANDT K.** *Curr. Gene Therapy,* 2002, vol. 2, 255-271
- **ELBASHIR, S. ; HARBORTH J. ; LENDECKEL W. ; YALVCIN, A. ; WEBER K ; TUSCHL T.** Duplexes of 21-nucleotide RNAs mediate RNA interference in cultured mammalian cells. *Nature,* 2001, vol. 411, 494-498
- **BRUMMELKAMP et al.** *Science,* 2002, vol. 296, 550-553
- **BEN-ISRAEL ; KLEIBERGER.** *Frontiers in Bioscience,* 2002, vol. 7, 1369-1395
- **HELT ; GALLOWAY.** *Carcinogenesis,* 2003, vol. 24, 159-169
- **WHITTAKER, G.R. et al.** *Virology,* 1998, vol. 246, 1-23
- **FRIEDBERG, E.C.** *TIBS,* 1992, vol. 17, 347
- **JANS, D.A. et al.** *Bioessays,* Juni 2000, vol. 22 (6), 532-44
- **YONEDA, Y.** *J. Biocehm.,* Mai 1997, vol. 121 (5), 811-7
- **BOULIKAS, T.** *Crit. Rev. Eukaryot. Gene Expr.,* 1993, vol. 3 (3), 193-227
- **LYONS RH.** *Mol. Cell Biol.,* 1987, vol. 7, 2451-2456
- **EFTHYMIADIS, A. ; BRIGGS, LJ ; JANS, DA.** *JBC,* 1998, vol. 273, 1623-1628
- Adenoviruses as cloning vectors. **GRUNHAUS, A. ; HORWITZ, M.S.** Seminars in Virology. Saunders Scientific Publications, 1994
- Adenoviridae: The virus and their replication. **SHENK, T. et al.** Fields Virology. Lippincott-Raven Publishers, 1996
- **NEVINS, J. R.** *Cell,* 1981, vol. 26, 213-220
- **WHYTE, P. et al.** *Nature,* 1988, vol. 334, 124-127
- **NEVINS, J. R.** *Science,* 1992, vol. 258, 424-429
- The Molecular Repertoire of Adenoviruses III: Current Topics. **SWAMINATHAN ; THIMMAPAYA.** Microbiology and Immunology. Springer Verlag, 1995, vol. 199, 177-194
- **SWAMINATHAN ; THIMMAPAYA.** *JBC,* 1996, vol. 258, 736-746
- **STEEGENGA, W. T. et al.** *Oncogene,* 1998, vol. 16, 349-357
- **BRIDGE ; KETNER.** *Virology,* 1990, vol. 174, 345-353
- **ORNELLES ; SHENK.** *J. Virology,* 1991, vol. 65, 424-429
- **MARSHALL S. HORWITZ.** *Virololgie,* 2001, vol. 279, 1-8
- **TOLLEFSON.** *J. Virology,* 1996, vol. 70, 2296-2306
- **DORONIN et al.** *J. Virology,* 2000, vol. 74, 6147-6155
- **NEVINS J. R.** *Cell,* 1981, vol. 26, 213-220
- **BOULANGER, P. ; BLAIR, E.** *Biochem. J.,* 1991, vol. 275, 281-299
- **WONG HK ; ZIFF EB.** *J Virol.,* 1994, vol. 68, 4910-20
- **MYMRYK, J. S. ; BAYLEY, S. T.** *Virus Research,* 1994, vol. 33, 89-97
- **WEIGEL, S. ; DOBBELSTEIN , M.** *J. Virology,* 2000, vol. 74, 764-772
- **KEITH N. LEPPARD.** *Seminars in Virology,* 1998, vol. 8, 301-307
- **WOLFFE, A. P.** Trends. *Cell Biology,* 1998, vol. 8, 318-323
- **SWAMYNATHAN, S. K. et al.** *FASEB J.,* 1998, vol. 12, 515-522
- **OKAMOTO, T. et al.** *Oncogene,* 2000, vol. 19, 6194-6202
- **LASHAM, A. et al.** *Gene,* 2000, vol. 252, 1-13
- **MERTENS. P. R. et al.** *JBC,* 1997, vol. 272, 22905-22912
- **SHIBAO, K. et al.** *Int. J. Cancer,* 1999, vol. 83, 732-737
- **CHEN, C-Y. et al.** *Genes & Development,* 2000, vol. 14, 1236-1248
- **OHGA, T. et al.** *Cancer Res.,* 1996, vol. 56, 4224-4228
- **HOLM, P. S. et al.** *JBC,* 2002, vol. 277, 10427-10434
- **GOODRUM, F. D. ; ORNELLES, D. A.** *J. Virology,* 1999, vol. 73, 7474-7488
- **STEIN U ; JURCHOTT K ; WALTHER W ; BERGMANN S ; SCHLAG PM ; ROYER HD.** *J Biol Chem.,* 2001, vol. 276 (30), 28562-9
- **HU Z ; JIN S ; SCOTTO KW.** *J Biol Chem.,* 28. Januar 2000, vol. 275 (4), 2979-85
- **OHGA T ; UCHIUMI T ; MAKINO Y ; KOIKE K ; WADA M ; KUWANO M ; KOHNO K.** *J Biol Chem.,* 1998, vol. 273 (11), 5997-6000
- **MAKINO Y. et al.** *Nucleic Acids Res.,* 1996, vol. 15, 1873-1878
- **CAO, G. ; KURIYAMA, S. ; GAO, J. ; MITORO, A. ; CUI, L. ; NAKATANI, T. ; ZHANG, X. ; KIKUKAWA, M. ; PAN, X. ; FUKUI, H.** *Int. J. Cancer,* 1998, vol. 78, 242-247
- **CHUNG, I. ; SCHWARTZ, PE. ; CRYSTAL, RC. ; PIZZORNO, G ; LEAVITT, J. ; DEISSEROTH, AB.** *Cancer Gene Therapy,* 1999, vol. 6, 99-106
- **COULSON, JM ; STALEY, J. ; WOLL, PJ.** *British J. Cancer,* 1999, vol. 80, 1935-1944

- **TSUKADA et al.** *Cancer Res.,* vol. 62, 3428-3477
- **ZHANG et al.** *Cancer Res.,* 2002, vol. 62, 3743-3750
- **HALLENBECK PL ; CHANG, YN ; HAY, C ; GO-LIGHTLY, D. ; STEWART, D. ; LIN, J. ; PHIPPS, S. ; CHIANG, YL.** *Human Gene Therapy,* 1999, vol. 10, 1721-1733
- **BRAUNSTEIN, I. et al.** *Cancer Research,* 2001, vol. 61, 5529-5536
- **MAJUMDAR, A. S. et al.** *Gene Therapy,* 2001, vol. 8, 568-578
- **HASAN S. et al.** *Nature,* 2001, vol. 15, 387-391
- **SHIBAO K et al.** *Int. Cancer,* 1999, vol. 83, 732-737
- **ODA Y et al.** *Clin. Cancer Res.,* 1998, vol. 4, 2273-2277
- **KAMIYA, M. ; NAKAZATP, Y.** *J Neurooncology,* 2002, vol. 59, 143-149
- **STIEWE et al.** *J. Biol. Chem.,* 2003, vol. 278, 14230-14236
- **PIGANEAU, N. et al.** *Angew. Chem. Int. Ed.,* 2000, vol. 39 (29), 4369-4373
- **WONG ; ZIFF.** *J. Virol.,* 1994, vol. 68, 4910-4920
- **PELLETIER, J. ; SONENBERG, N.** *Nature,* 1988, vol. 334, 320-325